# NEW EUROPEAN PATENT SPECIFICATION

(11) **EP 3 346 003 B2**
(45) Date of publication and mention of the opposition decision: **18.11.2026**
(45) Mention of the grant of the patent: 09.06.2021
(21) Application number: 17206792.8
(22) Date of filing: 23.10.2013
(51) Int. Cl.: C12N 9/22, C12N 15/82, C12N 15/63, C12N 15/10, C12N 15/11

(54) **COMPOSITION FOR CLEAVING A TARGET DNA COMPRISING A GUIDE RNA SPECIFIC FOR THE TARGET DNA AND CAS PROTEIN-ENCODING NUCLEIC ACID OR CAS PROTEIN, AND USE THEREOF**
ZUSAMMENSETZUNG ZUR SPALTUNG EINER ZIEL-DNA MIT EINER FÜR DIE ZIEL-DNA SPEZIFISCHEN LEIT-DNA UND FÜR CAS- PROTEIN CODIERENDE NUKLEINSÄURE ODER CAS-PROTEIN SOWIE VERWENDUNG DAVON
COMPOSITION POUR LE CLIVAGE D'UN ADN CIBLE COMPRENANT UN ARN GUIDE SPÉCIFIQUE DE L'ADN CIBLE ET UN ACIDE NUCLÉIQUE CODANT POUR LA PROTÉINE CAS OU LA PROTÉINE CAS, ET LEUR UTILISATION

(30) Priority: 23.10.2012 US 201261717324 P; 20.03.2013 US 201361803599 P; 20.06.2013 US 201361837481 P
(43) Date of publication of application: 11.07.2018
(62) Document number(s) of the earlier application(s) in accordance with Art. 76 EPC: 13849670.8 / 2 912 175
(73) Proprietor: Toolgen Incorporated, Seoul 07789 (KR)
(72) Inventor: Kim, Jin-Soo, Seoul 151-747 (KR); Cho, Seung Woo, Seoul 151-747 (KR); Kim, Sojung, Seoul 151-747 (KR)
(74) Representative: D Young & Co LLP

(56) References cited:
- WO-A1-2013/176772
- WO-A1-2014/089290
- WO-A1-2014/144155
- WO-A1-2014/204725
- WO-A1-2015/026883
- WO-A2-2014/197568
- M. JINEK ET AL: "A Programmable Dual-RNA-Guided DNA Endonuclease in Adaptive Bacterial Immunity", SCIENCE, vol. 337, no. 6096, 17 August 2012 (2012-08-17), pages 816 - 821, XP055299674, ISSN: 0036-8075, DOI: 10.1126/science.1225829
- SHAN QIWEI ET AL: "Targeted genome modification of crop plants using a CRISPR-Cas system", NATURE BIOTECHNOLOGY, vol. 31, no. 8, 8 August 2013 (2013-08-08), pages 688 - 690, XP055153530, ISSN: 1087-0156, DOI: 10.1038/nbt.2650
- QIWEI SHAN ET AL: "Supplementary Material for Targeted genome modification of crop plants using a CRISPR-Cas system", NATURE BIOTECHNOLOGY, vol. 31, no. 8, 8 August 2013 (2013-08-08), pages 1 - 19, XP055463550, ISSN: 1087-0156, DOI: 10.1038/nbt.2650
- LI JIAN-FENG ET AL: "Multiplex and homologous recombination-mediated genome editing in Arabidopsis and Nicotiana benthamiana using guide RNA and Cas9", NATURE BIOTECHNOLOGY, vol. 31, no. 8, 8 August 2013 (2013-08-08), pages 688 - 691, XP055133339, ISSN: 1087-0156, DOI: 10.1038/nbt.2654
- VLADIMIR NEKRASOV ET AL: "Targeted mutagenesis in the model plant Nicotiana benthamiana using Cas9 RNA-guided endonuclease", NATURE BIOTECHNOLOGY, vol. 31, no. 8, 8 August 2013 (2013-08-08), pages 691 - 693, XP055129106, ISSN: 1087-0156, DOI: 10.1038/nbt.2655
- ZHENGYAN FENG ET AL: "Efficient genome editing in plants using a CRISPR/Cas system", CELL RESEARCH, vol. 23, no. 10, 20 August 2013 (2013-08-20), pages 1229 - 1232, XP055153531, ISSN: 1001-0602, DOI: 10.1038/cr.2013.114
- W. JIANG ET AL: "Demonstration of CRISPR/Cas9/sgRNA-mediated targeted gene modification in Arabidopsis, tobacco, sorghum and rice", NUCLEIC ACIDS RESEARCH, vol. 41, no. 20, 2 September 2013 (2013-09-02), pages e188 - e188, XP055219328, ISSN: 0305-1048, DOI: 10.1093/nar/gkt780
- JIN MIAO ET AL: "Targeted mutagenesis in rice using CRISPR-Cas system", CELL RESEARCH, vol. 23, no. 10, 3 September 2013 (2013-09-03), pages 1233 - 1236, XP055153533, ISSN: 1001-0602, DOI: 10.1038/cr.2013.123
- F ANN RAN ET AL: "Double Nicking by RNA-Guided CRISPR Cas9 for Enhanced Genome Editing Specificity", CELL, vol. 154, no. 6, 29 August 2013 (2013-08-29), pages 1380 - 1389, XP055299681, ISSN: 0092-8674, DOI: 10.1016/j.cell.2013.08.021
- P. MALI ET AL: "RNA-Guided Human Genome Engineering via Cas9", SCIENCE, vol. 339, no. 6121, 3 January 2013 (2013-01-03), pages 823 - 826, XP055111247, ISSN: 0036-8075, DOI: 10.1126/science.1232033
- SEUNG WOO CHO ET AL: "Targeted genome engineering in human cells with the Cas9 RNA-guided endonuclease (includes Supplementary information)", NATURE BIOTECHNOLOGY, GALE GROUP INC, vol. 31, no. 3, 29 January 2013 (2013-01-29), pages 230 - 232, 1, XP002699850, ISSN: 1087-0156, [retrieved on 20130129], DOI: 10.1038/NBT.2507
- L. CONG ET AL: "Multiplex Genome Engineering Using CRISPR/Cas Systems", SCIENCE, vol. 339, no. 6121, 15 February 2013 (2013-02-15), pages 819 - 823, XP055067741, ISSN: 0036-8075, DOI: 10.1126/science.1231143
- MARTIN JINEK ET AL: "RNA-programmed genome editing in human cells", ELIFE, ELIFE SCIENCES PUBLICATIONS LTD, GB, vol. 2, 29 January 2013 (2013-01-29), pages e00471 - 1, XP002699851, ISSN: 2050-084X, [retrieved on 20130628], DOI: 10.7554/ELIFE.00471
- SONG LUO: "Non-transgenic Plant Genome Editing Using Purified Sequence-Specific Nucleases", 12 June 2015 (2015-06-12), XP055488400, Retrieved from the Internet <URL:https://www.sciencedirect.com/science/article/pii/S1674205215002658/pdfft?md5=edad2b323b078e43912891830c4914a8&pid=1-s2.0-S1674205215002658-main.pdf> [retrieved on 20180627]
- SERGEI SVITASHEV ET AL: "Genome editing in maize directed by CRISPR-Cas9 ribonucleoprotein complexes", NATURE COMMUNICATIONS, vol. 7, 16 November 2016 (2016-11-16), pages 13274, XP055359029, DOI: 10.1038/ncomms13274
- JE WOOK WOO ET AL: "DNA-free genome editing in plants with preassembled CRISPR-Cas9 ribonucleoproteins", NATURE BIOTECHNOLOGY, vol. 33, no. 11, 19 October 2015 (2015-10-19), us, pages 1162 - 1164, XP055290196, ISSN: 1087-0156, DOI: 10.1038/nbt.3389

   <110> ToolGen Inc.
   <120> Composition for cleaving a target DNA comprising a guide RNA specific for the target DNA and Cas protein-encoding nucleic acid or Cas protein, and use thereof
   <130> 104653PCEPT1
   <140> Divisional Application based on EP 13849670.8
      <141> 2013-10-23
   <150> US 61/717,324
      <151> 2012-10-23
   <150> US 61/803,599
      <151> 2013-03-20
   <150> US 61/837,481
      <151> 2013-06-20
   <160> 111
   <170> KopatentIn 2.0
   <210> 1
      <211> 4107
      <212> DNA
      <213> Artificial Sequence
   <220>
      <223> Cas9-coding sequence
   <400> 1 
atggacaaga agtacagcat cggcctggac atcggtacca acagcgtggg ctgggccgtg 60

atcaccgacg agtacaaggt gcccagcaag aagttcaagg tgctgggcaa caccgaccgc 120

cacagcatca agaagaacct gatcggcgcc ctgctgttcg acagcggcga gaccgccgag 180

gccacccgcc tgaagcgcac cgcccgccgc cgctacaccc gccgcaagaa ccgcatctgc 240

tacctgcagg agatcttcag caacgagatg gccaaggtgg acgacagctt cttccaccgc 300

ctggaggaga gcttcctggt ggaggaggac aagaagcacg agcgccaccc catcttcggc 360

aacatcgtgg acgaggtggc ctaccacgag aagtacccca ccatctacca cctgcgcaag 420

aagctggtgg acagcaccga caaggccgac ctgcgcctga tctacctggc cctggcccac 480

atgatcaagt tccgcggcca cttcctgatc gagggcgacc tgaaccccga caacagcgac 540

gtggacaagc tgttcatcca gctggtgcag acctacaacc agctgttcga ggagaacccc 600

atcaacgcca gcggcgtgga cgccaaggcc atcctgagcg cccgcctgag caagagccgc 660

cgcctggaga acctgatcgc ccagctgccc ggcgagaaga agaacggcct gttcggcaac 720

ctgatcgccc tgagcctggg cctgaccccc aacttcaaga gcaacttcga cctggccgag 780

gacgccaagc tgcagctgag caaggacacc tacgacgacg acctggacaa cctgctggcc 840

cagatcggcg accagtacgc cgacctgttc ctggccgcca agaacctgag cgacgccatc 900
 
ctgctgagcg acatcctgcg cgtgaacacc gagatcacca aggcccccct gagcgccagc 960

atgatcaagc gctacgacga gcaccaccag gacctgaccc tgctgaaggc cctggtgcgc 1020

cagcagctgc ccgagaagta caaggagatc ttcttcgacc agagcaagaa cggctacgcc 1080

ggctacatcg acggcggcgc cagccaggag gagttctaca agttcatcaa gcccatcctg 1140

gagaagatgg acggcaccga ggagctgctg gtgaagctga accgcgagga cctgctgcgc 1200

aagcagcgca ccttcgacaa cggcagcatc ccccaccaga tccacctggg cgagctgcac 1260

gccatcctgc gccgccagga ggacttctac cccttcctga aggacaaccg cgagaagatc 1320

gagaagatcc tgaccttccg catcccctac tacgtgggcc ccctggcccg cggcaacagc 1380

cgcttcgcct ggatgacccg caagagcgag gagaccatca ccccctggaa cttcgaggag 1440

gtggtggaca agggcgccag cgcccagagc ttcatcgagc gcatgaccaa cttcgacaag 1500

aacctgccca acgagaaggt gctgcccaag cacagcctgc tgtacgagta cttcaccgtg 1560

tacaacgagc tgaccaaggt gaagtacgtg accgagggca tgcgcaagcc cgccttcctg 1620

agcggcgagc agaagaaggc catcgtggac ctgctgttca agaccaaccg caaggtgacc 1680

gtgaagcagc tgaaggagga ctacttcaag aagatcgagt gcttcgacag cgtggagatc 1740

agcggcgtgg aggaccgctt caacgccagc ctgggcacct accacgacct gctgaagatc 1800

atcaaggaca aggacttcct ggacaacgag gagaacgagg acatcctgga ggacatcgtg 1860

ctgaccctga ccctgttcga ggaccgcgag atgatcgagg agcgcctgaa gacctacgcc 1920

cacctgttcg acgacaaggt gatgaagcag ctgaagcgcc gccgctacac cggctggggc 1980

cgcctgagcc gcaagcttat caacggcatc cgcgacaagc agagcggcaa gaccatcctg 2040

gacttcctga agagcgacgg cttcgccaac cgcaacttca tgcagctgat ccacgacgac 2100

agcctgacct tcaaggagga catccagaag gcccaggtga gcggccaggg cgacagcctg 2160

cacgagcaca tcgccaacct ggccggcagc cccgccatca agaagggcat cctgcagacc 2220

gtgaaggtgg tggacgagct ggtgaaggtg atgggccgcc acaagcccga gaacatcgtg 2280

atcgagatgg cccgcgagaa ccagaccacc cagaagggcc agaagaacag ccgcgagcgc 2340

atgaagcgca tcgaggaggg catcaaggag ctgggcagcc agatcctgaa ggagcacccc 2400

gtggagaaca cccagctgca gaacgagaag ctgtacctgt actacctgca gaacggccgc 2460

gacatgtacg tggaccagga gctggacatc aaccgcctga gcgactacga cgtggaccac 2520

atcgtgcccc agagcttcct gaaggacgac agcatcgaca acaaggtgct gacccgcagc 2580

gacaagaacc gcggcaagag cgacaacgtg cccagcgagg aggtggtgaa gaagatgaag 2640

aactactggc gccagctgct gaacgccaag ctgatcaccc agcgcaagtt cgacaacctg 2700

accaaggccg agcgcggcgg cctgagcgag ctggacaagg ccggcttcat caagcgccag 2760
 
ctggtggaga cccgccagat caccaagcac gtggcccaga tcctggacag ccgcatgaac 2820

accaagtacg acgagaacga caagctgatc cgcgaggtga aggtgatcac cctgaagagc 2880

aagctggtga gcgacttccg caaggacttc cagttctaca aggtgcgcga gatcaacaac 2940

taccaccacg cccacgacgc ctacctgaac gccgtggtgg gcaccgccct gatcaagaag 3000

taccccaagc tggagagcga gttcgtgtac ggcgactaca aggtgtacga cgtgcgcaag 3060

atgatcgcca agagcgagca ggagatcggc aaggccaccg ccaagtactt cttctacagc 3120

aacatcatga acttcttcaa gaccgagatc accctggcca acggcgagat ccgcaagcgc 3180

cccctgatcg agaccaacgg cgagaccggc gagatcgtgt gggacaaggg ccgcgacttc 3240

gccaccgtgc gcaaggtgct gagcatgccc caggtgaaca tcgtgaagaa gaccgaggtg 3300

cagaccggcg gcttcagcaa ggagagcatc ctgcccaagc gcaacagcga caagctgatc 3360

gcccgcaaga aggactggga ccccaagaag tacggcggct tcgacagccc caccgtggcc 3420

tacagcgtgc tggtggtggc caaggtggag aagggcaaga gcaagaagct gaagagcgtg 3480

aaggagctgc tgggcatcac catcatggag cgcagcagct tcgagaagaa ccccatcgac 3540

ttcctggagg ccaagggcta caaggaggtg aagaaggacc tgatcatcaa gctgcccaag 3600

tacagcctgt tcgagctgga gaacggccgc aagcgcatgc tggccagcgc cggcgagctg 3660

cagaagggca acgagctggc cctgcccagc aagtacgtga acttcctgta cctggccagc 3720

cactacgaga agctgaaggg cagccccgag gacaacgagc agaagcagct gttcgtggag 3780

cagcacaagc actacctgga cgagatcatc gagcagatca gcgagttcag caagcgcgtg 3840

atcctggccg acgccaacct ggacaaggtg ctgagcgcct acaacaagca ccgcgacaag 3900

cccatccgcg agcaggccga gaacatcatc cacctgttca ccctgaccaa cctgggcgcc 3960

cccgccgcct tcaagtactt cgacaccacc atcgaccgca agcgctacac cagcaccaag 4020

gaggtgctgg acgccaccct gatccaccag agcatcaccg gtctgtacga gacccgcatc 4080

gacctgagcc agctgggcgg cgactaa 4107
   <210> 2
      <211> 21
      <212> PRT
      <213> Artificial Sequence
   <220>
      <223> peptide tag
   <400> 2 
Gly Gly Ser Gly Pro Pro Lys Lys Lys Arg Lys Val Tyr Pro Tyr Asp
1               5                   10                  15

Val Pro Asp Tyr Ala
            20
   <210> 3
      <211> 34
      <212> DNA
      <213> Artificial Sequence
   <220>
      <223> F primer for CCR5
   <400> 3
      aattcatgac atcaattatt atacatcgga ggag 34
   <210> 4
      <211> 34
      <212> DNA
      <213> Artificial Sequence
   <220>
      <223> R primer for CCR5
   <400> 4
      gatcctcctc cgatgtataa taattgatgt catg 34
   <210> 5
      <211> 20
      <212> DNA
      <213> Artificial Sequence
   <220>
      <223> F1 primer for CCR5
   <400> 5
      ctccatggtg ctatagagca 20
   <210> 6
      <211> 21
      <212> DNA
      <213> Artificial Sequence
   <220>
      <223> F2 primer for CCR5
   <400> 6
      gagccaagct ctccatctag t 21
   <210> 7
      <211> 20
      <212> DNA
      <213> Artificial Sequence
   <220>
      <223> R primer for CCR5
   <400> 7
      gccctgtcaa gagttgacac 20
   <210> 8
      <211> 20
      <212> DNA
      <213> Artificial Sequence
   <220>
      <223> F1 primer for C4BPB
   <400> 8
      tatttggctg gttgaaaggg 20
   <210> 9
      <211> 24
      <212> DNA
      <213> Artificial Sequence
   <220>
      <223> R1 primer for C4BPB
   <400> 9
      aaagtcatga aataaacaca ccca 24
   <210> 10
      <211> 24
      <212> DNA
      <213> Artificial Sequence
   <220>
      <223> F2 primer for C4BPB
   <400> 10
      ctgcattgat atggtagtac catg 24
   <210> 11
      <211> 21
      <212> DNA
      <213> Artificial Sequence
   <220>
      <223> R2 primer for C4BPB
   <400> 11
      gctgttcatt gcaatggaat g 21
   <210> 12
      <211> 20
      <212> DNA
      <213> Artificial Sequence
   <220>
      <223> F1 primer for ADCY5
   <400> 12
      gctcccacct tagtgctctg 20
   <210> 13
      <211> 20
      <212> DNA
      <213> Artificial Sequence
   <220>
      <223> R1 primer for ADCY5
   <400> 13
      ggtggcagga acctgtatgt 20
   <210> 14
      <211> 21
      <212> DNA
      <213> Artificial Sequence
   <220>
      <223> F2 primer for ADCY5
   <400> 14
      gtcattggcc agagatgtgg a 21
   <210> 15
      <211> 20
      <212> DNA
      <213> Artificial Sequence
   <220>
      <223> R2 primer for ADCY5
   <400> 15
      gtcccatgac aggcgtgtat 20
   <210> 16
      <211> 20
      <212> DNA
      <213> Artificial Sequence
   <220>
      <223> F primer for KCNJ6
   <400> 16
      gcctggccaa gtttcagtta 20
   <210> 17
      <211> 20
      <212> DNA
      <213> Artificial Sequence
   <220>
      <223> R1 primer for KCNJ6
   <400> 17
      tggagccatt ggtttgcatc 20
   <210> 18
      <211> 22
      <212> DNA
      <213> Artificial Sequence
   <220>
      <223> R2 primer for KCNJ6
   <400> 18
      ccagaactaa gccgtttctg ac 22
   <210> 19
      <211> 20
      <212> DNA
      <213> Artificial Sequence
   <220>
      <223> F1 primer for CNTNAP2
   <400> 19
      atcaccgaca accagtttcc 20
   <210> 20
      <211> 20
      <212> DNA
      <213> Artificial Sequence
   <220>
      <223> F2 primer for CNTNAP2
   <400> 20
      tgcagtgcag actctttcca 20
   <210> 21
      <211> 20
      <212> DNA
      <213> Artificial Sequence
   <220>
      <223> R primer for CNTNAP2
   <400> 21
      aaggacacag ggcaactgaa 20
   <210> 22
      <211> 20
      <212> DNA
      <213> Artificial Sequence
   <220>
      <223> F1 primer for N/A Chr. 5
   <400> 22
      tgtggaacga gtggtgacag 20
   <210> 23
      <211> 22
      <212> DNA
      <213> Artificial Sequence
   <220>
      <223> R1 primer for N/A Chr. 5
   <400> 23
      gctggattag gaggcaggat tc 22
   <210> 24
      <211> 22
      <212> DNA
      <213> Artificial Sequence
   <220>
      <223> F2 primer for N/A Chr. 5
   <400> 24
      gtgctgagaa cgcttcatag ag 22
   <210> 25
      <211> 23
      <212> DNA
      <213> Artificial Sequence
   <220>
      <223> R2 primer for N/A Chr. 5
   <400> 25
      ggaccaaacc acattcttct cac 23
   <210> 26
      <211> 20
      <212> DNA
      <213> Artificial Sequence
   <220>
      <223> F primer for deletion
   <400> 26
      ccacatctcg ttctcggttt 20
   <210> 27
      <211> 20
      <212> DNA
      <213> Artificial Sequence
   <220>
      <223> R primer for deletion
   <400> 27
      tcacaagccc acagatattt 20
   <210> 28
      <211> 105
      <212> RNA
      <213> Artificial Sequence
   <220>
      <223> sgRNA for CCR5
   <400> 28 
ggugacauca auuauuauac auguuuuaga gcuagaaaua gcaaguuaaa auaaggcuag 60

uccguuauca acuugaaaaa guggcaccga gucggugcuu uuuuu 105
   <210> 29
      <211> 44
      <212> RNA
      <213> Artificial Sequence
   <220>
      <223> crRNA for CCR5
   <400> 29
      ggugacauca auuauuauac auguuuuaga gcuaugcugu uuug 44
   <210> 30
      <211> 86
      <212> RNA
      <213> Artificial Sequence
   <220>
      <223> tracrRNA for CCR5
   <400> 30 
ggaaccauuc aaaacagcau agcaaguuaa aauaaggcua guccguuauc aacuugaaaa 60

aguggcaccg agucggugcu uuuuuu 86
   <210> 31
      <211> 86
      <212> DNA
      <213> Artificial Sequence
   <220>
      <223> Foxn1 #1 sgRNA
   <400> 31 
gaaattaata cgactcacta taggcagtct gacgtcacac ttccgtttta gagctagaaa 60

tagcaagtta aaataaggct agtccg 86
   <210> 32
      <211> 86
      <212> DNA
      <213> Artificial Sequence
   <220>
      <223> Foxn1 #2 sgRNA
   <400> 32 
gaaattaata cgactcacta taggacttcc aggctccacc cgacgtttta gagctagaaa 60

tagcaagtta aaataaggct agtccg 86
   <210> 33
      <211> 86
      <212> DNA
      <213> Artificial Sequence
   <220>
      <223> Foxn1 #3 sgRNA
   <400> 33 
gaaattaata cgactcacta taggccaggc tccacccgac tggagtttta gagctagaaa 60

tagcaagtta aaataaggct agtccg 86
   <210> 34
      <211> 86
      <212> DNA
      <213> Artificial Sequence
   <220>
      <223> Foxn1 #4 sgRNA
   <400> 34 
gaaattaata cgactcacta taggactgga gggcgaaccc caaggtttta gagctagaaa 60

tagcaagtta aaataaggct agtccg 86
   <210> 35
      <211> 86
      <212> DNA
      <213> Artificial Sequence
   <220>
      <223> Foxn1 #5 sgRNA
   <400> 35 
gaaattaata cgactcacta taggacccca aggggacctc atgcgtttta gagctagaaa 60

tagcaagtta aaataaggct agtccg 86
   <210> 36
      <211> 86
      <212> DNA
      <213> Artificial Sequence
   <220>
      <223> Prkdc #1 sgRNA
   <400> 36 
gaaattaata cgactcacta taggttagtt ttttccagag acttgtttta gagctagaaa 60

tagcaagtta aaataaggct agtccg 86
   <210> 37
      <211> 86
      <212> DNA
      <213> Artificial Sequence
   <220>
      <223> Prkdc #2 sgRNA
   <400> 37 
gaaattaata cgactcacta taggttggtt tgcttgtgtt tatcgtttta gagctagaaa 60

tagcaagtta aaataaggct agtccg 86
   <210> 38
      <211> 86
      <212> DNA
      <213> Artificial Sequence
   <220>
      <223> Prkdc #3 sgRNA
   <400> 38 
gaaattaata cgactcacta taggcacaag caaaccaaag tctcgtttta gagctagaaa 60

tagcaagtta aaataaggct agtccg 86
   <210> 39
      <211> 86
      <212> DNA
      <213> Artificial Sequence
   <220>
      <223> Prkdc #4 sgRNA
   <400> 39 
gaaattaata cgactcacta taggcctcaa tgctaagcga cttcgtttta gagctagaaa 60

tagcaagtta aaataaggct agtccg 86
   <210> 40
      <211> 29
      <212> DNA
      <213> Artificial Sequence
   <220>
      <223> F1 primer for Foxn1
   <400> 40
      gtctgtctat catctcttcc cttctctcc 29
   <210> 41
      <211> 25
      <212> DNA
      <213> Artificial Sequence
   <220>
      <223> F2 primer for Foxn1
   <400> 41
      tccctaatcc gatggctagc tccag 25
   <210> 42
      <211> 23
      <212> DNA
      <213> Artificial Sequence
   <220>
      <223> R1 primer for Foxn1
   <400> 42
      acgagcagct gaagttagca tgc 23
   <210> 43
      <211> 32
      <212> DNA
      <213> Artificial Sequence
   <220>
      <223> R2 primer for Foxn1
   <400> 43
      ctactcaatg ctcttagagc taccaggctt gc 32
   <210> 44
      <211> 20
      <212> DNA
      <213> Artificial Sequence
   <220>
      <223> F primer for Prkdc
   <400> 44
      gactgttgtg gggagggccg 20
   <210> 45
      <211> 24
      <212> DNA
      <213> Artificial Sequence
   <220>
      <223> F2 primer for Prkdc
   <400> 45
      gggagggccg aaagtcttat tttg 24
   <210> 46
      <211> 28
      <212> DNA
      <213> Artificial Sequence
   <220>
      <223> R1 primer for Prkdc
   <400> 46
      cctgaagact gaagttggca gaagtgag 28
   <210> 47
      <211> 27
      <212> DNA
      <213> Artificial Sequence
   <220>
      <223> R2 primer for Prkdc
   <400> 47
      ctttagggct tcttctctac aatcacg 27
   <210> 48
      <211> 38
      <212> DNA
      <213> Artificial Sequence
   <220>
      <223> F primer for Foxn1
   <400> 48
      ctcggtgtgt agccctgacc tcggtgtgta gccctgac 38
   <210> 49
      <211> 21
      <212> DNA
      <213> Artificial Sequence
   <220>
      <223> R primer for Foxn1
   <400> 49
      agactggcct ggaactcaca g 21
   <210> 50
      <211> 23
      <212> DNA
      <213> Artificial Sequence
   <220>
      <223> F primer for Foxn1
   <400> 50
      cactaaagcc tgtcaggaag ccg 23
   <210> 51
      <211> 21
      <212> DNA
      <213> Artificial Sequence
   <220>
      <223> R primer for Foxn1
   <400> 51
      ctgtggagag cacacagcag c 21
   <210> 52
      <211> 19
      <212> DNA
      <213> Artificial Sequence
   <220>
      <223> F primer for Foxn1
   <400> 52
      gctgcgacct gagaccatg 19
   <210> 53
      <211> 26
      <212> DNA
      <213> Artificial Sequence
   <220>
      <223> R primer for Foxn1
   <400> 53
      cttcaatggc ttcctgctta ggctac 26
   <210> 54
      <211> 23
      <212> DNA
      <213> Artificial Sequence
   <220>
      <223> F primer for Foxn1
   <400> 54
      ggttcagatg aggccatcct ttc 23
   <210> 55
      <211> 24
      <212> DNA
      <213> Artificial Sequence
   <220>
      <223> R primer for Foxn1
   <400> 55
      cctgatctgc aggcttaacc cttg 24
   <210> 56
      <211> 22
      <212> DNA
      <213> Artificial Sequence
   <220>
      <223> F primer for Prkdc
   <400> 56
      ctcacctgca catcacatgt gg 22
   <210> 57
      <211> 20
      <212> DNA
      <213> Artificial Sequence
   <220>
      <223> R primer for Prkdc
   <400> 57
      ggcatccacc ctatggggtc 20
   <210> 58
      <211> 25
      <212> DNA
      <213> Artificial Sequence
   <220>
      <223> F primer for Prkdc
   <400> 58
      gccttgacct agagcttaaa gagcc 25
   <210> 59
      <211> 25
      <212> DNA
      <213> Artificial Sequence
   <220>
      <223> R primer for Prkdc
   <400> 59
      ggtcttgtta gcaggaagga cactg 25
   <210> 60
      <211> 27
      <212> DNA
      <213> Artificial Sequence
   <220>
      <223> F primer for Prkdc
   <400> 60
      aaaactctgc ttgatgggat atgtggg 27
   <210> 61
      <211> 26
      <212> DNA
      <213> Artificial Sequence
   <220>
      <223> R primer for Prkdc
   <400> 61
      ctctcactgg ttatctgtgc tccttc 26
   <210> 62
      <211> 23
      <212> DNA
      <213> Artificial Sequence
   <220>
      <223> F primer for Prkdc
   <400> 62
      ggatcaatag gtggtggggg atg 23
   <210> 63
      <211> 27
      <212> DNA
      <213> Artificial Sequence
   <220>
      <223> R primer for Prkdc
   <400> 63
      gtgaatgaca caatgtgaca gcttcag 27
   <210> 64
      <211> 28
      <212> DNA
      <213> Artificial Sequence
   <220>
      <223> F primer for Prkdc
   <400> 64
      cacaagacag acctctcaac attcagtc 28
   <210> 65
      <211> 32
      <212> DNA
      <213> Artificial Sequence
   <220>
      <223> R primer for Prkdc
   <400> 65
      gtgcatgcat ataatccatt ctgattgctc tc 32
   <210> 66
      <211> 17
      <212> DNA
      <213> Artificial Sequence
   <220>
      <223> F1 primer for Prkdc
   <400> 66
      gggaggcaga ggcaggt 17
   <210> 67
      <211> 23
      <212> DNA
      <213> Artificial Sequence
   <220>
      <223> F2 primer for Prkdc
   <400> 67
      ggatctctgt gagtttgagg cca 23
   <210> 68
      <211> 24
      <212> DNA
      <213> Artificial Sequence
   <220>
      <223> R1 primer for Prkdc
   <400> 68
      gctccagaac tcactcttag gctc 24
   <210> 69
      <211> 20
      <212> DNA
      <213> Artificial Sequence
   <220>
      <223> Primer for Foxn1
   <400> 69
      ctactccctc cgcagtctga 20
   <210> 70
      <211> 20
      <212> DNA
      <213> Artificial Sequence
   <220>
      <223> Primer for Foxn1
   <400> 70
      ccaggcctag gttccaggta 20
   <210> 71
      <211> 20
      <212> DNA
      <213> Artificial Sequence
   <220>
      <223> Primer for Prkdc
   <400> 71
      ccccagcatt gcagatttcc 20
   <210> 72
      <211> 23
      <212> DNA
      <213> Artificial Sequence
   <220>
      <223> Primer for Prkdc
   <400> 72
      agggcttctt ctctacaatc acg 23
   <210> 73
      <211> 86
      <212> DNA
      <213> Artificial Sequence
   <220>
      <223> BRI1 target 1
   <400> 73 
gaaattaata cgactcacta taggtttgaa agatggaagc gcgggtttta gagctagaaa 60

tagcaagtta aaataaggct agtccg 86
   <210> 74
      <211> 86
      <212> DNA
      <213> Artificial Sequence
   <220>
      <223> BRI1 target 2
   <400> 74 
gaaattaata cgactcacta taggtgaaac taaactggtc cacagtttta gagctagaaa 60

tagcaagtta aaataaggct agtccg 86
   <210> 75
      <211> 64
      <212> DNA
      <213> Artificial Sequence
   <220>
      <223> Universal
   <400> 75 
aaaaaagcac cgactcggtg ccactttttc aagttgataa cggactagcc ttattttaac 60

ttgc 64
   <210> 76
      <211> 65
      <212> DNA
      <213> Artificial Sequence
   <220>
      <223> Templates for crRNA
   <400> 76 
gaaattaata cgactcacta taggnnnnnn nnnnnnnnnn nnnngtttta gagctatgct 60

gtttt 65
   <210> 77
      <211> 67
      <212> DNA
      <213> Artificial Sequence
   <220>
      <223> tracrRNA
   <400> 77 
gaaattaata cgactcacta taggaaccat tcaaaacagc atagcaagtt aaaataaggc 60

tagtccg 67
   <210> 78
      <211> 69
      <212> DNA
      <213> Artificial Sequence
   <220>
      <223> tracrRNA
   <400> 78 
aaaaaaagca ccgactcggt gccacttttt caagttgata acggactagc cttattttaa 60

cttgctatg 69
   <210> 79
      <211> 20
      <212> DNA
      <213> Artificial Sequence
   <220>
      <223> Primer
   <400> 79
      ctccatggtg ctatagagca 20
   <210> 80
      <211> 21
      <212> DNA
      <213> Artificial Sequence
   <220>
      <223> Primer
   <400> 80
      gagccaagct ctccatctag t 21
   <210> 81
      <211> 20
      <212> DNA
      <213> Artificial Sequence
   <220>
      <223> Primer
   <400> 81
      gccctgtcaa gagttgacac 20
   <210> 82
      <211> 22
      <212> DNA
      <213> Artificial Sequence
   <220>
      <223> Primer
   <400> 82
      gcacagggtg gaacaagatg ga 22
   <210> 83
      <211> 24
      <212> DNA
      <213> Artificial Sequence
   <220>
      <223> Primer
   <400> 83
      gccaggtacc tatcgattgt cagg 24
   <210> 84
      <211> 21
      <212> DNA
      <213> Artificial Sequence
   <220>
      <223> Primer
   <400> 84
      gagccaagct ctccatctag t 21
   <210> 85
      <211> 20
      <212> DNA
      <213> Artificial Sequence
   <220>
      <223> Primer
   <400> 85
      actctgactg ggtcaccagc 20
   <210> 86
      <211> 20
      <212> DNA
      <213> Artificial Sequence
   <220>
      <223> Primer
   <400> 86
      tatttggctg gttgaaaggg 20
   <210> 87
      <211> 24
      <212> DNA
      <213> Artificial Sequence
   <220>
      <223> Primer
   <400> 87
      aaagtcatga aataaacaca ccca 24
   <210> 88
      <211> 24
      <212> DNA
      <213> Artificial Sequence
   <220>
      <223> Primer
   <400> 88
      ctgcattgat atggtagtac catg 24
   <210> 89
      <211> 21
      <212> DNA
      <213> Artificial Sequence
   <220>
      <223> Primer
   <400> 89
      gctgttcatt gcaatggaat g 21
   <210> 90
      <211> 22
      <212> DNA
      <213> Artificial Sequence
   <220>
      <223> Primer
   <400> 90
      atggagttgg acatggccat gg 22
   <210> 91
      <211> 28
      <212> DNA
      <213> Artificial Sequence
   <220>
      <223> Primer
   <400> 91
      actcactatc cacagttcag catttacc 28
   <210> 92
      <211> 23
      <212> DNA
      <213> Artificial Sequence
   <220>
      <223> Primer
   <400> 92
      tggagatagc tgtcagcaac ttt 23
   <210> 93
      <211> 29
      <212> DNA
      <213> Artificial Sequence
   <220>
      <223> Primer
   <400> 93
      caacaaagca aaggtaaagt tggtaatag 29
   <210> 94
      <211> 25
      <212> DNA
      <213> Artificial Sequence
   <220>
      <223> Primer
   <400> 94
      ggtttcagga gatgtgttac aaggc 25
   <210> 95
      <211> 27
      <212> DNA
      <213> Artificial Sequence
   <220>
      <223> Primer
   <400> 95
      gattgtgcaa ttcctatgca atcggtc 27
   <210> 96
      <211> 25
      <212> DNA
      <213> Artificial Sequence
   <220>
      <223> Primer
   <400> 96
      cactgggtac ttaatctgta gcctc 25
   <210> 97
      <211> 23
      <212> DNA
      <213> Artificial Sequence
   <220>
      <223> Primer
   <400> 97
      ggttccaagt cattcccagt agc 23
   <210> 98
      <211> 30
      <212> DNA
      <213> Artificial Sequence
   <220>
      <223> Primer
   <400> 98
      catcactgca gttgtaggtt ataactatcc 30
   <210> 99
      <211> 26
      <212> DNA
      <213> Artificial Sequence
   <220>
      <223> Primer
   <400> 99
      ttgaaaacca cagatctggt tgaacc 26
   <210> 100
      <211> 22
      <212> DNA
      <213> Artificial Sequence
   <220>
      <223> Primer
   <400> 100
      ggagtgccaa gagaatatct gg 22
   <210> 101
      <211> 32
      <212> DNA
      <213> Artificial Sequence
   <220>
      <223> Primer
   <400> 101
      ctgaaactgg tttcaaaata ttcgttttaa gg 32
   <210> 102
      <211> 22
      <212> DNA
      <213> Artificial Sequence
   <220>
      <223> Primer
   <400> 102
      gctctgtatg ccctgtagta gg 22
   <210> 103
      <211> 22
      <212> DNA
      <213> Artificial Sequence
   <220>
      <223> Primer
   <400> 103
      tttgcatctg accttacctt tg 22
   <210> 104
      <211> 23
      <212> DNA
      <213> Artificial Sequence
   <220>
      <223> Target sequence of RGEN
   <400> 104
      aatgaccact acatcctcaa ggg 23
   <210> 105
      <211> 23
      <212> DNA
      <213> Artificial Sequence
   <220>
      <223> Target sequence of RGEN
   <400> 105
      agatgatgtc tcatcatcag agg 23
   <210> 106
      <211> 4170
      <212> DNA
      <213> Artificial Sequence
   <220>
      <223> Cas9-coding sequence in p3s-Cas9HC (humanized, C-term tagging, human cell experiments)
   <400> 106 atggacaaga agtacagcat cggcctggac atcggtacca acagcgtggg ctgggccgtg 60 
atcaccgacg agtacaaggt gcccagcaag aagttcaagg tgctgggcaa caccgaccgc 120

cacagcatca agaagaacct gatcggcgcc ctgctgttcg acagcggcga gaccgccgag 180

gccacccgcc tgaagcgcac cgcccgccgc cgctacaccc gccgcaagaa ccgcatctgc 240

tacctgcagg agatcttcag caacgagatg gccaaggtgg acgacagctt cttccaccgc 300

ctggaggaga gcttcctggt ggaggaggac aagaagcacg agcgccaccc catcttcggc 360

aacatcgtgg acgaggtggc ctaccacgag aagtacccca ccatctacca cctgcgcaag 420

aagctggtgg acagcaccga caaggccgac ctgcgcctga tctacctggc cctggcccac 480

atgatcaagt tccgcggcca cttcctgatc gagggcgacc tgaaccccga caacagcgac 540

gtggacaagc tgttcatcca gctggtgcag acctacaacc agctgttcga ggagaacccc 600

atcaacgcca gcggcgtgga cgccaaggcc atcctgagcg cccgcctgag caagagccgc 660

cgcctggaga acctgatcgc ccagctgccc ggcgagaaga agaacggcct gttcggcaac 720

ctgatcgccc tgagcctggg cctgaccccc aacttcaaga gcaacttcga cctggccgag 780

gacgccaagc tgcagctgag caaggacacc tacgacgacg acctggacaa cctgctggcc 840

cagatcggcg accagtacgc cgacctgttc ctggccgcca agaacctgag cgacgccatc 900

ctgctgagcg acatcctgcg cgtgaacacc gagatcacca aggcccccct gagcgccagc 960

atgatcaagc gctacgacga gcaccaccag gacctgaccc tgctgaaggc cctggtgcgc 1020

cagcagctgc ccgagaagta caaggagatc ttcttcgacc agagcaagaa cggctacgcc 1080

ggctacatcg acggcggcgc cagccaggag gagttctaca agttcatcaa gcccatcctg 1140

gagaagatgg acggcaccga ggagctgctg gtgaagctga accgcgagga cctgctgcgc 1200

aagcagcgca ccttcgacaa cggcagcatc ccccaccaga tccacctggg cgagctgcac 1260

gccatcctgc gccgccagga ggacttctac cccttcctga aggacaaccg cgagaagatc 1320

gagaagatcc tgaccttccg catcccctac tacgtgggcc ccctggcccg cggcaacagc 1380

cgcttcgcct ggatgacccg caagagcgag gagaccatca ccccctggaa cttcgaggag 1440

gtggtggaca agggcgccag cgcccagagc ttcatcgagc gcatgaccaa cttcgacaag 1500

aacctgccca acgagaaggt gctgcccaag cacagcctgc tgtacgagta cttcaccgtg 1560

tacaacgagc tgaccaaggt gaagtacgtg accgagggca tgcgcaagcc cgccttcctg 1620

agcggcgagc agaagaaggc catcgtggac ctgctgttca agaccaaccg caaggtgacc 1680

gtgaagcagc tgaaggagga ctacttcaag aagatcgagt gcttcgacag cgtggagatc 1740

agcggcgtgg aggaccgctt caacgccagc ctgggcacct accacgacct gctgaagatc 1800

atcaaggaca aggacttcct ggacaacgag gagaacgagg acatcctgga ggacatcgtg 1860

ctgaccctga ccctgttcga ggaccgcgag atgatcgagg agcgcctgaa gacctacgcc 1920
 
cacctgttcg acgacaaggt gatgaagcag ctgaagcgcc gccgctacac cggctggggc 1980
cgcctgagcc gcaagcttat caacggcatc cgcgacaagc agagcggcaa gaccatcctg 2040
gacttcctga agagcgacgg cttcgccaac cgcaacttca tgcagctgat ccacgacgac 2100
agcctgacct tcaaggagga catccagaag gcccaggtga gcggccaggg cgacagcctg 2160
cacgagcaca tcgccaacct ggccggcagc cccgccatca agaagggcat cctgcagacc 2220
gtgaaggtgg tggacgagct ggtgaaggtg atgggccgcc acaagcccga gaacatcgtg 2280
atcgagatgg cccgcgagaa ccagaccacc cagaagggcc agaagaacag ccgcgagcgc 2340
atgaagcgca tcgaggaggg catcaaggag ctgggcagcc agatcctgaa ggagcacccc 2400
gtggagaaca cccagctgca gaacgagaag ctgtacctgt actacctgca gaacggccgc 2460
gacatgtacg tggaccagga gctggacatc aaccgcctga gcgactacga cgtggaccac 2520
atcgtgcccc agagcttcct gaaggacgac agcatcgaca acaaggtgct gacccgcagc 2580
gacaagaacc gcggcaagag cgacaacgtg cccagcgagg aggtggtgaa gaagatgaag 2640
aactactggc gccagctgct gaacgccaag ctgatcaccc agcgcaagtt cgacaacctg 2700
accaaggccg agcgcggcgg cctgagcgag ctggacaagg ccggcttcat caagcgccag 2760
ctggtggaga cccgccagat caccaagcac gtggcccaga tcctggacag ccgcatgaac 2820
accaagtacg acgagaacga caagctgatc cgcgaggtga aggtgatcac cctgaagagc 2880
aagctggtga gcgacttccg caaggacttc cagttctaca aggtgcgcga gatcaacaac 2940
taccaccacg cccacgacgc ctacctgaac gccgtggtgg gcaccgccct gatcaagaag 3000
taccccaagc tggagagcga gttcgtgtac ggcgactaca aggtgtacga cgtgcgcaag 3060
atgatcgcca agagcgagca ggagatcggc aaggccaccg ccaagtactt cttctacagc 3120
aacatcatga acttcttcaa gaccgagatc accctggcca acggcgagat ccgcaagcgc 3180
cccctgatcg agaccaacgg cgagaccggc gagatcgtgt gggacaaggg ccgcgacttc 3240
gccaccgtgc gcaaggtgct gagcatgccc caggtgaaca tcgtgaagaa gaccgaggtg 3300
cagaccggcg gcttcagcaa ggagagcatc ctgcccaagc gcaacagcga caagctgatc 3360
gcccgcaaga aggactggga ccccaagaag tacggcggct tcgacagccc caccgtggcc 3420
tacagcgtgc tggtggtggc caaggtggag aagggcaaga gcaagaagct gaagagcgtg 3480
aaggagctgc tgggcatcac catcatggag cgcagcagct tcgagaagaa ccccatcgac 3540
ttcctggagg ccaagggcta caaggaggtg aagaaggacc tgatcatcaa gctgcccaag 3600
tacagcctgt tcgagctgga gaacggccgc aagcgcatgc tggccagcgc cggcgagctg 3660
cagaagggca acgagctggc cctgcccagc aagtacgtga acttcctgta cctggccagc 3720
cactacgaga agctgaaggg cagccccgag gacaacgagc agaagcagct gttcgtggag 3780
cagcacaagc actacctgga cgagatcatc gagcagatca gcgagttcag caagcgcgtg 3840
 
atcctggccg acgccaacct ggacaaggtg ctgagcgcct acaacaagca ccgcgacaag 3900

cccatccgcg agcaggccga gaacatcatc cacctgttca ccctgaccaa cctgggcgcc 3960

cccgccgcct tcaagtactt cgacaccacc atcgaccgca agcgctacac cagcaccaag 4020

gaggtgctgg acgccaccct gatccaccag agcatcaccg gtctgtacga gacccgcatc 4080

gacctgagcc agctgggcgg cgacggcggc tccggacctc caaagaaaaa gagaaaagta 4140

tacccctacg acgtgcccga ctacgcctaa 4170
   <210> 107
      <211> 4194
      <212> DNA
      <213> Artificial Sequence
   <220>
      <223> Cas9 coding sequence in p3s-Cas9HN (humanized codon, N-term tagging (underlined), human cell experiments)
   <400> 107 
atggtgtacc cctacgacgt gcccgactac gccgaattgc ctccaaaaaa gaagagaaag 60

gtagggatcc gaattcccgg ggaaaaaccg gacaagaagt acagcatcgg cctggacatc 120

ggtaccaaca gcgtgggctg ggccgtgatc accgacgagt acaaggtgcc cagcaagaag 180

ttcaaggtgc tgggcaacac cgaccgccac agcatcaaga agaacctgat cggcgccctg 240

ctgttcgaca gcggcgagac cgccgaggcc acccgcctga agcgcaccgc ccgccgccgc 300

tacacccgcc gcaagaaccg catctgctac ctgcaggaga tcttcagcaa cgagatggcc 360

aaggtggacg acagcttctt ccaccgcctg gaggagagct tcctggtgga ggaggacaag 420

aagcacgagc gccaccccat cttcggcaac atcgtggacg aggtggccta ccacgagaag 480

taccccacca tctaccacct gcgcaagaag ctggtggaca gcaccgacaa ggccgacctg 540

cgcctgatct acctggccct ggcccacatg atcaagttcc gcggccactt cctgatcgag 600

ggcgacctga accccgacaa cagcgacgtg gacaagctgt tcatccagct ggtgcagacc 660

tacaaccagc tgttcgagga gaaccccatc aacgccagcg gcgtggacgc caaggccatc 720

ctgagcgccc gcctgagcaa gagccgccgc ctggagaacc tgatcgccca gctgcccggc 780

gagaagaaga acggcctgtt cggcaacctg atcgccctga gcctgggcct gacccccaac 840

ttcaagagca acttcgacct ggccgaggac gccaagctgc agctgagcaa ggacacctac 900

gacgacgacc tggacaacct gctggcccag atcggcgacc agtacgccga cctgttcctg 960

gccgccaaga acctgagcga cgccatcctg ctgagcgaca tcctgcgcgt gaacaccgag 1020

atcaccaagg cccccctgag cgccagcatg atcaagcgct acgacgagca ccaccaggac 1080

ctgaccctgc tgaaggccct ggtgcgccag cagctgcccg agaagtacaa ggagatcttc 1140
 
ttcgaccaga gcaagaacgg ctacgccggc tacatcgacg gcggcgccag ccaggaggag 1200

ttctacaagt tcatcaagcc catcctggag aagatggacg gcaccgagga gctgctggtg 1260

aagctgaacc gcgaggacct gctgcgcaag cagcgcacct tcgacaacgg cagcatcccc 1320

caccagatcc acctgggcga gctgcacgcc atcctgcgcc gccaggagga cttctacccc 1380

ttcctgaagg acaaccgcga gaagatcgag aagatcctga ccttccgcat cccctactac 1440

gtgggccccc tggcccgcgg caacagccgc ttcgcctgga tgacccgcaa gagcgaggag 1500

accatcaccc cctggaactt cgaggaggtg gtggacaagg gcgccagcgc ccagagcttc 1560

atcgagcgca tgaccaactt cgacaagaac ctgcccaacg agaaggtgct gcccaagcac 1620

agcctgctgt acgagtactt caccgtgtac aacgagctga ccaaggtgaa gtacgtgacc 1680

gagggcatgc gcaagcccgc cttcctgagc ggcgagcaga agaaggccat cgtggacctg 1740

ctgttcaaga ccaaccgcaa ggtgaccgtg aagcagctga aggaggacta cttcaagaag 1800

atcgagtgct tcgacagcgt ggagatcagc ggcgtggagg accgcttcaa cgccagcctg 1860

ggcacctacc acgacctgct gaagatcatc aaggacaagg acttcctgga caacgaggag 1920

aacgaggaca tcctggagga catcgtgctg accctgaccc tgttcgagga ccgcgagatg 1980

atcgaggagc gcctgaagac ctacgcccac ctgttcgacg acaaggtgat gaagcagctg 2040

aagcgccgcc gctacaccgg ctggggccgc ctgagccgca agcttatcaa cggcatccgc 2100

gacaagcaga gcggcaagac catcctggac ttcctgaaga gcgacggctt cgccaaccgc 2160

aacttcatgc agctgatcca cgacgacagc ctgaccttca aggaggacat ccagaaggcc 2220

caggtgagcg gccagggcga cagcctgcac gagcacatcg ccaacctggc cggcagcccc 2280

gccatcaaga agggcatcct gcagaccgtg aaggtggtgg acgagctggt gaaggtgatg 2340

ggccgccaca agcccgagaa catcgtgatc gagatggccc gcgagaacca gaccacccag 2400

aagggccaga agaacagccg cgagcgcatg aagcgcatcg aggagggcat caaggagctg 2460

ggcagccaga tcctgaagga gcaccccgtg gagaacaccc agctgcagaa cgagaagctg 2520

tacctgtact acctgcagaa cggccgcgac atgtacgtgg accaggagct ggacatcaac 2580

cgcctgagcg actacgacgt ggaccacatc gtgccccaga gcttcctgaa ggacgacagc 2640

atcgacaaca aggtgctgac ccgcagcgac aagaaccgcg gcaagagcga caacgtgccc 2700

agcgaggagg tggtgaagaa gatgaagaac tactggcgcc agctgctgaa cgccaagctg 2760

atcacccagc gcaagttcga caacctgacc aaggccgagc gcggcggcct gagcgagctg 2820

gacaaggccg gcttcatcaa gcgccagctg gtggagaccc gccagatcac caagcacgtg 2880

gcccagatcc tggacagccg catgaacacc aagtacgacg agaacgacaa gctgatccgc 2940

gaggtgaagg tgatcaccct gaagagcaag ctggtgagcg acttccgcaa ggacttccag 3000

ttctacaagg tgcgcgagat caacaactac caccacgccc acgacgccta cctgaacgcc 3060
 
gtggtgggca ccgccctgat caagaagtac cccaagctgg agagcgagtt cgtgtacggc 3120

gactacaagg tgtacgacgt gcgcaagatg atcgccaaga gcgagcagga gatcggcaag 3180

gccaccgcca agtacttctt ctacagcaac atcatgaact tcttcaagac cgagatcacc 3240

ctggccaacg gcgagatccg caagcgcccc ctgatcgaga ccaacggcga gaccggcgag 3300

atcgtgtggg acaagggccg cgacttcgcc accgtgcgca aggtgctgag catgccccag 3360

gtgaacatcg tgaagaagac cgaggtgcag accggcggct tcagcaagga gagcatcctg 3420

cccaagcgca acagcgacaa gctgatcgcc cgcaagaagg actgggaccc caagaagtac 3480

ggcggcttcg acagccccac cgtggcctac agcgtgctgg tggtggccaa ggtggagaag 3540

ggcaagagca agaagctgaa gagcgtgaag gagctgctgg gcatcaccat catggagcgc 3600

agcagcttcg agaagaaccc catcgacttc ctggaggcca agggctacaa ggaggtgaag 3660

aaggacctga tcatcaagct gcccaagtac agcctgttcg agctggagaa cggccgcaag 3720

cgcatgctgg ccagcgccgg cgagctgcag aagggcaacg agctggccct gcccagcaag 3780

tacgtgaact tcctgtacct ggccagccac tacgagaagc tgaagggcag ccccgaggac 3840

aacgagcaga agcagctgtt cgtggagcag cacaagcact acctggacga gatcatcgag 3900

cagatcagcg agttcagcaa gcgcgtgatc ctggccgacg ccaacctgga caaggtgctg 3960

agcgcctaca acaagcaccg cgacaagccc atccgcgagc aggccgagaa catcatccac 4020

ctgttcaccc tgaccaacct gggcgccccc gccgccttca agtacttcga caccaccatc 4080

gaccgcaagc gctacaccag caccaaggag gtgctggacg ccaccctgat ccaccagagc 4140

atcaccggtc tgtacgagac ccgcatcgac ctgagccagc tgggcggcga ctaa 4194
   <210> 108
      <211> 4107
      <212> DNA
      <213> Artificial Sequence
   <220>
      <223> Cas9-coding sequence in Streptococcus pyogenes
   <400> 108 
atggataaga aatactcaat aggcttagat atcggcacaa atagcgtcgg atgggcggtg 60

atcactgatg aatataaggt tccgtctaaa aagttcaagg ttctgggaaa tacagaccgc 120

cacagtatca aaaaaaatct tataggggct cttttatttg acagtggaga gacagcggaa 180

gcgactcgtc tcaaacggac agctcgtaga aggtatacac gtcggaagaa tcgtatttgt 240

tatctacagg agattttttc aaatgagatg gcgaaagtag atgatagttt ctttcatcga 300

cttgaagagt cttttttggt ggaagaagac aagaagcatg aacgtcatcc tatttttgga 360

aatatagtag atgaagttgc ttatcatgag aaatatccaa ctatctatca tctgcgaaaa 420
 
aaattggtag attctactga taaagcggat ttgcgcttaa tctatttggc cttagcgcat 480

atgattaagt ttcgtggtca ttttttgatt gagggagatt taaatcctga taatagtgat 540

gtggacaaac tatttatcca gttggtacaa acctacaatc aattatttga agaaaaccct 600

attaacgcaa gtggagtaga tgctaaagcg attctttctg cacgattgag taaatcaaga 660

cgattagaaa atctcattgc tcagctcccc ggtgagaaga aaaatggctt atttgggaat 720

ctcattgctt tgtcattggg tttgacccct aattttaaat caaattttga tttggcagaa 780

gatgctaaat tacagctttc aaaagatact tacgatgatg atttagataa tttattggcg 840

caaattggag atcaatatgc tgatttgttt ttggcagcta agaatttatc agatgctatt 900

ttactttcag atatcctaag agtaaatact gaaataacta aggctcccct atcagcttca 960

atgattaaac gctacgatga acatcatcaa gacttgactc ttttaaaagc tttagttcga 1020

caacaacttc cagaaaagta taaagaaatc ttttttgatc aatcaaaaaa cggatatgca 1080

ggttatattg atgggggagc tagccaagaa gaattttata aatttatcaa accaatttta 1140

gaaaaaatgg atggtactga ggaattattg gtgaaactaa atcgtgaaga tttgctgcgc 1200

aagcaacgga cctttgacaa cggctctatt ccccatcaaa ttcacttggg tgagctgcat 1260

gctattttga gaagacaaga agacttttat ccatttttaa aagacaatcg tgagaagatt 1320

gaaaaaatct tgacttttcg aattccttat tatgttggtc cattggcgcg tggcaatagt 1380

cgttttgcat ggatgactcg gaagtctgaa gaaacaatta ccccatggaa ttttgaagaa 1440

gttgtcgata aaggtgcttc agctcaatca tttattgaac gcatgacaaa ctttgataaa 1500

aatcttccaa atgaaaaagt actaccaaaa catagtttgc tttatgagta ttttacggtt 1560

tataacgaat tgacaaaggt caaatatgtt actgaaggaa tgcgaaaacc agcatttctt 1620

tcaggtgaac agaagaaagc cattgttgat ttactcttca aaacaaatcg aaaagtaacc 1680

gttaagcaat taaaagaaga ttatttcaaa aaaatagaat gttttgatag tgttgaaatt 1740

tcaggagttg aagatagatt taatgcttca ttaggtacct accatgattt gctaaaaatt 1800

attaaagata aagatttttt ggataatgaa gaaaatgaag atatcttaga ggatattgtt 1860

ttaacattga ccttatttga agatagggag atgattgagg aaagacttaa aacatatgct 1920

cacctctttg atgataaggt gatgaaacag cttaaacgtc gccgttatac tggttgggga 1980

cgtttgtctc gaaaattgat taatggtatt agggataagc aatctggcaa aacaatatta 2040

gattttttga aatcagatgg ttttgccaat cgcaatttta tgcagctgat ccatgatgat 2100

agtttgacat ttaaagaaga cattcaaaaa gcacaagtgt ctggacaagg cgatagttta 2160

catgaacata ttgcaaattt agctggtagc cctgctatta aaaaaggtat tttacagact 2220

gtaaaagttg ttgatgaatt ggtcaaagta atggggcggc ataagccaga aaatatcgtt 2280
 
attgaaatgg cacgtgaaaa tcagacaact caaaagggcc agaaaaattc gcgagagcgt 2340

atgaaacgaa tcgaagaagg tatcaaagaa ttaggaagtc agattcttaa agagcatcct 2400

gttgaaaata ctcaattgca aaatgaaaag ctctatctct attatctcca aaatggaaga 2460

gacatgtatg tggaccaaga attagatatt aatcgtttaa gtgattatga tgtcgatcac 2520

attgttccac aaagtttcct taaagacgat tcaatagaca ataaggtctt aacgcgttct 2580

gataaaaatc gtggtaaatc ggataacgtt ccaagtgaag aagtagtcaa aaagatgaaa 2640

aactattgga gacaacttct aaacgccaag ttaatcactc aacgtaagtt tgataattta 2700

acgaaagctg aacgtggagg tttgagtgaa cttgataaag ctggttttat caaacgccaa 2760

ttggttgaaa ctcgccaaat cactaagcat gtggcacaaa ttttggatag tcgcatgaat 2820

actaaatacg atgaaaatga taaacttatt cgagaggtta aagtgattac cttaaaatct 2880

aaattagttt ctgacttccg aaaagatttc caattctata aagtacgtga gattaacaat 2940

taccatcatg cccatgatgc gtatctaaat gccgtcgttg gaactgcttt gattaagaaa 3000

tatccaaaac ttgaatcgga gtttgtctat ggtgattata aagtttatga tgttcgtaaa 3060

atgattgcta agtctgagca agaaataggc aaagcaaccg caaaatattt cttttactct 3120

aatatcatga acttcttcaa aacagaaatt acacttgcaa atggagagat tcgcaaacgc 3180

cctctaatcg aaactaatgg ggaaactgga gaaattgtct gggataaagg gcgagatttt 3240

gccacagtgc gcaaagtatt gtccatgccc caagtcaata ttgtcaagaa aacagaagta 3300

cagacaggcg gattctccaa ggagtcaatt ttaccaaaaa gaaattcgga caagcttatt 3360

gctcgtaaaa aagactggga tccaaaaaaa tatggtggtt ttgatagtcc aacggtagct 3420

tattcagtcc tagtggttgc taaggtggaa aaagggaaat cgaagaagtt aaaatccgtt 3480

aaagagttac tagggatcac aattatggaa agaagttcct ttgaaaaaaa tccgattgac 3540

tttttagaag ctaaaggata taaggaagtt aaaaaagact taatcattaa actacctaaa 3600

tatagtcttt ttgagttaga aaacggtcgt aaacggatgc tggctagtgc cggagaatta 3660

caaaaaggaa atgagctggc tctgccaagc aaatatgtga atttttatat tttagctagt 3720

cattatgaaa agttgaaggg tagtccagaa gataacgaac aaaaacaatt gtttgtggag 3780

cagcataagc attatttaga tgagattatt gagcaaatca gtgaattttc taagcgtgtt 3840

attttagcag atgccaattt agataaagtt cttagtgcat ataacaaaca tagagacaaa 3900

ccaatacgtg aacaagcaga aaatattatt catttattta cgttgacgaa tcttggagct 3960

cccgctgctt ttaaatattt tgatacaaca attgatcgta aacgatatac gtctacaaaa 4020

gaagttttag atgccactct tatccatcaa tccatcactg gtctttatga aacacgcatt 4080

gatttgagtc agctaggagg tgactaa 4107
   <210> 109
      <211> 1368
      <212> PRT
      <213> Artificial Sequence
   <220>
      <223> Amino acid sequence of Cas9 from S.pyogenes
   <400> 109
   <210> 110
      <211> 4221
      <212> DNA
      <213> Artificial Sequence
   <220>
      <223> Cas9-coding sequence in pET-Cas9N3T for the production of recombinant Cas9 protein in E. coli (humanized codon; hexa-His-tag and a nuclear localization signal at the N terminus)
   <400> 110 
atgggcagca gccatcatca tcatcatcat gtgtacccct acgacgtgcc cgactacgcc 60

gaattgcctc caaaaaagaa gagaaaggta gggatcgaga acctgtactt ccagggcgac 120

aagaagtaca gcatcggcct ggacatcggt accaacagcg tgggctgggc cgtgatcacc 180

gacgagtaca aggtgcccag caagaagttc aaggtgctgg gcaacaccga ccgccacagc 240

atcaagaaga acctgatcgg cgccctgctg ttcgacagcg gcgagaccgc cgaggccacc 300

cgcctgaagc gcaccgcccg ccgccgctac acccgccgca agaaccgcat ctgctacctg 360

caggagatct tcagcaacga gatggccaag gtggacgaca gcttcttcca ccgcctggag 420

gagagcttcc tggtggagga ggacaagaag cacgagcgcc accccatctt cggcaacatc 480

gtggacgagg tggcctacca cgagaagtac cccaccatct accacctgcg caagaagctg 540

gtggacagca ccgacaaggc cgacctgcgc ctgatctacc tggccctggc ccacatgatc 600

aagttccgcg gccacttcct gatcgagggc gacctgaacc ccgacaacag cgacgtggac 660

aagctgttca tccagctggt gcagacctac aaccagctgt tcgaggagaa ccccatcaac 720

gccagcggcg tggacgccaa ggccatcctg agcgcccgcc tgagcaagag ccgccgcctg 780

gagaacctga tcgcccagct gcccggcgag aagaagaacg gcctgttcgg caacctgatc 840

gccctgagcc tgggcctgac ccccaacttc aagagcaact tcgacctggc cgaggacgcc 900

aagctgcagc tgagcaagga cacctacgac gacgacctgg acaacctgct ggcccagatc 960

ggcgaccagt acgccgacct gttcctggcc gccaagaacc tgagcgacgc catcctgctg 1020
 
agcgacatcc tgcgcgtgaa caccgagatc accaaggccc ccctgagcgc cagcatgatc 1080

aagcgctacg acgagcacca ccaggacctg accctgctga aggccctggt gcgccagcag 1140

ctgcccgaga agtacaagga gatcttcttc gaccagagca agaacggcta cgccggctac 1200

atcgacggcg gcgccagcca ggaggagttc tacaagttca tcaagcccat cctggagaag 1260

atggacggca ccgaggagct gctggtgaag ctgaaccgcg aggacctgct gcgcaagcag 1320

cgcaccttcg acaacggcag catcccccac cagatccacc tgggcgagct gcacgccatc 1380

ctgcgccgcc aggaggactt ctaccccttc ctgaaggaca accgcgagaa gatcgagaag 1440

atcctgacct tccgcatccc ctactacgtg ggccccctgg cccgcggcaa cagccgcttc 1500

gcctggatga cccgcaagag cgaggagacc atcaccccct ggaacttcga ggaggtggtg 1560

gacaagggcg ccagcgccca gagcttcatc gagcgcatga ccaacttcga caagaacctg 1620

cccaacgaga aggtgctgcc caagcacagc ctgctgtacg agtacttcac cgtgtacaac 1680

gagctgacca aggtgaagta cgtgaccgag ggcatgcgca agcccgcctt cctgagcggc 1740

gagcagaaga aggccatcgt ggacctgctg ttcaagacca accgcaaggt gaccgtgaag 1800

cagctgaagg aggactactt caagaagatc gagtgcttcg acagcgtgga gatcagcggc 1860

gtggaggacc gcttcaacgc cagcctgggc acctaccacg acctgctgaa gatcatcaag 1920

gacaaggact tcctggacaa cgaggagaac gaggacatcc tggaggacat cgtgctgacc 1980

ctgaccctgt tcgaggaccg cgagatgatc gaggagcgcc tgaagaccta cgcccacctg 2040

ttcgacgaca aggtgatgaa gcagctgaag cgccgccgct acaccggctg gggccgcctg 2100

agccgcaagc ttatcaacgg catccgcgac aagcagagcg gcaagaccat cctggacttc 2160

ctgaagagcg acggcttcgc caaccgcaac ttcatgcagc tgatccacga cgacagcctg 2220

accttcaagg aggacatcca gaaggcccag gtgagcggcc agggcgacag cctgcacgag 2280

cacatcgcca acctggccgg cagccccgcc atcaagaagg gcatcctgca gaccgtgaag 2340

gtggtggacg agctggtgaa ggtgatgggc cgccacaagc ccgagaacat cgtgatcgag 2400

atggcccgcg agaaccagac cacccagaag ggccagaaga acagccgcga gcgcatgaag 2460

cgcatcgagg agggcatcaa ggagctgggc agccagatcc tgaaggagca ccccgtggag 2520

aacacccagc tgcagaacga gaagctgtac ctgtactacc tgcagaacgg ccgcgacatg 2580

tacgtggacc aggagctgga catcaaccgc ctgagcgact acgacgtgga ccacatcgtg 2640

ccccagagct tcctgaagga cgacagcatc gacaacaagg tgctgacccg cagcgacaag 2700

aaccgcggca agagcgacaa cgtgcccagc gaggaggtgg tgaagaagat gaagaactac 2760

tggcgccagc tgctgaacgc caagctgatc acccagcgca agttcgacaa cctgaccaag 2820

gccgagcgcg gcggcctgag cgagctggac aaggccggct tcatcaagcg ccagctggtg 2880

gagacccgcc agatcaccaa gcacgtggcc cagatcctgg acagccgcat gaacaccaag 2940
 
tacgacgaga acgacaagct gatccgcgag gtgaaggtga tcaccctgaa gagcaagctg 3000

gtgagcgact tccgcaagga cttccagttc tacaaggtgc gcgagatcaa caactaccac 3060

cacgcccacg acgcctacct gaacgccgtg gtgggcaccg ccctgatcaa gaagtacccc 3120

aagctggaga gcgagttcgt gtacggcgac tacaaggtgt acgacgtgcg caagatgatc 3180

gccaagagcg agcaggagat cggcaaggcc accgccaagt acttcttcta cagcaacatc 3240

atgaacttct tcaagaccga gatcaccctg gccaacggcg agatccgcaa gcgccccctg 3300

atcgagacca acggcgagac cggcgagatc gtgtgggaca agggccgcga cttcgccacc 3360

gtgcgcaagg tgctgagcat gccccaggtg aacatcgtga agaagaccga ggtgcagacc 3420

ggcggcttca gcaaggagag catcctgccc aagcgcaaca gcgacaagct gatcgcccgc 3480

aagaaggact gggaccccaa gaagtacggc ggcttcgaca gccccaccgt ggcctacagc 3540

gtgctggtgg tggccaaggt ggagaagggc aagagcaaga agctgaagag cgtgaaggag 3600

ctgctgggca tcaccatcat ggagcgcagc agcttcgaga agaaccccat cgacttcctg 3660

gaggccaagg gctacaagga ggtgaagaag gacctgatca tcaagctgcc caagtacagc 3720

ctgttcgagc tggagaacgg ccgcaagcgc atgctggcca gcgccggcga gctgcagaag 3780

ggcaacgagc tggccctgcc cagcaagtac gtgaacttcc tgtacctggc cagccactac 3840

gagaagctga agggcagccc cgaggacaac gagcagaagc agctgttcgt ggagcagcac 3900

aagcactacc tggacgagat catcgagcag atcagcgagt tcagcaagcg cgtgatcctg 3960

gccgacgcca acctggacaa ggtgctgagc gcctacaaca agcaccgcga caagcccatc 4020

cgcgagcagg ccgagaacat catccacctg ttcaccctga ccaacctggg cgcccccgcc 4080

gccttcaagt acttcgacac caccatcgac cgcaagcgct acaccagcac caaggaggtg 4140

ctggacgcca ccctgatcca ccagagcatc accggtctgt acgagacccg catcgacctg 4200

agccagctgg gcggcgacta a 4221
   <210> 111
      <211> 1406
      <212> PRT
      <213> Artificial Sequence
   <220>
      <223> Amino acid sequence of Cas9 (pET-Cas9N3T)
   <400> 111

Met Gly Ser Ser His His His His His His Val Tyr Pro Tyr Asp Val
1               5                   10                  15

Pro Asp Tyr Ala Glu Leu Pro Pro Lys Lys Lys Arg Lys Val Gly Ile
            20                  25                  30

Glu Asn Leu Tyr Phe Gln Gly Asp Lys Lys Tyr Ser Ile Gly Leu Asp

        35                  40                  45

Ile Gly Thr Asn Ser Val Gly Trp Ala Val Ile Thr Asp Glu Tyr Lys
    50                  55                  60

Val Pro Ser Lys Lys Phe Lys Val Leu Gly Asn Thr Asp Arg His Ser
65                  70                  75                  80

Ile Lys Lys Asn Leu Ile Gly Ala Leu Leu Phe Asp Ser Gly Glu Thr
                85                  90                  95

Ala Glu Ala Thr Arg Leu Lys Arg Thr Ala Arg Arg Arg Tyr Thr Arg
            100                 105                 110

Arg Lys Asn Arg Ile Cys Tyr Leu Gln Glu Ile Phe Ser Asn Glu Met
        115                 120                 125

Ala Lys Val Asp Asp Ser Phe Phe His Arg Leu Glu Glu Ser Phe Leu
    130                 135                 140

Val Glu Glu Asp Lys Lys His Glu Arg His Pro Ile Phe Gly Asn Ile
145                 150                 155                 160

Val Asp Glu Val Ala Tyr His Glu Lys Tyr Pro Thr Ile Tyr His Leu
                165                 170                 175

Arg Lys Lys Leu Val Asp Ser Thr Asp Lys Ala Asp Leu Arg Leu Ile
            180                 185                 190

Tyr Leu Ala Leu Ala His Met Ile Lys Phe Arg Gly His Phe Leu Ile
        195                 200                 205

Glu Gly Asp Leu Asn Pro Asp Asn Ser Asp Val Asp Lys Leu Phe Ile
    210                 215                 220

Gln Leu Val Gln Thr Tyr Asn Gln Leu Phe Glu Glu Asn Pro Ile Asn
225                 230                 235                 240

Ala Ser Gly Val Asp Ala Lys Ala Ile Leu Ser Ala Arg Leu Ser Lys
                245                 250                 255

Ser Arg Arg Leu Glu Asn Leu Ile Ala Gln Leu Pro Gly Glu Lys Lys
            260                 265                 270

Asn Gly Leu Phe Gly Asn Leu Ile Ala Leu Ser Leu Gly Leu Thr Pro
        275                 280                 285

Asn Phe Lys Ser Asn Phe Asp Leu Ala Glu Asp Ala Lys Leu Gln Leu
    290                 295                 300

Ser Lys Asp Thr Tyr Asp Asp Asp Leu Asp Asn Leu Leu Ala Gln Ile
305                 310                 315                 320

Gly Asp Gln Tyr Ala Asp Leu Phe Leu Ala Ala Lys Asn Leu Ser Asp
                325                 330                 335

Ala Ile Leu Leu Ser Asp Ile Leu Arg Val Asn Thr Glu Ile Thr Lys
            340                 345                 350

Ala Pro Leu Ser Ala Ser Met Ile Lys Arg Tyr Asp Glu His His Gln
        355                 360                 365

Asp Leu Thr Leu Leu Lys Ala Leu Val Arg Gln Gln Leu Pro Glu Lys

370                 375                 380

Tyr Lys Glu Ile Phe Phe Asp Gln Ser Lys Asn Gly Tyr Ala Gly Tyr
385                 390                 395                 400

Ile Asp Gly Gly Ala Ser Gln Glu Glu Phe Tyr Lys Phe Ile Lys Pro
                405                 410                 415

Ile Leu Glu Lys Met Asp Gly Thr Glu Glu Leu Leu Val Lys Leu Asn
            420                 425                 430

Arg Glu Asp Leu Leu Arg Lys Gln Arg Thr Phe Asp Asn Gly Ser Ile
        435                 440                 445

Pro His Gln Ile His Leu Gly Glu Leu His Ala Ile Leu Arg Arg Gln
    450                 455                 460

Glu Asp Phe Tyr Pro Phe Leu Lys Asp Asn Arg Glu Lys Ile Glu Lys
465                 470                 475                 480

Ile Leu Thr Phe Arg Ile Pro Tyr Tyr Val Gly Pro Leu Ala Arg Gly
                485                 490                 495

Asn Ser Arg Phe Ala Trp Met Thr Arg Lys Ser Glu Glu Thr Ile Thr
            500                 505                 510

Pro Trp Asn Phe Glu Glu Val Val Asp Lys Gly Ala Ser Ala Gln Ser
        515                 520                 525

Phe Ile Glu Arg Met Thr Asn Phe Asp Lys Asn Leu Pro Asn Glu Lys
    530                 535                 540

Val Leu Pro Lys His Ser Leu Leu Tyr Glu Tyr Phe Thr Val Tyr Asn
545                 550                 555                 560

Glu Leu Thr Lys Val Lys Tyr Val Thr Glu Gly Met Arg Lys Pro Ala
                565                 570                 575

Phe Leu Ser Gly Glu Gln Lys Lys Ala Ile Val Asp Leu Leu Phe Lys
            580                 585                 590

Thr Asn Arg Lys Val Thr Val Lys Gln Leu Lys Glu Asp Tyr Phe Lys
        595                 600                 605

Lys Ile Glu Cys Phe Asp Ser Val Glu Ile Ser Gly Val Glu Asp Arg
    610                 615                 620

Phe Asn Ala Ser Leu Gly Thr Tyr His Asp Leu Leu Lys Ile Ile Lys
625                 630                 635                 640

Asp Lys Asp Phe Leu Asp Asn Glu Glu Asn Glu Asp Ile Leu Glu Asp
                645                 650                 655

Ile Val Leu Thr Leu Thr Leu Phe Glu Asp Arg Glu Met Ile Glu Glu
            660                 665                 670

Arg Leu Lys Thr Tyr Ala His Leu Phe Asp Asp Lys Val Met Lys Gln
        675                 680                 685

Leu Lys Arg Arg Arg Tyr Thr Gly Trp Gly Arg Leu Ser Arg Lys Leu
    690                 695                 700

Ile Asn Gly Ile Arg Asp Lys Gln Ser Gly Lys Thr Ile Leu Asp Phe

705                 710                 715                 720

Leu Lys Ser Asp Gly Phe Ala Asn Arg Asn Phe Met Gln Leu Ile His
                725                 730                 735

Asp Asp Ser Leu Thr Phe Lys Glu Asp Ile Gln Lys Ala Gln Val Ser
            740                 745                 750

Gly Gln Gly Asp Ser Leu His Glu His Ile Ala Asn Leu Ala Gly Ser
        755                 760                 765

Pro Ala Ile Lys Lys Gly Ile Leu Gln Thr Val Lys Val Val Asp Glu
    770                 775                 780

Leu Val Lys Val Met Gly Arg His Lys Pro Glu Asn Ile Val Ile Glu
785                 790                 795                 800

Met Ala Arg Glu Asn Gln Thr Thr Gln Lys Gly Gln Lys Asn Ser Arg
                805                 810                 815

Glu Arg Met Lys Arg Ile Glu Glu Gly Ile Lys Glu Leu Gly Ser Gln
            820                 825                 830

Ile Leu Lys Glu His Pro Val Glu Asn Thr Gln Leu Gln Asn Glu Lys
        835                 840                 845

Leu Tyr Leu Tyr Tyr Leu Gln Asn Gly Arg Asp Met Tyr Val Asp Gln
    850                 855                 860

Glu Leu Asp Ile Asn Arg Leu Ser Asp Tyr Asp Val Asp His Ile Val
865                 870                 875                 880

Pro Gln Ser Phe Leu Lys Asp Asp Ser Ile Asp Asn Lys Val Leu Thr
                885                 890                 895

Arg Ser Asp Lys Asn Arg Gly Lys Ser Asp Asn Val Pro Ser Glu Glu
            900                 905                 910

Val Val Lys Lys Met Lys Asn Tyr Trp Arg Gln Leu Leu Asn Ala Lys
        915                 920                 925

Leu Ile Thr Gln Arg Lys Phe Asp Asn Leu Thr Lys Ala Glu Arg Gly
    930                 935                 940

Gly Leu Ser Glu Leu Asp Lys Ala Gly Phe Ile Lys Arg Gln Leu Val
945                 950                 955                 960

Glu Thr Arg Gln Ile Thr Lys His Val Ala Gln Ile Leu Asp Ser Arg
                965                 970                 975

Met Asn Thr Lys Tyr Asp Glu Asn Asp Lys Leu Ile Arg Glu Val Lys
            980                 985                 990

Val Ile Thr Leu Lys Ser Lys Leu Val Ser Asp Phe Arg Lys Asp Phe
        995                 1000                1005

Gln Phe Tyr Lys Val Arg Glu Ile Asn Asn Tyr His His Ala His Asp
    1010                1015                1020

Ala Tyr Leu Asn Ala Val Val Gly Thr Ala Leu Ile Lys Lys Tyr Pro
1025                1030                1035                1040

Lys Leu Glu Ser Glu Phe Val Tyr Gly Asp Tyr Lys Val Tyr Asp Val

1045                1050                1055

Arg Lys Met Ile Ala Lys Ser Glu Gln Glu Ile Gly Lys Ala Thr Ala
        1060                1065                1070

Lys Tyr Phe Phe Tyr Ser Asn Ile Met Asn Phe Phe Lys Thr Glu Ile
    1075                1080                1085

Thr Leu Ala Asn Gly Glu Ile Arg Lys Arg Pro Leu Ile Glu Thr Asn
  1090                1095                1100

Gly Glu Thr Gly Glu Ile Val Trp Asp Lys Gly Arg Asp Phe Ala Thr
1105                1110                1115                1120

Val Arg Lys Val Leu Ser Met Pro Gln Val Asn Ile Val Lys Lys Thr
                1125                1130                1135

Glu Val Gln Thr Gly Gly Phe Ser Lys Glu Ser Ile Leu Pro Lys Arg
        1140                1145                1150

Asn Ser Asp Lys Leu Ile Ala Arg Lys Lys Asp Trp Asp Pro Lys Lys
    1155                1160                1165

Tyr Gly Gly Phe Asp Ser Pro Thr Val Ala Tyr Ser Val Leu Val Val
  1170                1175                1180

Ala Lys Val Glu Lys Gly Lys Ser Lys Lys Leu Lys Ser Val Lys Glu
1185                1190                1195                1200

Leu Leu Gly Ile Thr Ile Met Glu Arg Ser Ser Phe Glu Lys Asn Pro
                1205                1210                1215

Ile Asp Phe Leu Glu Ala Lys Gly Tyr Lys Glu Val Lys Lys Asp Leu
        1220                1225                1230

Ile Ile Lys Leu Pro Lys Tyr Ser Leu Phe Glu Leu Glu Asn Gly Arg
    1235                1240                1245

Lys Arg Met Leu Ala Ser Ala Gly Glu Leu Gln Lys Gly Asn Glu Leu
  1250                1255                1260

Ala Leu Pro Ser Lys Tyr Val Asn Phe Leu Tyr Leu Ala Ser His Tyr
1265                1270                1275                1280

Glu Lys Leu Lys Gly Ser Pro Glu Asp Asn Glu Gln Lys Gln Leu Phe
                1285                1290                1295

Val Glu Gln His Lys His Tyr Leu Asp Glu Ile Ile Glu Gln Ile Ser
        1300                1305                1310

Glu Phe Ser Lys Arg Val Ile Leu Ala Asp Ala Asn Leu Asp Lys Val
    1315                1320                1325

Leu Ser Ala Tyr Asn Lys His Arg Asp Lys Pro Ile Arg Glu Gln Ala
  1330                1335                1340

Glu Asn Ile Ile His Leu Phe Thr Leu Thr Asn Leu Gly Ala Pro Ala
1345                1350                1355                1360

Ala Phe Lys Tyr Phe Asp Thr Thr Ile Asp Arg Lys Arg Tyr Thr Ser
                1365                1370                1375

Thr Lys Glu Val Leu Asp Ala Thr Leu Ile His Gln Ser Ile Thr Gly

1380                1385                1390

Leu Tyr Glu Thr Arg Ile Asp Leu Ser Gln Leu Gly Gly Asp
    1395                1400                1405

## Description

### Technical Field

As defined in the claims, the present invention generally relates to targeted genome editing in plant protoplasts. More particularly, the present invention generally relates to a composition for cleaving a target DNA in protoplasts comprising a guide RNA specific for the target DNA and Cas9 protein, and use thereof.

### Background Art

CRISPRs (Clustered Regularly Interspaced Short Palindromic Repeats) are loci containing multiple short direct repeats that are found in the genomes of approximately 40% of sequenced bacteria and 90% of sequenced archaea. CRISPR functions as a prokaryotic immune system, in that it confers resistance to exogenous genetic elements such as plasmids and phages. The CRISPR system provides a form of acquired immunity. Short segments of foreign DNA, called spacers, are incorporated into the genome between CRISPR repeats, and serve as a memory of past exposures. CRISPR spacers are then used to recognize and silence exogenous genetic elements in a manner analogous to RNAi in eukaryotic organisms.

Cas9, an essential protein component in the Type II CRISPR/Cas system, forms an active endonuclease when complexed with two RNAs termed CRISPR RNA (crRNA) and trans-activating crRNA (tracrRNA), thereby slicing foreign genetic elements in invading phages or plasmids to protect the host cells. crRNA is transcribed from the CRISPR element in the host genome, which was previously captured from such foreign invaders. Recently, Jinek et al. (1) demonstrated that a single-chain chimeric RNA produced by fusing an essential portion of crRNA and tracrRNA could replace the two RNAs in the Cas9/RNA complex to form a functional endonuclease.

CRISPR/Cas systems offer an advantage to zinc finger and transcription activator-like effector DNA-binding proteins, as the site specificity in nucleotide binding CRISPR-Cas proteins is governed by a RNA molecule instead of the DNA-binding protein, which can be more challenging to design and synthesize.

Shan et al., Nature Biotechnology, vol. 31, no. 8, 08 August 2013, pages 686 - 688; Li et al., Nature Biotechnology, vol. 31, no. 8, 08 August 2013, pages 688 - 691; Nekrasov et al., Nature Biotechnology, vol. 31, no. 8, 08 August 2013, pages 691 - 693; Feng et al., Cell Research, vol. 23, no. 10, 20 August 2013, pages 1229 - 1232; Jiang et al., Nucleic Acids Research, vol. 41, no. 20, 02 September 2013, pages e188 - e188; and Miao et al., Cell Research, vol. 23, no. 10, 03 September 2013, pages 1233 - 1236 relate to CRISPR/Cas9-mediated genome modification in plants using DNA plasmids encoding Cas9 and sgRNA as vectors for plant cell transformation.

However, until now, genome editing in plants using a ribonucleoprotein particle, or RNP, comprising an RNA-guided endonuclease(RGEN) based on CRISPR/Cas system has not been developed.

Meanwhile, Restriction fragment length polymorphism (RFLP) is one of the oldest, most convenient, and least expensive methods of genotyping that is still used widely in molecular biology and genetics but is often limited by the lack of appropriate sites recognized by restriction endonucleases.

Engineered nuclease-induced mutations are detected by various methods, which include mismatch-sensitive T7 endonuclease I (T7E1) or Surveyor nuclease assays, RFLP, capillary electrophoresis of fluorescent PCR products, Dideoxy sequencing, and deep sequencing. The T7E1 and Surveyor assays are widely used but are cumbersome. Furthermore, theses enzymes tend to underestimate mutation frequencies because mutant sequences can form homoduplexes with each other and cannot distinguish homozygous biallelic mutant clones from wildtype cells. RFLP is free of these limitations and therefore is a method of choice. Indeed, RFLP was one of the first methods to detect engineered nuclease-mediated mutations in cells and animals. Unfortunately, however, RFLP is limited by the availability of appropriate restriction sites. It is possible that no restriction sites are available at the target site of interest.

### Disclosure of invention

Until now, a genome editing and genotyping method in plants using an RNP comprising an RNA-guided endonuclease (RGEN) based on CRISPR/Cas system has not been developed.

Under these circumstances, the present inventors have made many efforts to develop a genome editing method in plants based on CRISPR/Cas system and finally established a programmable RNA-guided endonuclease that cleave DNA in a targeted manner in plant protoplasts.

In addition, the present inventors have made many efforts to develop a novel method of using RNA-guided endonucleases (RGENs) in RFLP analysis. They have used RGENs to genotype recurrent mutations found in cancer and those induced in cells and organisms by engineered nucleases including RGENs themselves, thereby completing the present invention.

### Summary of the Invention

It is an object of the present invention to provide a composition for cleaving a target DNA in plant protoplasts, or for introducing two nicks into a target DNA in plant protoplasts, comprising a guide RNA specific for target DNA, and Cas9.

It is a further object of the invention to provide a method of cleaving a target DNA and a method of introducing two nicks in different strands of a target DNA, in a plant protoplast. It is a still further object of the invention to provide a kit for cleaving a target DNA in a plant protoplast.

In a first aspect, the present invention provides a CRISPR/Cas9 composition for cleaving a target DNA in a plant protoplast, wherein the target DNA is endogenous DNA, and wherein the composition comprises a complex of (a) a CRISPR-associated protein 9 (Cas9) polypeptide; and (b) a guide RNA specific to the target DNA, wherein the guide RNA is (i) a dual guide RNA comprising a CRISPR RNA (crRNA) and a trans activating crRNA (tracrRNA); or (ii) a single-chain guide RNA comprising a CRISPR RNA (crRNA) fused to a trans activating crRNA (tracrRNA), wherein the composition further comprises polyethylene glycol (PEG).

In one embodiment, the composition comprises two guide RNAs, wherein each guide RNA is specific for one of the strands of the target DNA and wherein the Cas9 polypeptide is a Cas9 nickase.

In a second aspect, the invention provides a method of cleaving a target DNA in a plant protoplast, wherein the target DNA is endogenous DNA, preferably genomic DNA, the method comprising introducing into the plant protoplast a complex of (a) a Cas9 polypeptide; and (b) a guide RNA specific to the target DNA, wherein the guide RNA is (i) a dual guide RNA comprising a CRISPR RNA (crRNA) and a trans activating crRNA (tracrRNA); or (ii) a single-chain guide RNA comprising a CRISPR RNA (crRNA) fused to a trans activating crRNA (tracrRNA), wherein the introduction is by polyethylene glycol (PEG)-mediated transfection.

In a third aspect, the invention provides a method of introducing two nicks in different strands of a target DNA in a plant protoplast, wherein the target DNA is endogenous DNA, preferably genomic DNA, the method comprising introducing into the plant protoplast a complex of (a) a Cas9 polypeptide, wherein the Cas9 polypeptide is a Cas9 nickase; and (b) two guide RNAs, wherein each of the two guide RNAs is specific for one of the strands of the target DNA, wherein the guide RNA is (i) a dual guide RNA comprising a CRISPR RNA (crRNA) and a trans activating crRNA (tracrRNA); or (ii) a single-chain guide RNA comprising a CRISPR RNA (crRNA) fused to a trans activating crRNA (tracrRNA), wherein the introduction is by polyethylene glycol (PEG)-mediated transfection.

In one embodiment of the above method, the two nicks are separated by at least 100 base pairs.

In certain embodiments, the PEG-mediated transfection comprises transfection in a 40% PEG transfection buffer comprising 40% PEG4000, 200 mM mannitol and 100
mM CaCl2.

In a fourth aspect, the invention provides a kit for cleaving a target DNA in a plant protoplast comprising the composition of the invention.

In certain embodiments, the Cas9 protein is from the genus *Streptococcus.* In certain embodiments, the Cas9 protein is from *Streptococcus pyogenes.*

In certain embodiments, the Cas9 nickase is a mutant form of Cas9 in which the catalytic aspartate residue is changed to any other amino acid, preferably alanine.

In certain embodiments, the Cas9 polypeptide comprises a Cas9 protein and a nuclear localization signal (NLS), wherein the NLS is at the N-terminus of the Cas9 protein.

In certain embodiments, the crRNA comprises two additional guanine nucleotides at its 5' end.

In certain embodiments, the target DNA is genomic DNA.

In certain embodiments, the target DNA comprises a trinucleotide protospacer adjacent motif (PAM) recognized by Cas9, wherein the Cas9 is from *Streptococcus pyogenes,* wherein the PAM consists of the trinucleotide 5'-NGG-3'.

The composition of the invention is also referred to herein as "system", "RGEN composition", or "RGEN(s)".

Any reference herein to embryos should be understood to exclude human embryos, and likewise any reference to animals should be understood to exclude humans.

The compositions of the invention can be used for inducing targeted mutagenesis in plant protoplasts.

### Brief Description of Drawings

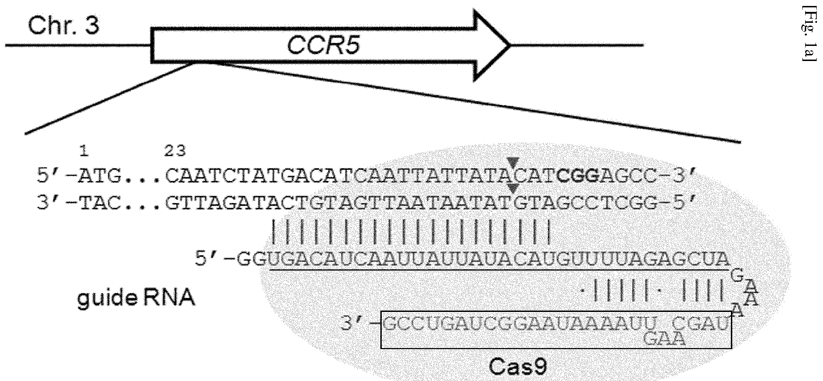

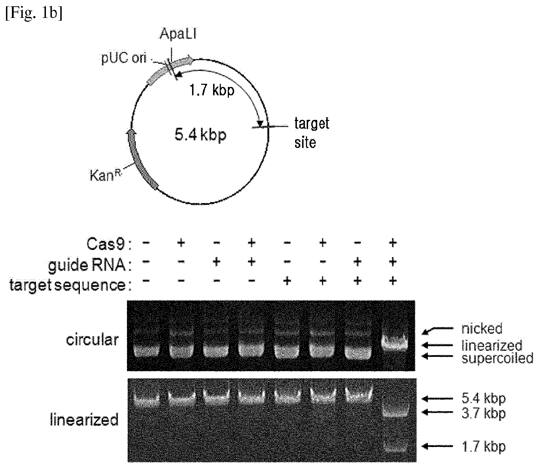

**Fig. 1** shows Cas9-catalyzed cleavage of plasmid DNA *in vitro.* (a) Schematic representation of target DNA _and chimeric RNA sequences. Red triangles indicate cleavage sites. The PAM sequence recognized by Cas9 is shown in bold. The sequences in the guide RNA derived from crRNA and tracrRNA are shown in box and underlined, respectively. (b) *In vitro* cleavage of plasmid DNA by Cas9. An intact circular plasmid or ApaLI-digested plasmid was incubated with Cas9 and guide RNA.

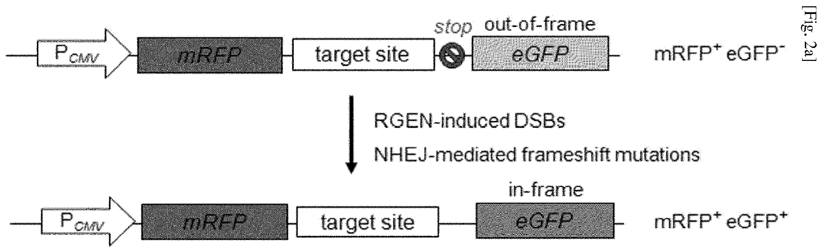

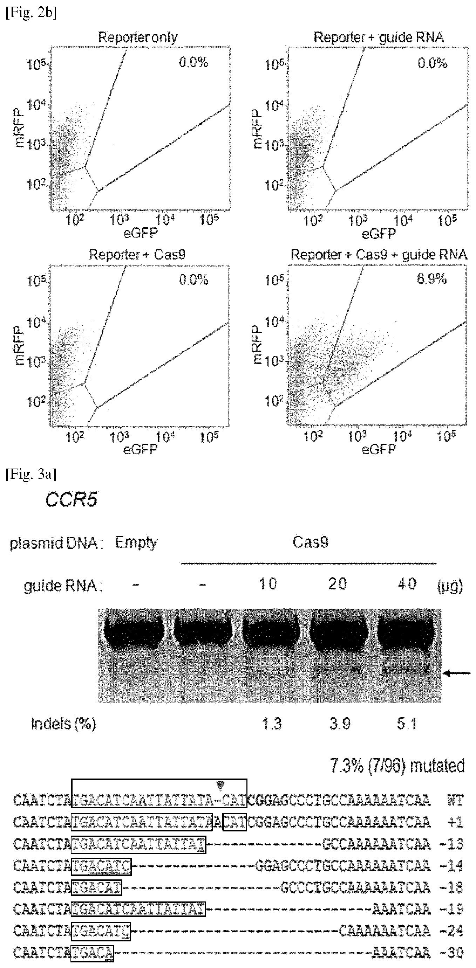

**Fig. 2** shows Cas9-induced mutagenesis at an episomal target site. (a) Schematic overview of cell-based assays using a RFP-GFP reporter. GFP is not expressed from this reporter because the GFP sequence is fused to the RFP sequence out-of-frame. The RFP-GFP fusion protein is expressed only when the target site between the two sequences is cleaved by a site-specific nuclease. (b) Flow cytometry of cells transfected with Cas9. The percentage of cells that express the RFP-GFP fusion protein is indicated.

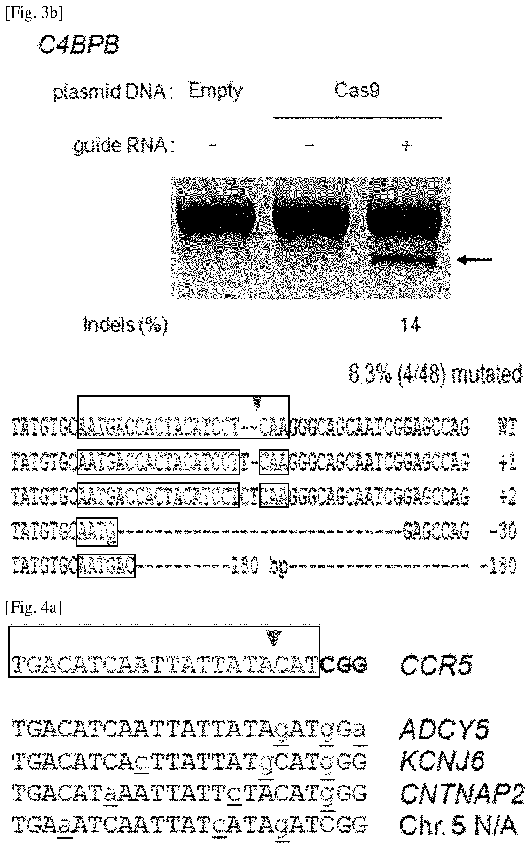

**Fig. 3** shows RGEN-driven mutations at endogenous chromosomal sites. (a) CCR5 locus. (b) C4BPB locus. (Top) The T7E1 assay was used to detect RGEN-driven mutations. Arrows indicate the expected position of DNA bands cleaved by T7E1. Mutation frequencies (Indels (%)) were calculated by measuring the band intensities. (Bottom) DNA sequences of the CCR5 and C4BPB wild-type (WT) and mutant clones. The region of the target sequence complementary to the guide RNA is shown in boc. The PAM sequence is shown in bold. Triangles indicate the cleavage site. Bases corresponding to microhomologies are underlined. The column on the right indicates the number of inserted or deleted bases.

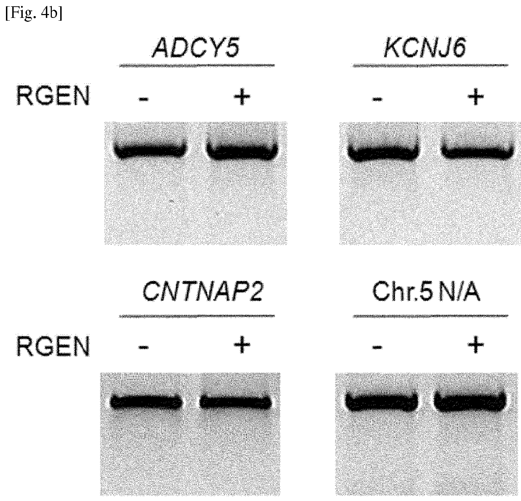

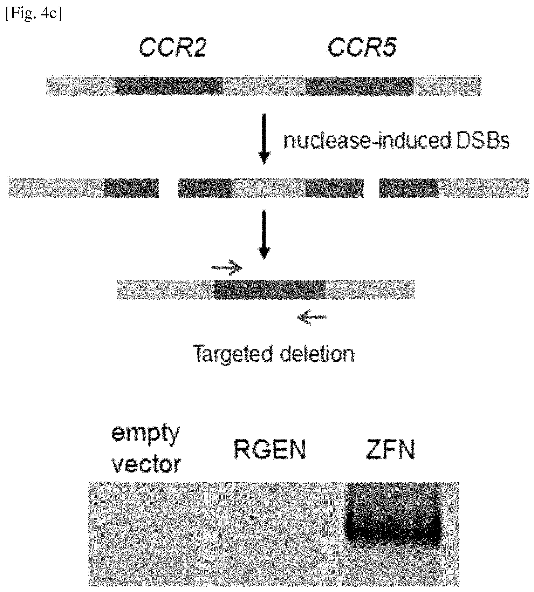

**Fig. 4** shows that RGEN-driven off-target mutations are undetectable. (a) On-target and potential off-target sequences. The human genome was searched *in silico* for potential off-target sites. Four sites were identified, each of which carries 3-base mismatches with the CCR5 on-target site. Mismatched bases are underlined. (b) The T7E1 assay was used to investigate whether these sites were mutated in cells transfected with the Cas9/RNA complex. No mutations were detected at these sites. N/A (not applicable), an intergenic site. (c) Cas9 did not induce off-target-associated chromosomal deletions. The CCR5-specific RGEN and ZFN were expressed in human cells. PCR was used to detect the induction of the 15-kb chromosomal deletions in these cells.

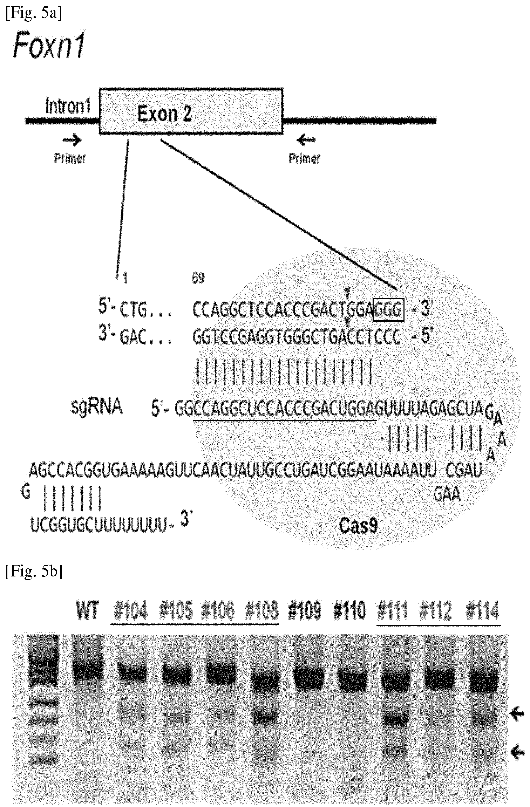

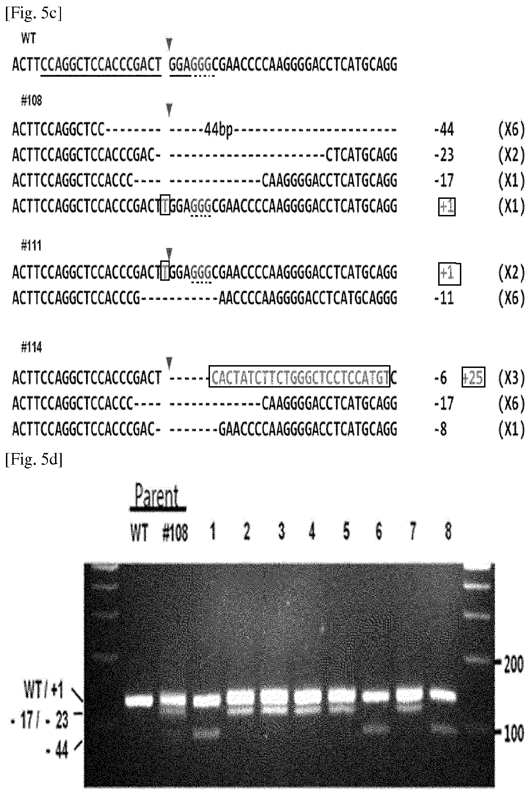

**Fig. 5** shows RGEN-induced Foxnl gene targeting in mice. (a) A schematic diagram depicting a sgRNA specific to exon 2 of the mouse Foxnl gene PAM in exon 2 is shown in red and the sequence in the sgRNA that is complementary to exon 2 is underlined. Triangles indicate cleavage sites. (b) Representative T7E1 assays demonstrating gene-targeting efficiencies of Cas9 mRNA plus Foxn1-specific sgRNA that were delivered via intra-cytoplasmic injection into one-cell stage mouse embryos. Numbers indicate independent founder mice generated from the highest dose. Arrows indicate bands cleaved by T7E1. (c) DNA sequences of mutant alleles observed in three Foxnl mutant founders identified in The number of occurrences is shown in parentheses. (d) PCR genotyping of F1 progenies derived from crossing Foxnl founder #108 and wild-type FVB/NTac. Note the segregation of the mutant alleles found in Foxnl founder #108 in the progenies.

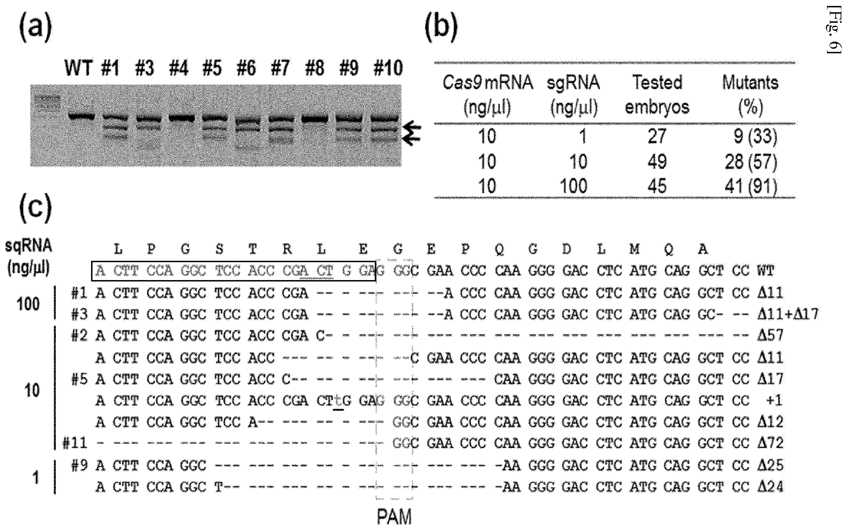

**Fig. 6** shows Foxnl gene targeting in mouse embryos by intra-cytoplasmic injection of Cas9 mRNA and Foxnl-sgRNA. (a) A representative result of a T7E1 assay monitoring the mutation rate after injecting the highest dose. Arrows indicate bands cleaved by T7E1. (b) A summary of T7E1 assay results. Mutant fractions among *in vitro* cultivated embryos obtained after intra-cytoplasmic injection of the indicated RGEN doses are indicated. (c) DNA sequences of Foxnl mutant alleles identified from a subset of T7E1-positive mutant embryos. The target sequence of the wild-type allele is denoted in box.

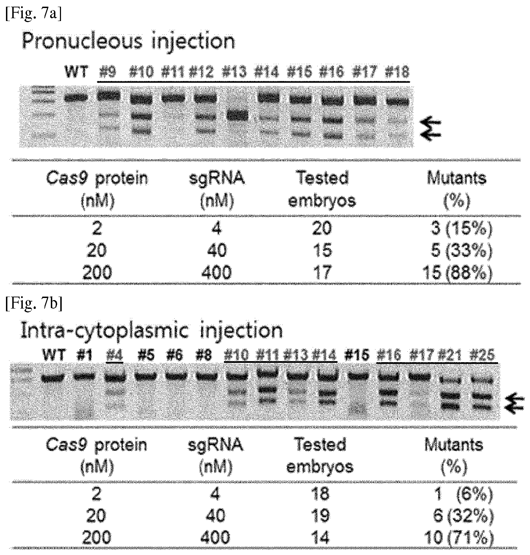

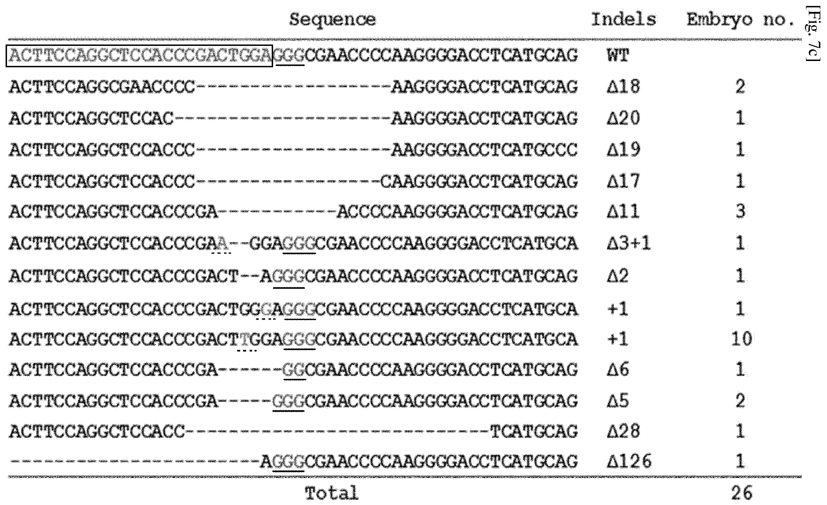

**Fig. 7** shows Foxn1 gene targeting in mouse embryos using the recombinant Cas9 protein: Foxnl-sgRNA complex. (a) and (b) are representative T7E1 assays results and their summaries. Embryos were cultivated *in vitro* after they underwent pronuclear (a) or intra-cytoplasmic injection (b). Numbers in red indicate T7E1-positive mutant founder mice. (c) DNA sequences of Foxnl mutant alleles identified from the *in vitro* cultivated embryos that were obtained by the pronucleus injection of recombinant Cas9 protein: Foxnl-sgRNA complex at the highest dose. The target sequence of the wild-type allele is denoted in box.

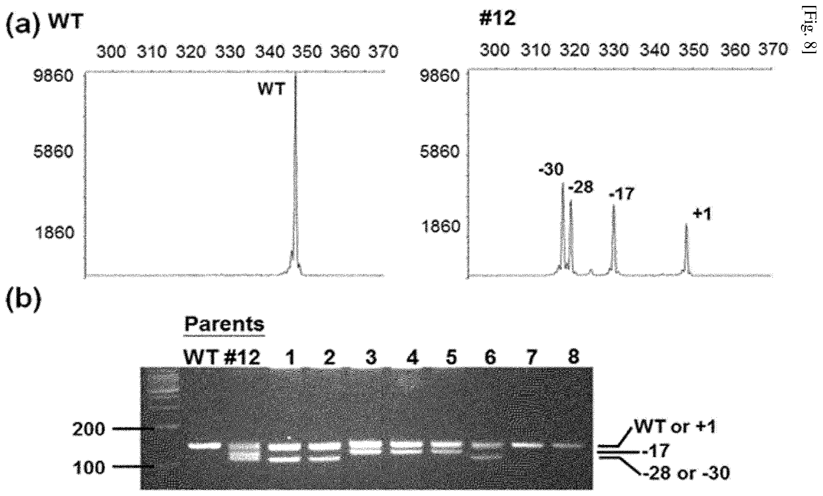

**Fig. 8** shows Germ-line transmission of the mutant alleles found in Foxnl mutant founder #12. (a) fPCR analysis. (b) PCR genotyping of wild-type FVB/NTac, the founder mouse, and their F1 progenies.

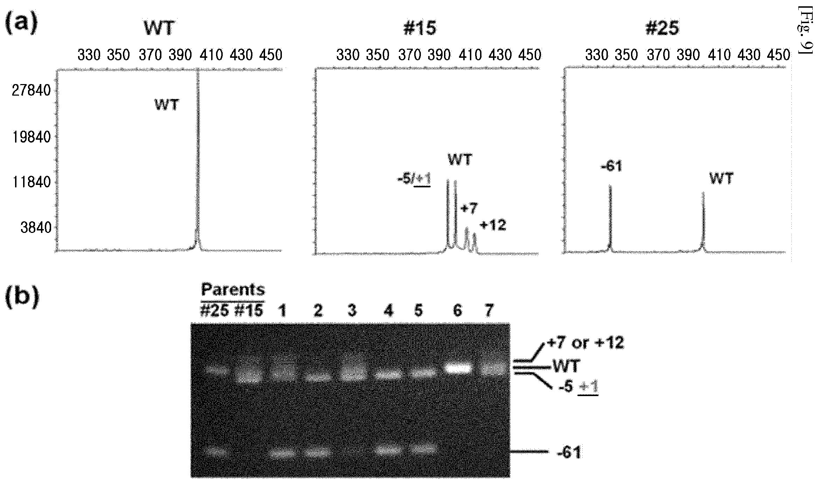

**Fig. 9** shows Genotypes of embryos generated by crossing Prkdc mutant founders. Prkdc mutant founders ♂25 and ♀15 were crossed and E13.5 embryos were isolated. (a) fPCR analysis of wild-type, founder ♂25, and founder ♀ 15. Note that, due to the technical limitations of fPCR analysis, these results showed small differences from the precise sequences of the mutant alleles; e.g., from the sequence analysis, Δ269/Δ61/WT and Δ5+1/+7/+12/WT were identified in founders ♂25 and ♀15, respectively. (b) Genotypes of the generated embryos.

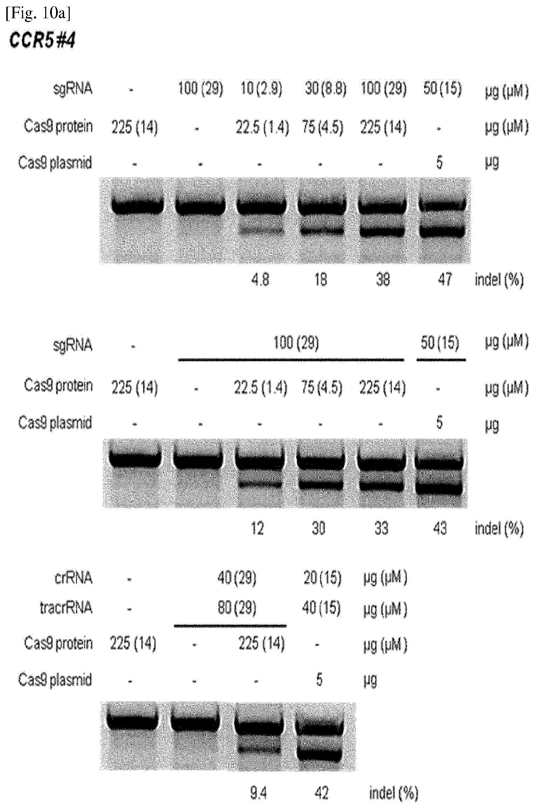

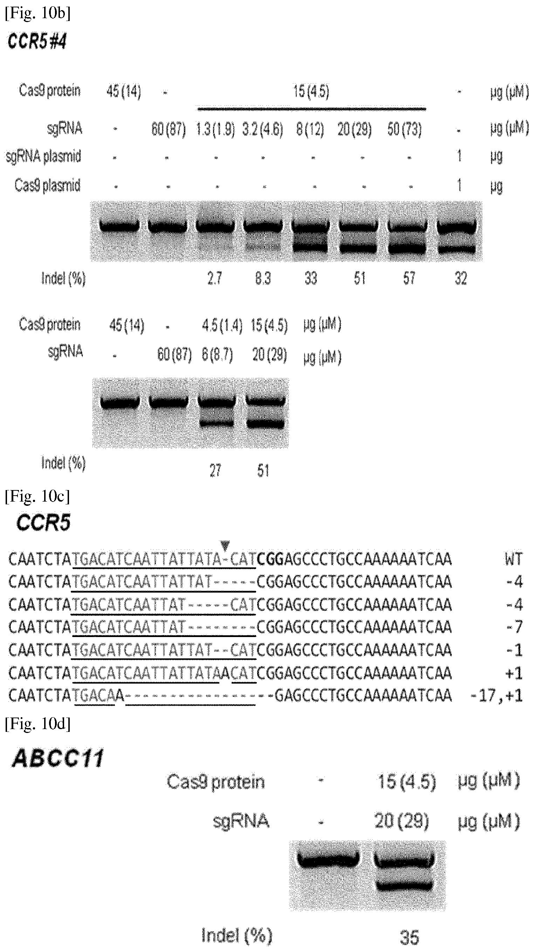

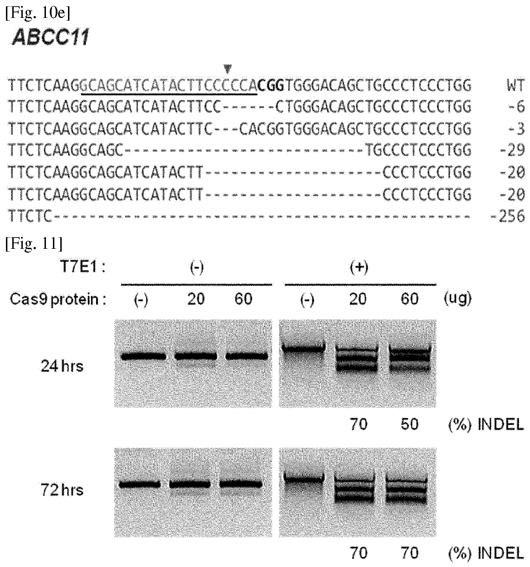

**Fig. 10** shows Cas9 protein/sgRNA complex induced targeted mutation.
**Fig. 11** shows recombinant Cas9 protein-induced mutations in Arabidopsis protoplasts.

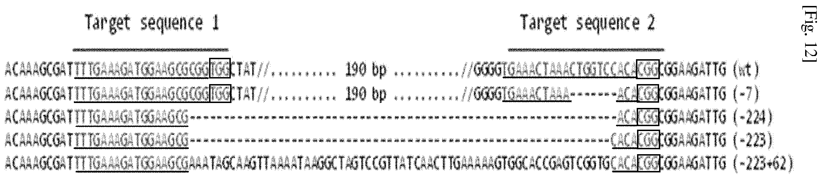

**Fig. 12** shows recombinant Cas9 protein-induced mutant sequences in the Arabidopsis BRI1 gene.

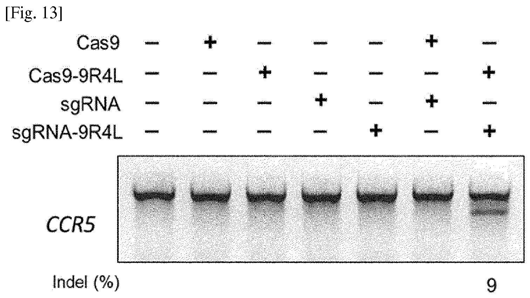

**Fig. 13** shows T7E1 assay showing endogenous CCR5 gene disruption in 293 cells by treatment of Cas9-mal-9R4L and sgRNA/C9R4LC complex.

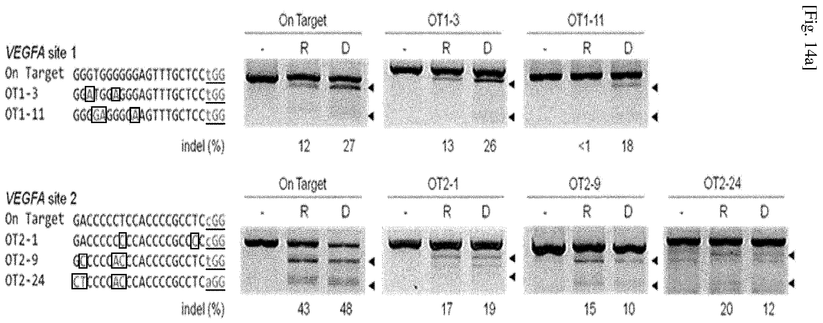

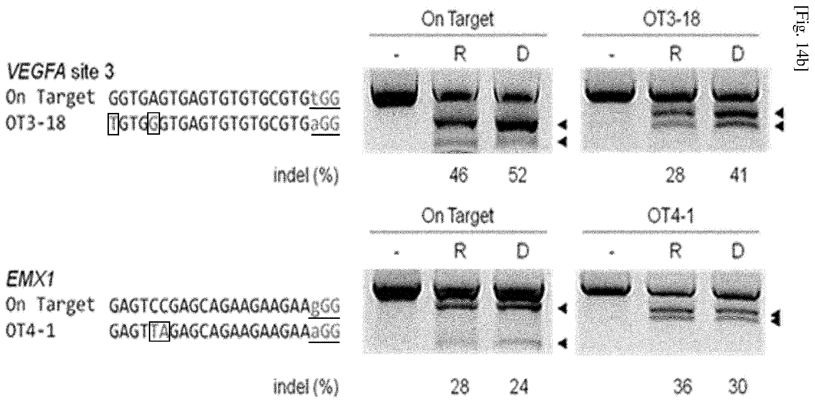

**Fig. 14** (a, b) shows mutation frequencies at on-target and off-target sites of RGENs reported in Fu et al. (2013). T7E1 assays analyzing genomic DNA from K562 cells (R) transfected serially with 20 µg of Cas9-encoding plasmid and with 60 µg and 120 µg of *in vitro* transcribed GX₁₉ crRNA and tracrRNA, respectively (1 x 10⁶ cells), or (D) co-transfected with 1 µg of Cas9-encoding plasmid and 1 µg of GX₁₉ sgRNA expression plasmid (2 x 10⁵ cells).

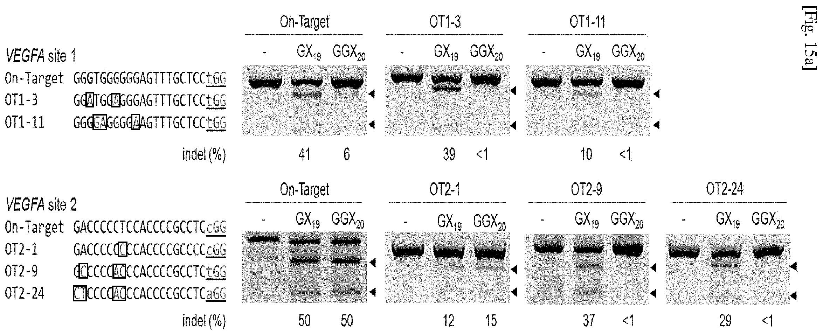

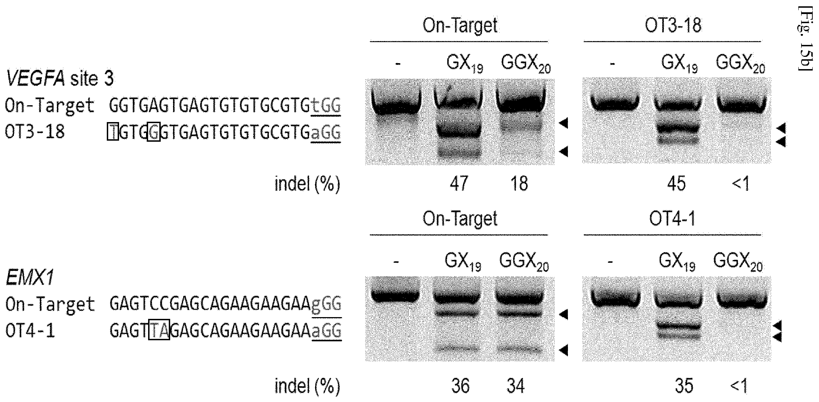

**Fig. 15** (a, b) shows comparison of guide RNA structure. Mutation frequencies of the RGENs reported in Fu et al. (2013) were measured at on-target and off-target sites using the T7E1 assay. K562 cells were co-transfected with the Cas9-encoding plasmid and the plasmid encoding GX₁₉ sgRNA or GGX₂₀ sgRNA. Off-target sites (OT1-3 etc.) are labeled as in Fu et al. (2013).

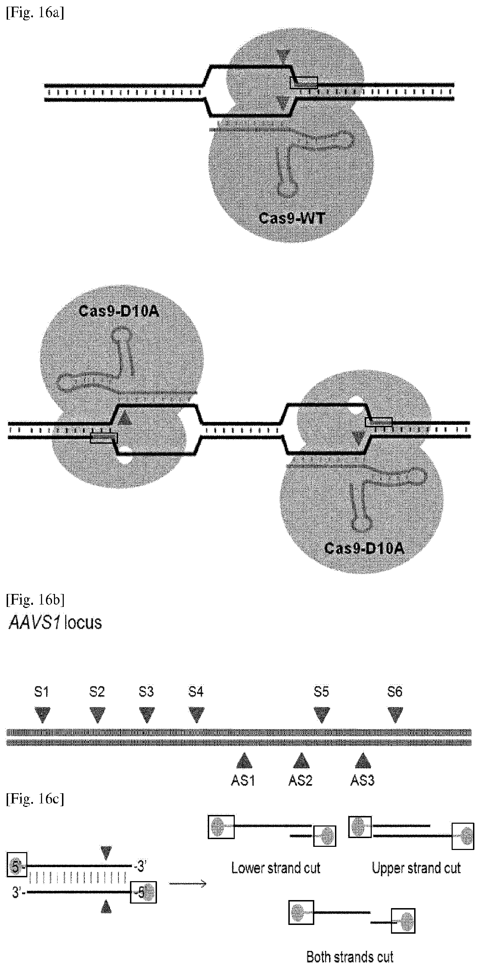

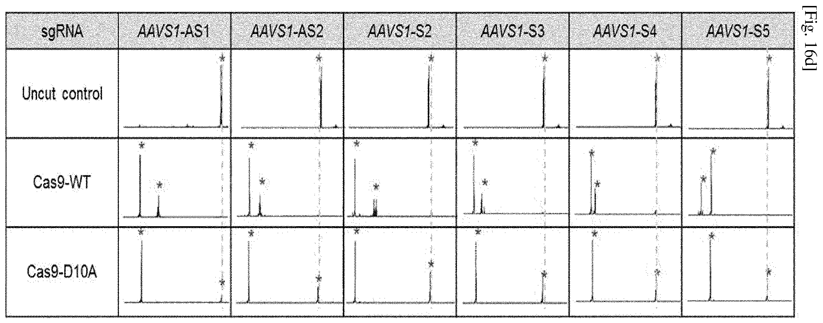

**Fig. 16** shows that *in vitro* DNA cleavage by Cas9 nickases. (a) Schematic overview of the Cas9 nuclease and the paired Cas9 nickase. The PAM sequences and cleavage sites are shown in box. (b) Target sites in the human AAVS1 locus. The position of each target site is shown in triangle. (c) Schematic overview of DNA cleavage reactions. FAM dyes (shown in box) were linked to both 5' ends of the DNA substrate. (d) DSBs and SSBs analyzed using fluorescent capillary eletrophoresis. Fluorescently-labeled DNA substrates were incubated with Cas9 nucleases or nickases before electrophoresis.

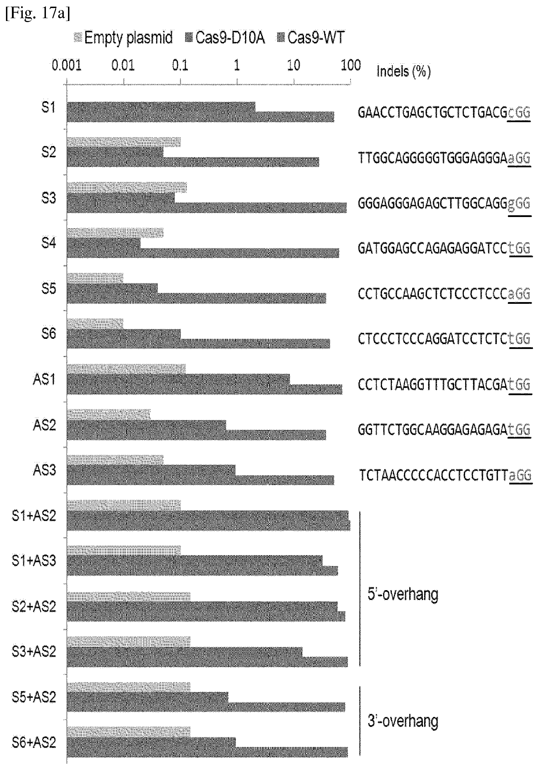

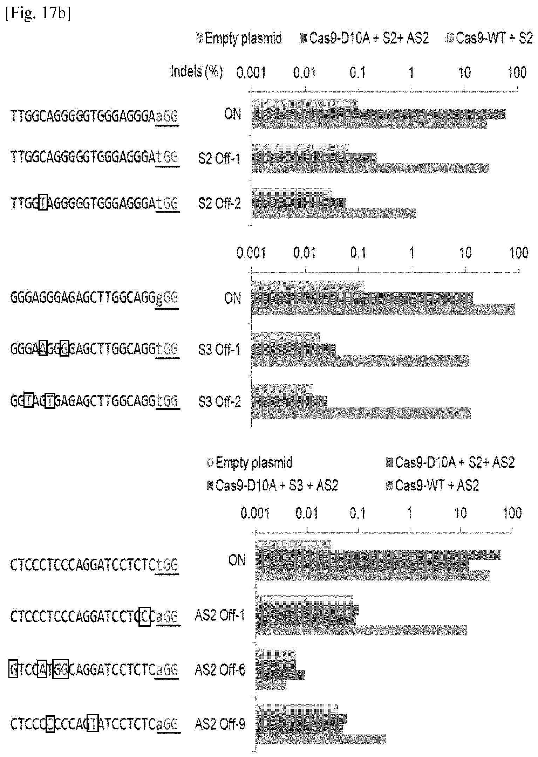

**Fig. 17** shows comparison of Cas9 nuclease and nickase behavior. (a) On-target mutation frequencies associated with Cas9 nucleases (WT), nickases (D10A), and paired nickases Paired nickases that would produce 5' overhangs or 3' overhangs are indicated. (b) Analysis of off-target effects of Cas9 nucleases and paired nickases. A total of seven potential off-target sites for three sgRNAs were analyzed.

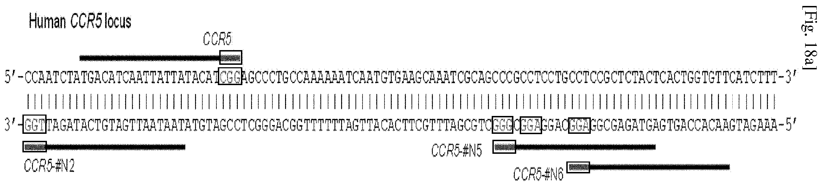

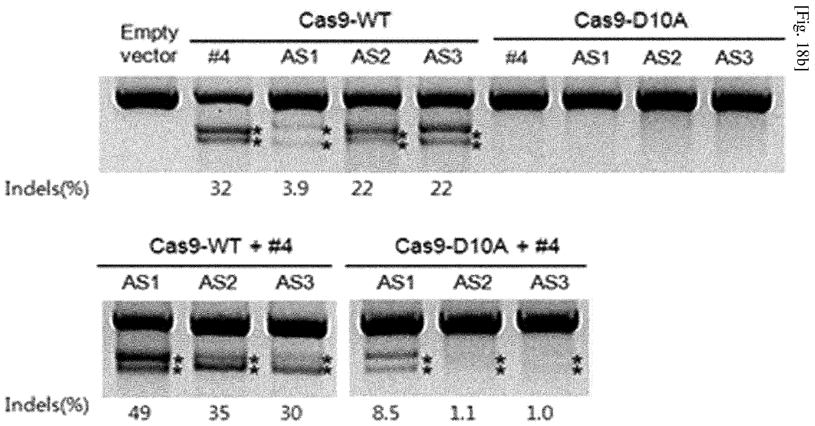

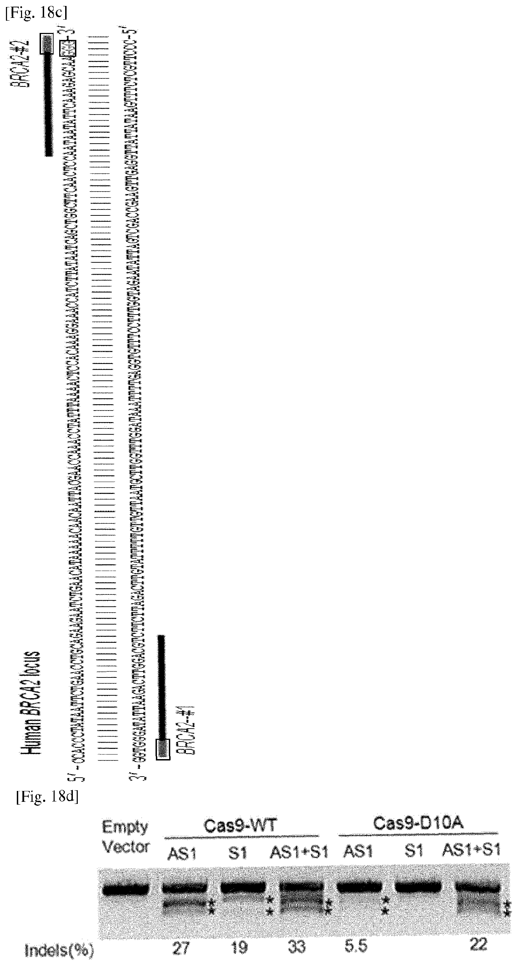

**Fig. 18** shows paired Cas9 nickases tested at other endogenous human loci. (a,c) The sgRNA target sites at human CCR5 and BRCA2 loci. PAM sequences are indicated in red. (b,d) Genome editing activities at each target site were detected by the T7E1 assay. The repair of two nicks that would produce 5' overhangs led to the formation of indels much more frequently than did those producing 3' overhangs.

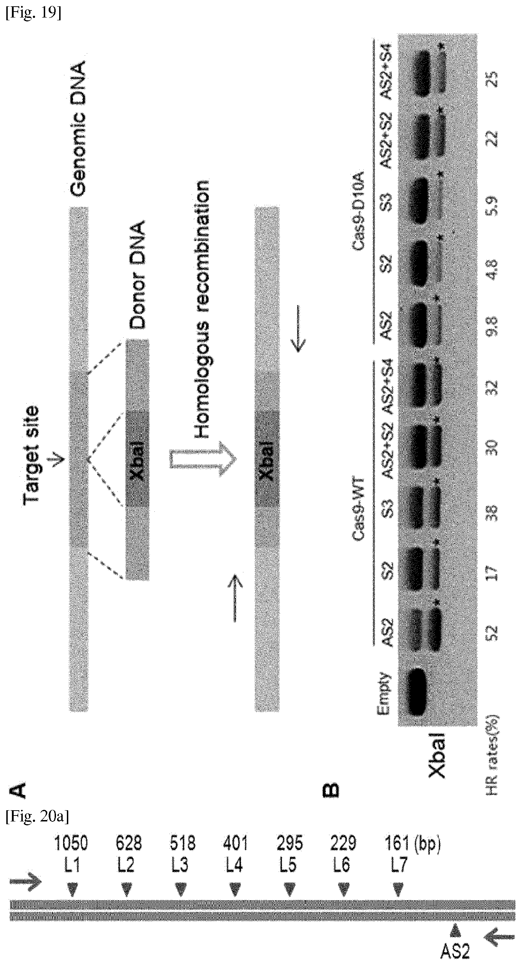

**Fig. 19** shows that paired Cas9 nickases mediate homologous recombination. (a) Strategy to detect homologous recombination. Donor DNA included an XbaI restriction enzyme site between two homology arms, whereas the endogenous target site lacked this site. A PCR assay was used to detect sequences that had undergone homologous recombination. To prevent amplification of contaminating donor DNA, primers specific to genomic DNA were used. (b) Efficiency of homologous recombination. Only amplicons of a region in which homologous recombination had occurred could be digested with XbaI; the intensities of the cleavage bands were used to measure the efficiency of this method.

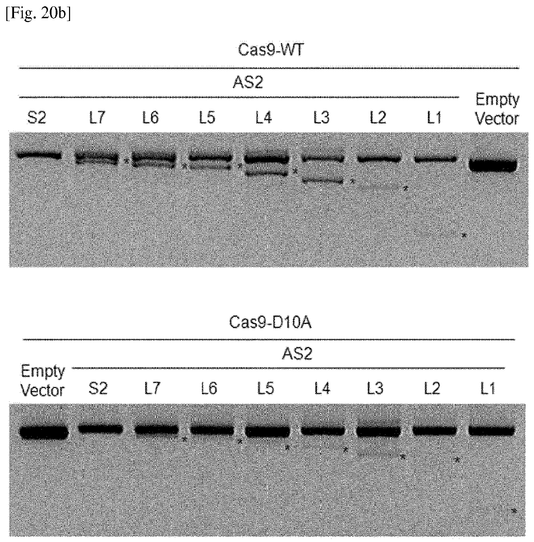

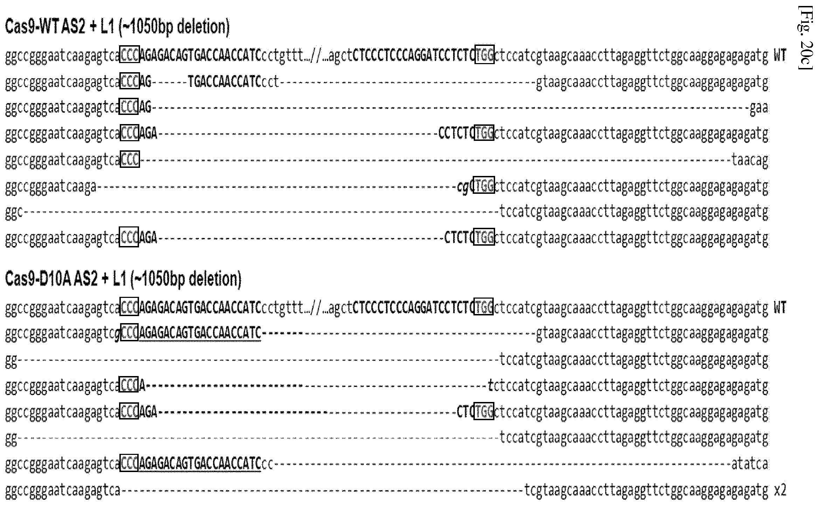

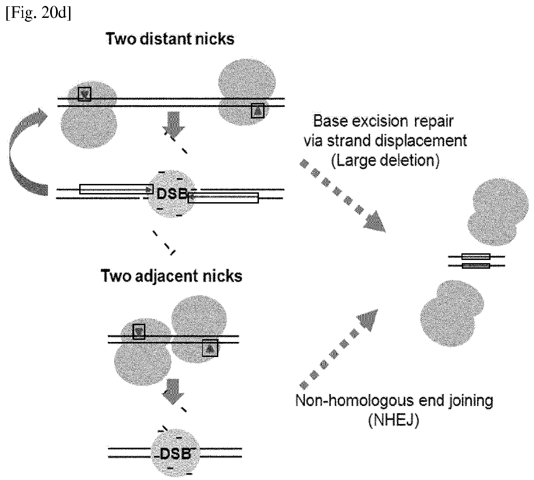

**Fig. 20** shows DNA splicing induced by paired Cas9 nickases. (a) The target sites of paired nickases in the human AAVS1 locus. The distances between the AS2 site and each of the other sites are shown. Arrows indicate PCR primers. (b) Genomic deletions detected using PCR. Asterisks indicate deletion-specific PCR products. (c) DNA sequences of deletion-specific PCR products obtained using AS2 and L1 sgRNAs. Target site PAM sequences are shown in box and sgRNA-matching sequences are shown in capital letters. Intact sgRNA-matching sequences are underlined. (d) A schematic model of paired Cas9 nickase-mediated chromosomal deletions. Newly-synthesized DNA strands are shown in box.

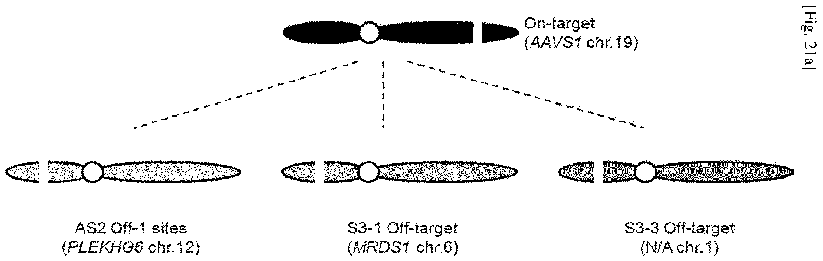

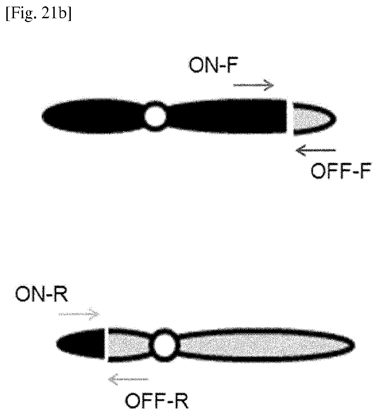

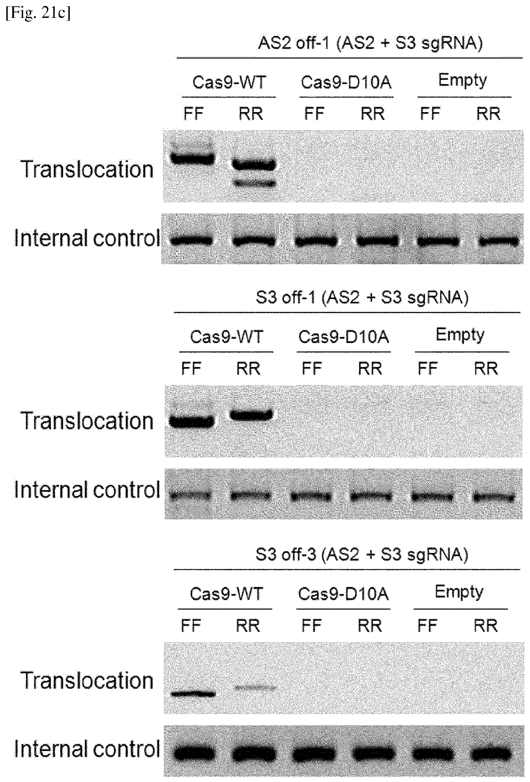

**Fig. 21** shows that paired Cas9 nickases do not induce translocations. (a) Schematic overview of chromosomal translocations between the on-target and off-target sites. (b) PCR amplification to detect chromosomal translocations. (c) Translocations induced by Cas9 nucleases but not by the nickase pair.

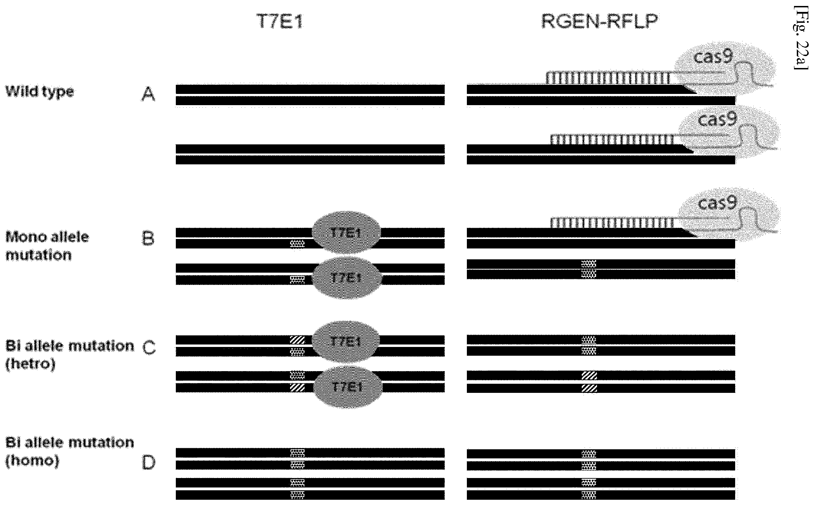

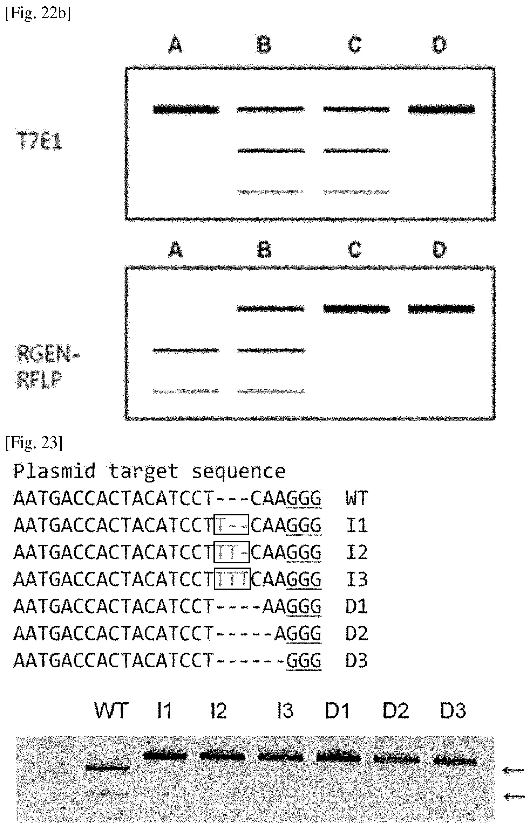

**Fig. 22** shows a conceptual diagram of the T7E1 and RFLP assays. (a) Comparison of assay cleavage reactions in four possible scenarios after engineered nuclease treatment in a diploid cell: (A) wild type, (B) a monoallelic mutation, (C) different biallelic mutations (hetero), and (D) identical biallelic mutations (homo). Black lines represent PCR products derived from each allele; dashed and dotted boxes indicate insertion/deletion mutations generated by NHEJ. (b) Expected results of T7E1 and RGEN digestion resolved by electrophoresis.
**Fig. 23** shows *in vitro* cleavage assay of a linearized plasmid containing the C4BPB target site bearing indels. DNA sequences of individual plasmid substrates (upper panel The PAM sequence is underlined. Inserted bases are shown in box. Arrows (bottom panel) indicate expected positions of DNA bands cleaved by the wild-type-specific RGEN after electrophoresis.

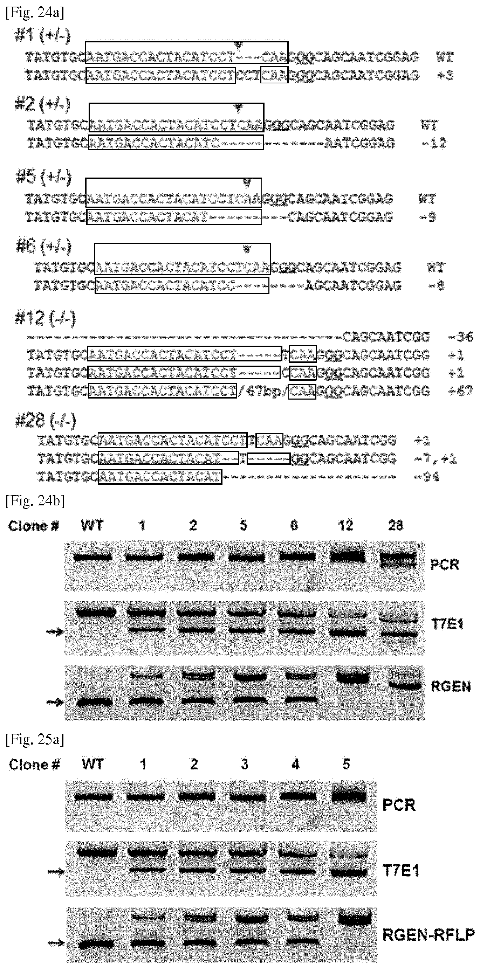

**Fig. 24** shows genotyping of mutations induced by engineered nucleases in cells via RGEN-mediated RFLP. (a) Genotype of C4BPB mutant K562 cell clones. (b) Comparison of the mismatch-sensitive T7E1 assay with RGEN-mediated RFLP analysis. Black arrows indicate the cleavage product by treatment of T7E1 enzyme or RGENs.

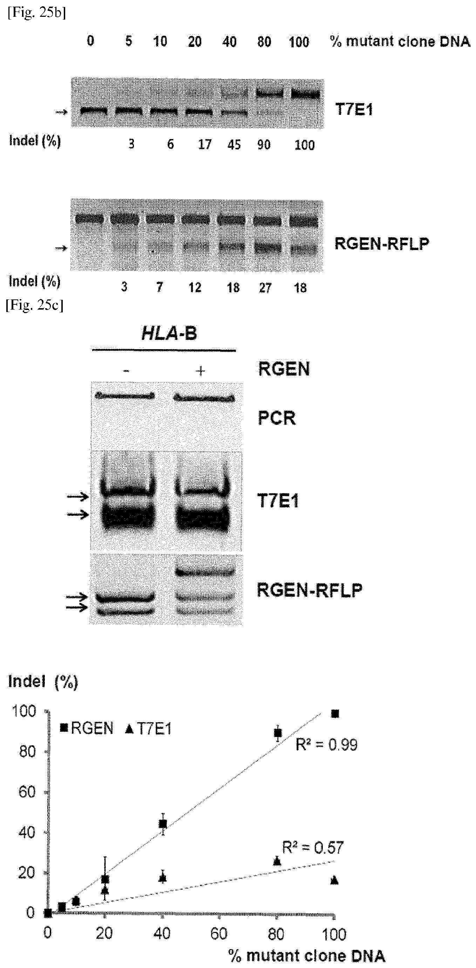

**Fig. 25** shows genotyping of RGEN-induced mutations via the RGEN-RFLP technique. (a) Analysis of C4BPB-disrupted clones using RGEN-RFLP and T7E1 assays. Arrows indicate expected positions of DNA bands cleaved by RGEN or T7E1. (b) Quantitative comparison of RGEN-RFLP analysis with T7E1 assays. Genomic DNA samples from wild-type and C4BPB-disrupted K562 cells were mixed in various ratios and subjected to PCR amplification. (c) Genotyping of RGEN-induced mutations in the HLA-B gene in HeLa cells with RFLP and T7E1 analyses.

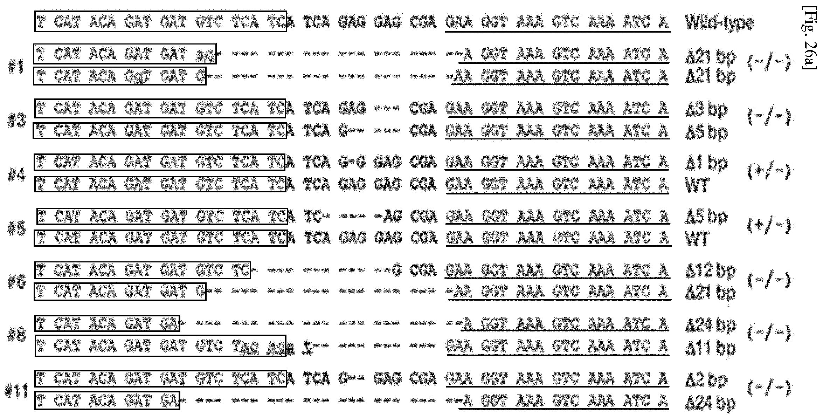

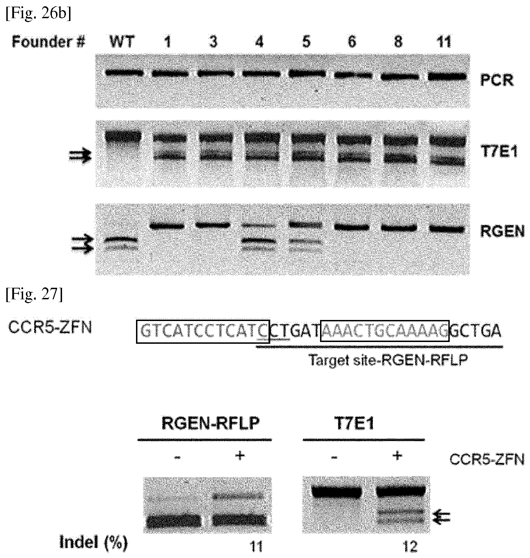

**Fig. 26** shows genotyping of mutations induced by engineered nucleases in organisms via RGEN-mediated RFLP. (a) Genotype of Pibfl mutant founder mice. (b) Comparison of the mismatch-sensitive T7E1 assay with RGEN-mediated RFLP analysis. Black arrows indicate the cleavage product by treatment of T7E1 enzyme or RGENs.
**Fig. 27** shows RGEN-mediated genotyping of ZFN-induced mutations The ZFN target site is shown in box. Black arrows indicate DNA bands cleaved by T7E1.

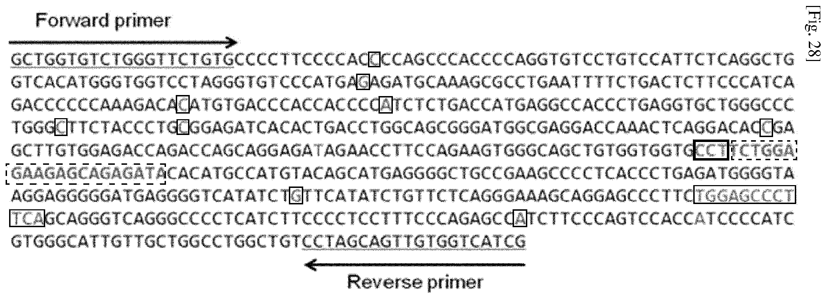

**Fig. 28** shows polymorphic sites in a region of the human HLA-B gene The sequence, which surrounds the RGEN target site, is that of a PCR amplicon from HeLa cells. Polymorphic positions are shown in box. The RGEN target site and the PAM sequence are shown in dashed and bolded box, respectively. Primer sequences are underlined.

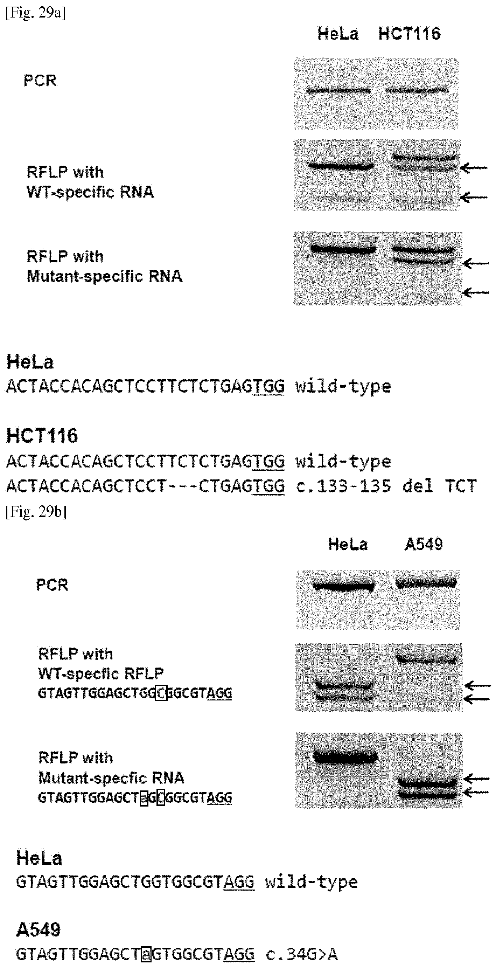

**Fig. 29** shows genotyping of oncogenic mutations via RGEN-RFLP analysis. (a) A recurrent mutation (c. 133-135 deletion of TCT) in the human CTNNB1 gene in HCT116 cells was detected by RGENs. HeLa cells were used as a negative control. (b) Genotyping of the KRAS substitution mutation (c.34 G>A) in the A549 cancer cell line with RGENs that contain mismatched guide RNA Mismatched nucleotides are shown in box. HeLa cells were used as a negative control. Arrows indicate DNA bands cleaved by RGENs. DNA sequences confirmed by Sanger sequencing are shown.

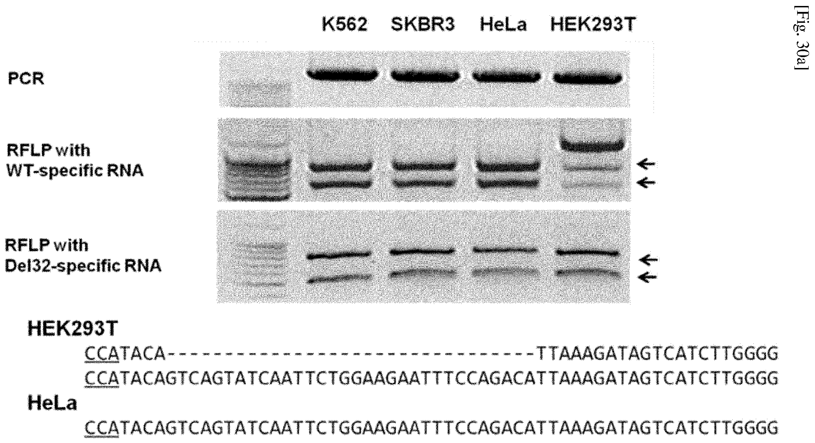

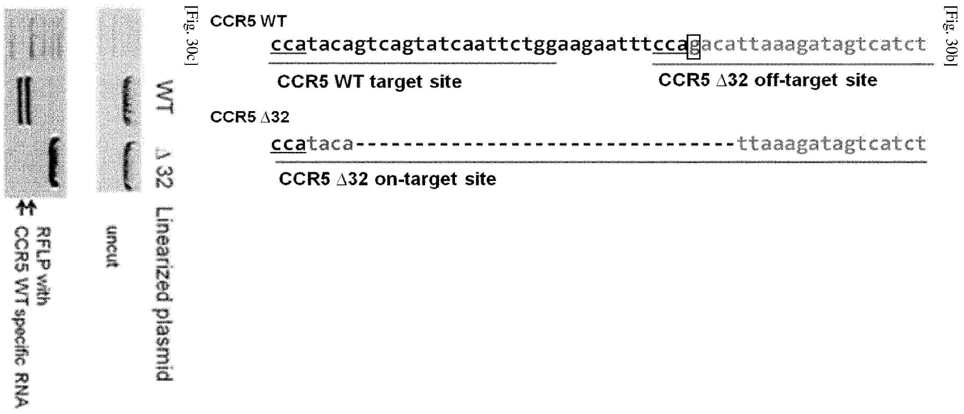

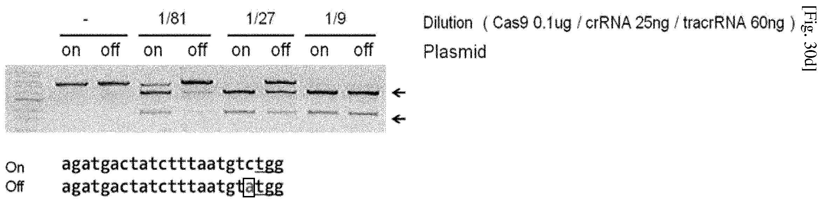

**Fig. 30** shows genotyping of the CCR5 delta32 allele in HEK293T cells via RGEN-RFLP analysis. (a) RGEN-RFLP assays of cell lines. K562, SKBR3, and HeLa cells were used as wild-type controls. Arrows indicate DNA bands cleaved by RGENs. (b) DNA sequence of wild-type and delta32 CCR5 alleles Both on-target and off-target sites of RGENs used in RFLP analysis are underlined. A single-nucleotide mismatch between the two sites is shown in box. The PAM sequence is underlined. (c) *In vitro* cleavage of plasmids harboring WT or del32 CCR5 alleles using the wild-type-specific RGEN. (d) Confirming the presence of an off-target site of the CCR5-delta32-specific RGEN at the CCR5 locus. *In vitro* cleavage assays of plasmids harboring either on-target or off-target sequences using various amounts of the del32-specific RGEN.

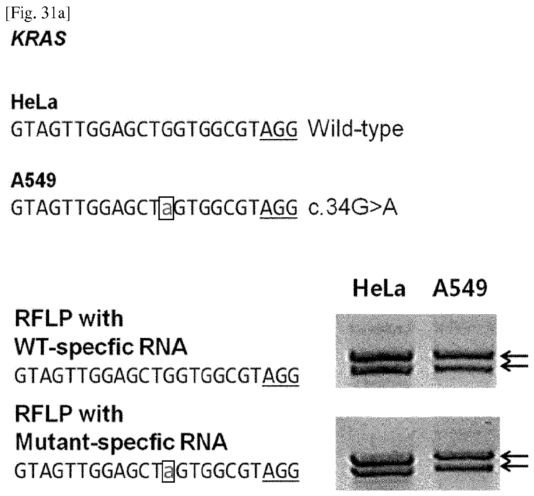

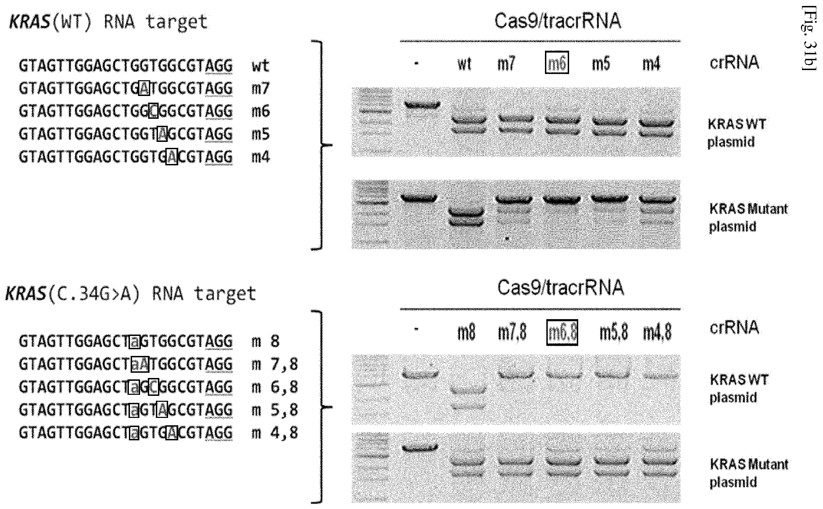

**Fig. 31** shows genotyping of a KRAS point mutation (c.34 G>A). (a) RGEN-RFLP analysis of the KRAS mutation (c.34 G>A) in cancer cell lines. PCR products from HeLa cells (used as a wild-type control) or A549 cells, which are homozygous for the point mutation, were digested with RGENs with perfectly matched crRNA specific to the wild-type sequence or the mutant sequence KRAS genotypes in these cells were confirmed by Sanger sequencing. (b) Plasmids harboring either the wild-type or mutant KRAS sequences were digested using RGENs with perfectly matched crRNAs or attenuated, one-base mismatched crRNAs Attenuated crRNAs that were chosen for genotyping are labeled in box above the gels.

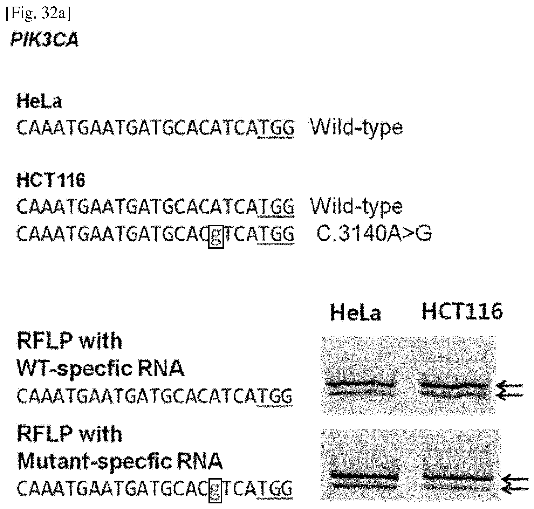

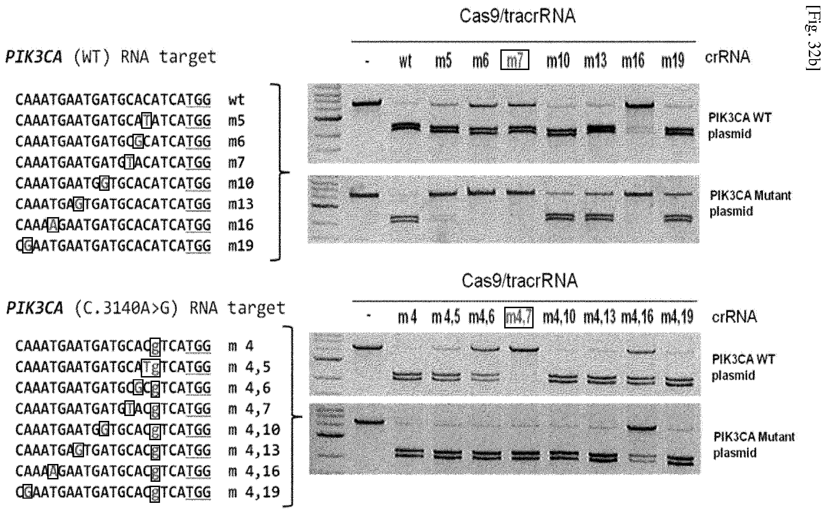

**Fig. 32** shows genotyping of a PIK3CA point mutation (c.3140 A>G). (a) RGEN-RFLP analysis of the PIK3CA mutation (c.3140 A>G) in cancer cell lines. PCR products from HeLa cells (used as a wild-type control) or HCT116 cells that are heterozygous for the point mutation were digested with RGENs with perfectly matched crRNA specific to the wild-type sequence or the mutant sequence PIK3CA genotypes in these cells were confirmed by Sanger sequencing. (b) Plasmids harboring either the wild-type or mutant PIK3CA sequences were digested using RGENs with perfectly matched crRNAs or attenuated, one-base mismatched crRNAs. Attenuated crRNAs that were chosen for genotyping are labeled in box above the gels.

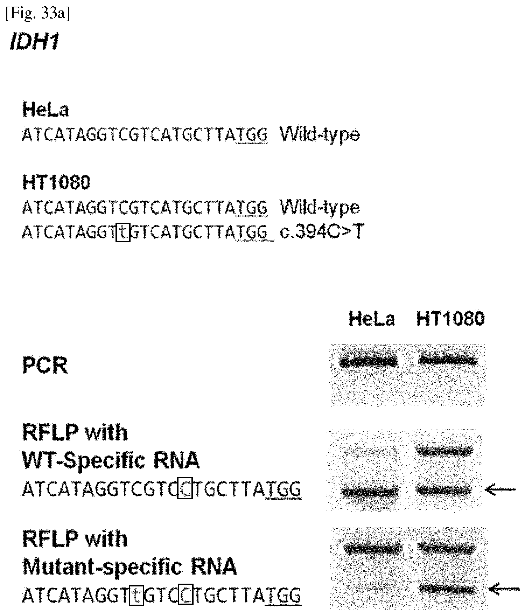

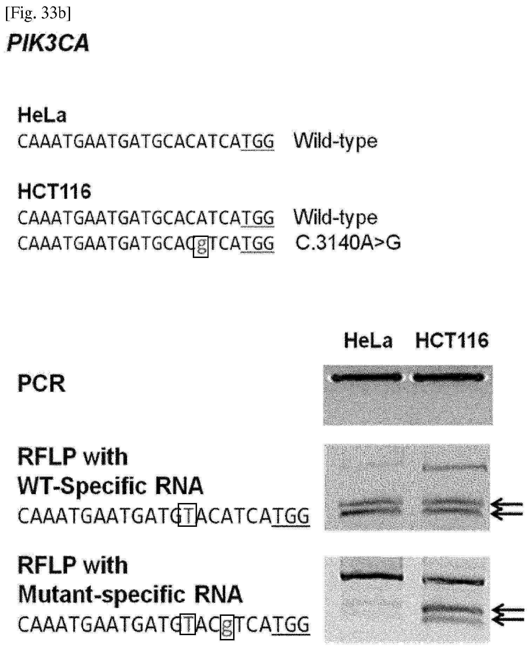

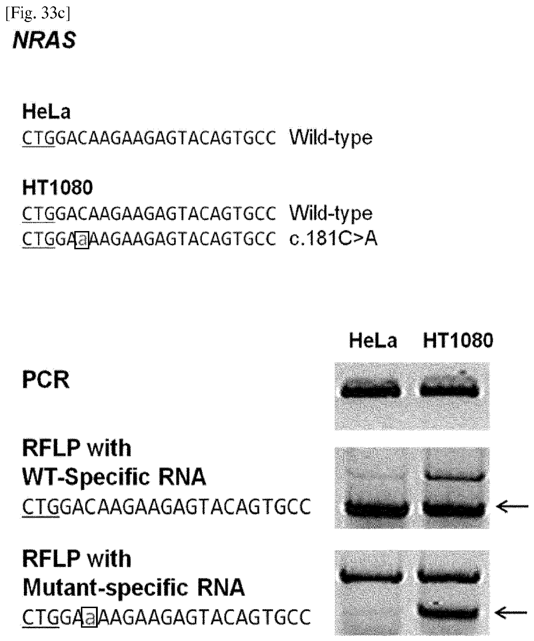

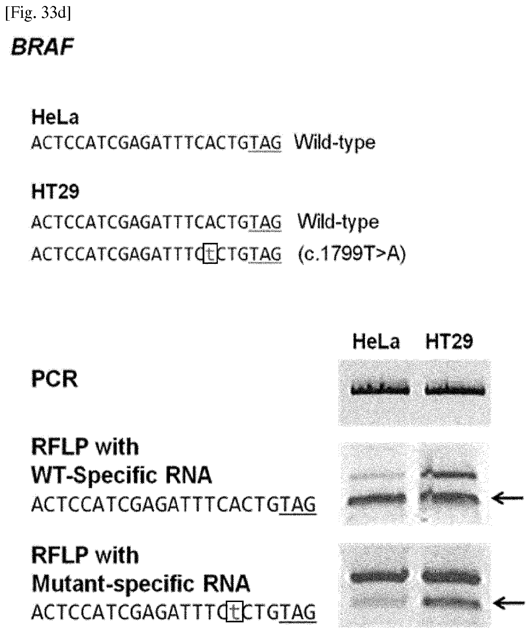

**Fig. 33** shows genotyping of recurrent point mutations in cancer cell lines. (a) RGEN-RFLP assays of recurrent oncogenic point mutations in IDH (c.394C>T, (b) PIK3CA (c.3140A>G, (c) NRAS (c.181C>A, (d) and BRAF genes (c.1799T>A Genotypes of each cell line confirmed by Sanger sequencing are shown. Mismatched nucleotides are shown in box. Black arrows indicate DNA bands cleaved by RGENs.

### Detailed Description

In accordance with one aspect, the present invention provides a composition for cleaving target DNA in plant protoplasts,
as defined in the claims. In addition, the present invention provides a use of the composition for cleaving target DNA in plant protoplasts.

In the present invention, the composition is also referred to as a RNA-guided endonuclease (RGEN) composition.

ZFNs and TALENs enable targeted mutagenesis in mammalian cells, model organisms, plants, and livestock, but the mutation frequencies obtained with individual nucleases are widely different from each other. Furthermore, some ZFNs and TALENs fail to show any genome editing activities. DNA methylation may limit the binding of these engineered nucleases to target sites. In addition, it is technically challenging and time-consuming to make customized nucleases.

The present inventors have developed a new RNA-guided endonuclease composition based on Cas9 protein to overcome the disadvantages of ZFNs and TALENs.

Prior to the present invention, an endonuclease activity of Cas proteins has been known.

Compared to ZFNs and TALENs, the present RGEN composition based on Cas9 protein can be more readily customized because only the synthetic guide RNA component is replaced to make a new genome-editing nuclease. No sub-cloning steps are involved to make customized RNA guided endonucleases. Furthermore, the relatively small size of the Cas gene (for example, 4.2 kbp for Cas9) as compared to a pair of TALEN genes (~6 kbp) provides an advantage for this RNA-guided endonuclease composition in some applications such as virus-mediated gene delivery. Further, this RNA-guided endonuclease does not have off-target effects and thus does not induce unwanted mutations, deletion, inversions, and duplications. These features make the present RNA-guided endonuclease composition a scalable, versatile, and convenient tools for genome engineering in eukaryotic cells and organisms. In addtion, RGEN can be designed to target any DNA sequence, almost any single nucleotide polymorphism or small insertion/deletion (indel) can be analyzed via RGEN-mediated RFLP. The specificity of RGENs is determined by the RNA component that hybridizes with a target DNA sequence of up to 20 base pairs (bp) in length and by the Cas9 protein that recognize the protospacer-adjacent motif (PAM). RGENs are readily reprogrammed by replacing the RNA component. Therefore, RGENs provide a platform to use simple and robust RFLP analysis for various sequence variations.

The target DNA may be an endogenous DNA, or artificial DNA, preferably, endogenous DNA.

As used herein, the term "Cas9 protein" refers to an essential protein component in the CRISPR/Cas9 system, forms an active endonuclease or nickase when complexed with two RNAs termed CRISPR RNA (crRNA) and trans-activating crRNA (tracrRNA).

The information on the gene and protein of Cas are available from GenBank of National Center for Biotechnology Information (NCBI), without limitation.

The CRISPR-associated (cas) genes encoding Cas proteins are often associated with CRISPR repeat-spacer arrays. More than forty different Cas protein families have been described. Of these protein families, Cas1 appears to be ubiquitous among different CRISPR/Cas systems. There are three types of CRISPR-Cas system. Among them, Type II CRISPR/Cas system involving Cas9 protein and crRNA and tracrRNA is representative and is well known. Particular combinations of cas genes and repeat structures have been used to define 8 CRISPR subtypes (Ecoli, Ypest, Nmeni, Dvulg, Tneap, Hmari, Apern, and Mtube).

The Cas protein may be linked to a protein transduction domain. The protein transduction domain may be poly-arginine or a TAT protein derived from HIV, but it is not limited thereto.

In the present invention, Cas9 protein may be any Cas9 protein provided that it has an endonuclease or nickase activity when complexed with a guide RNA.

The term Cas9 protein may include variants thereof. The variant of the Cas9 protein may be a mutant form of Cas9 in which the cataytic asapartate residue is changed to any other amino acid. Preferably, the other amino acid may be an alanine, but it is not limited thereto.

Further, Cas9 protein may be the one isolated from an organism such as *Streptococcus sp.,* preferably Strep*tococcus pyogens* or a recombinant protein, but it is not limited thereto.

The Cas9 protein derived from *Streptococcus pyogens* may recognizes NGG trinucleotide. The Cas9 protein may comprise an amino acid sequence of SEQ ID NO: 109, but it is not limited thereto.

The term "recombinant" when used with reference, e.g., to a cell, nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, a recombinant Cas9 protein may be generated by reconstituting Cas9 protein-encoding sequence using the human codon table.

Cas9 encoding sequence may be derived from *Streptococcus sp.,* and preferably derived from *Streptococcus pyogenes.* For example, Cas9 encoding nucleic acid may comprise the nucleotide sequence of SEQ ID. NO: 1. Moreover, Cas9-encoding nucleic acid may comprise the nucleotide sequence having homology of at least 50% to the sequence of SEQ ID NO: 1, preferably at least 60, 70, 80, 90, 95, 97, 98,or 99% to the SEQ ID NO:1, but it is not limited thereto. Cas9 encoding nucleic acid may comprise the nucleotide sequence of SEQ ID NOs.108, 110, 106, or 107.

As used herein, the term "guide RNA" refers to a RNA which is specific for the target DNA and can form a complex with Cas9 protein and bring Cas9 protein to the target DNA.

In the present invention, the guide RNA may consist of two RNA molecules, *i.e.,* CRISPR RNA(crRNA) and transactivating crRNA(tracrRNA) or be a single-chain RNA(sgRNA) produced by fusion of an essential portion of crRNA and tracrRNA.

The guide RNA may be a dualRNA comprising a crRNA and a tracrRNA.

If the guide RNA comprises the essential portion of crRNA and tracrRNA and a portion complementary to a target, any guide RNA may be used in the present invention.

The crRNA may hybridize with a target DNA.

The RGEN may consist of Cas9 protein, and dualRNA (invariable tracrRNA and target-specific crRNA), orCas9 protein and sgRNA (fusion of an essential portion of invariable tracrRNA and target-specific crRNA), and may be readily reprogrammed by replacing crRNA.

The guide RNA further comprises one or more additional nucleotides at the 5' end of the single-chain guide RNA or the crRNA of the dualRNA.

Preferably, the guide RNA further comprises 2-additional guanine nucleotides at the 5' end of the single-chain guide RNA or the crRNA of the dualRNA.

When the guide RNA is transfected in the form of an isolated RNA into a cell or organism, the guide RNA may be prepared by *in vitro* transcription using any *in vitro* transcription system known in the art. As used herein, the term "isolated RNA" may be interchangeable to "naked RNA". This is cost- and time- saving because it does not require a step of cloning.

The present RGEN composition comprising Cas9 protein and a guide RNA can specifically cleave a target DNA due to a specificity of the guide RNA for a target and an endonuclease or nickase activity of Cas9 protein.

As used herein, the term "cleavage" refers to the breakage of the covalent backbone of a nucleotide molecule.

In the present invention, a guide RNA may be prepared to be specific for any target which is to be cleaved. Therefore, the present RGEN composition can cleave any target DNA by manipulating or genotyping the target-specific portion of the guide RNA.

The guide RNA and the Cas9 protein may function as a pair. As used herein, the term "paired Cas9 nickase" may refer to the guide RNA and the Cas9 protein functioning as a pair. The pair comprises two guide RNAs. The guide RNA and Cas9 protein may function as a pair, and induce two nicks on different DNA strand. The two nicks may be separated by at least 100 bps, but are not limited thereto.

In the Examples, the present inventors confirmed that paired Cas9 nickase allow targeted mutagenesis and large deletions of up to 1-kbp chromosomal segments in human cells. Importantly, paired nickases did not induce indels at off-target sites at which their corresponding nucleases induce mutations. Furthermore, unlike nucleases, paired nickases did not promote unwanted translocations associated with off-target DNA cleavages. In principle, paired nickases double the specificity of Cas9-mediated mutagenesis and will broaden the utility of RNA-guided enzymes in applications that require precise genome editing such as gene and cell therapy.

In one specific embodiment, the guide RNA may comprise the nucleotide sequence of SEQ ID NO: 1, wherein the portion of nucleotide position 3 ~ 22 is a target-specific portion and thus, the sequence of this portion may be changed depending on a target.

As used herein, a eukaryotic cell or organism may be yeast, fungus, protozoa, plant, higher plant, and insect, or amphibian cells, or mammalian cells such as CHO, HeLa, HEK293, and COS-1, for example, cultured cells *(in vitro),* graft cells and primary cell culture *(in vitro* and *ex vivo),* and *in vivo* cells, and also mammalian cells including human, which are commonly used in the art, without limitation.

It was found that Cas9 protein/single-chain guide RNA could generate site-specific DNA double-strand breaks *in vitro* and in mammalian cells, whose spontaneous repair induced targeted genome mutations at high frequencies.

Moreover, it was found that gene-knockout mice could be induced by the injection of Cas9 protein/guide RNA complexes or Cas9 mRNA/guide RNA into one-cell stage embryo and germ-line transmittable mutations could be generated by Cas9/guide RNA system.

Using Cas9 protein rather than a nucleic acid encoding Cas9 protein to induce a targeted mutagenesis is advantageous because exogeneous DNA is not introduced into an organism. Thus, the composition comprising Cas9 protein and a guide RNA may be used to develop therapeutics or value-added crops, livestock, poultry, fish, pets, etc.

In another aspect, the present invention provides a composition for inducing targeted mutagenesis in plant protoplasts,
as defined in the claims. In addition, the present invention provides a use of the composition for inducing targeted mutagenesis in plant protoplasts.

A guide RNA, Cas9 protein-encoding nucleic acid or Cas9 protein are as described in the above.

The composition of the invention may be provided in a kit for cleaving a target DNA or inducing targeted mutagenesis in plant protoplasts.

A guide RNA, Cas9 protein-encoding nucleic acid or Cas9 protein are as described above.

There is provided a kit for cleaving a target DNA in a plant protoplast wherein the kit comprises the composition of the invention.

The present kit may comprise some additional components necessary for transferring the guide RNA and Cas9 component to a cell or an organism. For example, the kit may comprise an injection buffer such as DEPC-treated injection buffer, and materials necessary for analysis of mutation of a target DNA, but are not limited thereto.

The composition of the invention may be used in a method for preparing a plant protoplast comprising transfecting the plant protoplast with a complex comprising Cas9 protein and a guide RNA.

A guide RNA, Cas9 protein-encoding nucleic acid or Cas9 protein are as described above.

A Cas9 protein and a guide RNA are transferred into a protoplast by PEG-mediated transfection.

The protoplast is co-transfected with a Cas9 protein and a guide RNA.

The eukaryotic cells disclosed herein may be yeast, fungus, protozoa, higher plant, and insect, or amphibian cells, or mammalian cells such as CHO, HeLa, HEK293, and COS-1, for example, cultured cells *(in vitro),* graft cells and primary cell culture *(in vitro* and *ex vivo),* and *in vivo* cells, and also mammalian cells including human, which are commonly used in the art, without limitation. Further the organism may be yeast, fungus, protozoa, plant, higher plant, insect, amphibian, or mammal.

In another aspect, the present invention provides methods for cleaving a target DNA or inducing targeted mutagenesis in plant protoplasts, comprising a step of treating a plant protoplast comprising a target DNA with a composition of the invention.

The step of treating a plant protoplast with the composition may be performed by transferring the present composition comprising a guide RNA specific for target DNA, and Cas9 protein into the plant protoplast.

As described in the above, such transfer is performed by PEG-mediated transfection.

The RGEN compositions of the invention may be used to produce a plant regenerated from the genome-modified protoplasts prepared by the method of the invention.

### Examples

Hereinafter, the present invention will be described in more detail with reference to Examples. However, these Examples are for illustrative purposes only, and the invention is not intended to be limited by these Examples.

### Example 1: Genome editing assay (reference example)

### 1-1. DNA cleavage activity of Cas9 protein

Firstly, the DNA cleavage activity of Cas9 derived from *Streptococcus pyogenes* in the presence or absence of a chimeric guide RNA *in vitro* was tested.

To this end, recombinant Cas9 protein that was expressed in and purified from *E. coli* was used to cleave a predigested or circular plasmid DNA that contained the 23-base pair (bp) human CCR5 target sequence. A Cas9 target sequence consists of a 20-bp DNA sequence complementary to crRNA or a chimeric guide RNA and the trinucleotide (5'-NGG-3') protospacer adjacent motif (PAM) recognized by Cas9 itself (Fig. 1A).

Specifically, the Cas9-coding sequence (4,104 bp), derived from *Streptococcus pyogenes* strain M1 GAS (NC_002737.1), was reconstituted using the human codon usage table and synthesized using oligonucleotides. First, 1-kb DNA segments were assembled using overlapping ∼35-mer oligonucleotides and Phusion polymerase (New England Biolabs) and cloned into T-vector (SolGent). A full-length Cas9 sequence was assembled using four 1-kbp DNA segments by overlap PCR. The Cas9-encoding DNA segment was subcloned into p3s, which was derived from pcDNA3.1 (Invitrogen). In this vector, a peptide tag (NH2-GGSGPPKKKRKVYPYDVPDYA-COOH, SEQ ID NO: 2) containing the HA epitope and a nuclear localization signal (NLS) was added to the C-terminus of Cas9. Expression and nuclear localization of the Cas9 protein in HEK 293T cells were confirmed by western blotting using anti-HA antibody (Santa Cruz).

Then, the Cas9 cassette was subcloned into pET28-b(+) and transformed into BL21(DE3). The expression of Cas9 was induced using 0.5 mM IPTG for 4 h at 25 □. The Cas9 protein containing the His6-tag at the C terminus was purified using Ni-NTA agarose resin (Qiagen) and dialyzed against 20 mM HEPES (pH 7.5), 150 mM KC1, 1 mM DTT, and 10% glycerol (1). Purified Cas9 (50 nM) was incubated with super-coiled or predigested plasmid DNA (300 ng) and chimeric RNA (50 nM) in a reaction volume of 20 µl in NEB buffer 3 for 1 h at 37 □. Digested DNA was analyzed by electrophoresis using 0.8% agarose gels.

Cas9 cleaved the plasmid DNA efficiently at the expected position only in the presence of the synthetic RNA and did not cleave a control plasmid that lacked the target sequence (Fig. 1B).

### 1-2. DNA cleavage by Cas9/guide RNA complex in human cells

A RFP-GFP reporter was used to investigate whether the Cas9/guide RNA complex can cleave the target sequence incorporated between the RFP and GFP sequences in mammalian cells.

In this reporter, the GFP sequence is fused to the RFP sequence out-of-frame (2). The active GFP is expressed only when the target sequence is cleaved by site-specific nucleases, which causes frameshifting small insertions or deletions (indels) around the target sequence via error-prone non-homologous end-joining (NHEJ) repair of the double-strand break (DSB) (Fig 2).

The RFP-GFP reporter plasmids used in this study were constructed as described previously (2). Oligonucleotides corresponding to target sites (Table 1) were synthesized (Macrogen) and annealed. The annealed oligonucleotides were ligated into a reporter vector digested with EcoRI and BamHI.

HEK 293T cells were co-transfected with Cas9-encoding plasmid (0.8 µg) and the RFP-GFP reporter plasmid (0.2 µg) in a 24-well plate using Lipofectamine 2000 (Invitrogen).

Meanwhile, the *in vitro* transcribed chimeric RNA had been prepared as follows. RNA was *in vitro* transcribed through run-off reactions using the MEGAshortscript T7 kit (Ambion) according to the manufacturer's manual. Templates for RNA *in vitro* transcription were generated by annealing two complementary single strand DNAs or by PCR amplification (Table 1). Transcribed RNA was resolved on a 8% denaturing urea-PAGE gel. The gel slice containing RNA was cut out and transferred to probe elution buffer. RNA was recovered in nuclease-free water followed by phenol :chloroform extraction, chloroform extraction, and ethanol precipitation. Purified RNAs were quantified by spectrometry.

At 12h post transfection, chimeric RNA (1 µg) prepared by *in vitro* transcription was transfected using Lipofectamine 2000.

At 3d post-transfection, transfected cells were subjected to flow cytometry and cells expressing both RFP and GFP were counted.

It was found that GFP-expressing cells were obtained only when the cells were transfected first with the Cas9 plasmid and then with the guide RNA 12 h later (Fig. 2), demonstrating that RGENs could recognize and cleave the target DNA sequence in cultured human cells. Thus GFP-experssing cells were obtained by serial-transfection of the Cas9 plasmid and the guide RNA rather than co-transfection.

**Table 1**

| Gene | | sequence (5' to 3') | SEQ ID NO. |
|---|---|---|---|
| Oligonucleotides used for the construction of the reporter plasmid | | | |
| CCR5 | F | AATTCATGACATCAATTATTATACATCGGAGGA G | 3 |
| | R | GATCCTCCTCCGATGTATAATAATTGATGTCAT G | 4 |

| Primers used in the T7E1 assay | | | |
|---|---|---|---|
| CCR5 | F1 | CTCCATGGTGCTATAGAGCA | 5 |
| | F2 | GAGCCAAGCTCTCCATCTAGT | 6 |
| | R | GCCCTGTCAAGAGTTGACAC | 7 |
| C4BPB | F1 | TATTTGGCTGGTTGAAAGGG | 8 |
| | R1 | AAAGTCATGAAATAAACACACCCA | 9 |
| | F2 | CTGCATTGATATGGTAGTACCATG | 10 |
| | R2 | GCTGTTCATTGCAATGGAATG | 11 |

| Primers used for the amplification of off-target sites | | | |
|---|---|---|---|
| ADCY5 | F1 | GCTCCCACCTTAGTGCTCTG | 12 |
| | R1 | GGTGGCAGGAACCTGTATGT | 13 |
| | F2 | GTCATTGGCCAGAGATGTGGA | 14 |
| | R2 | GTCCCATGACAGGCGTGTAT | 15 |
| KCNJ6 | F | GCCTGGCCAAGTTTCAGTTA | 16 |
| | R1 | TGGAGCCATTGGTTTGCATC | 17 |
| | R2 | CCAGAACTAAGCCGTTTCTGAC | 18 |
| CNTNAP2 | F1 | ATCACCGACAACCAGTTTCC | 19 |
| | F2 | TGCAGTGCAGACTCTTTCCA | 20 |
| | R | AAGGACACAGGGCAACTGAA | 21 |
| N/A Chr. 5 | F1 | TGTGGAACGAGTGGTGACAG | 22 |
| | R1 | GCTGGATTAGGAGGCAGGATTC | 23 |
| | F2 | GTGCTGAGAACGCTTCATAGAG | 24 |
| | R2 | GGACCAAACCACATTCTTCTCAC | 25 |

| Primers used for the detection of chromosomal deletions | | | |
|---|---|---|---|
| Deletion | F | CCACATCTCGTTCTCGGTTT | 26 |
| | R | TCACAAGCCCACAGATATTT | 27 |

### 1-3. Targeted disruption of endogeneous genes in mammalian cells by RGEN

To test whether RGENs could be used for targeted disruption of endogenous genes in mammalian cells, genomic DNA isolated from transfected cells using T7 endonuclease I (T7E1), a mismatch-sensitive endonuclease that specifically recognizes and cleaves heteroduplexes formed by the hybridization of wild-type and mutant DNA sequences was analyzed (3).

To introduce DSBs in mammalian cells using RGENs, 2x10⁶ K562 cells were transfected with 20 µg of Cas9-encoding plasmid using the 4D-Nucleofector, SF Cell Line 4D-Nucleofector X Kit, Program FF-120 (Lonza) according to the manufacturer's protocol. For this experiment, K562 (ATCC, CCL-243) cells were grown in RPMI-1640 with 10% FBS and the penicillin/streptomycin mix (100 U/ml and 100 µg/ml, respectively).

After 24h, 10-40 µg of *in vitro* transcribed chimeric RNA was nucleofected into 1x10⁶ K562 cells. The *in vitro* transcribed chimeric RNA had been prepared as described in the Example 1-2.

Cells were collected two days after RNA transfection and genomic DNA was isolated. The region including the target site was PCR-amplified using the primers described in Table 1. The amplicons were subjected to the T7E1 assay as described previously (3). For sequencing analysis, PCR products corresponding to genomic modifications were purified and cloned into the T-Blunt vector using the T-Blunt PCR Cloning Kit (SolGent). Cloned products were sequenced using the M13 primer.

It was found that mutations were induced only when the cells were transfected serially with Cas9-encoding plasmid and then with guide RNA (Fig. 3). Mutation frequencies (Indels (%) in Fig. 3A) estimated from the relative DNA band intensities were RNA-dosage dependent, ranging from 1.3% to 5.1%. DNA sequencing analysis of the PCR amplicons corroborated the induction of RGEN-mediated mutations at the endogenous sites. Indels and microhomologies, characteristic of error-prone NHEJ, were observed at the target site. The mutation frequency measured by direct sequencing was 7.3% (= 7 mutant clones/96 clones), on par with those obtained with zinc finger nucleases (ZFNs) or transcription-activator-like effector nucleases (TALENs).

Serial-transfection of Cas9 plasmid and guide RNA was required to induce mutations in cells. But when plasmids that encode guide RNA, serial transfection was unnecessary and cells were co-transfected with Cas9 plasmid and guide RNA-encoding plasmid.

In the meantime, both ZFNs and TALENs have been successfully developed to disrupt the human CCR5 gene (3-6), which encodes a G-protein-coupled chemokine receptor, an essential co-receptor of HIV infection. A CCR5-specific ZFN is now under clinical investigation in the US for the treatment of AIDS (7). These ZFNs and TALENs, however, have off-target effects, inducing both local mutations at sites whose sequences are homologous to the on-target sequence (6, 8-10) and genome rearrangements that arise from the repair of two concurrent DSBs induced at on-target and off-target sites (11-12). The most striking off-target sites associated with these CCR5-specific engineered nucleases reside in the CCR2 locus, a close homolog of CCR5, located 15-kbp upstream of CCR5. To avoid off-target mutations in the CCR2 gene and unwanted deletions, inversions, and duplications of the 15-kbp chromosomal segment between the CCR5 on-target and CCR2 off-target sites, the present inventors intentionally chose the target site of our CCR5-specific RGEN to recognize a region within the CCR5 sequence that has no apparent homology with the CCR2 sequence.

The present inventors investigated whether the CCR5-specific RGEN had off-target effects. To this end, we searched for potential off-target sites in the human genome by identifying sites that are most homologous to the intended 23-bp target sequence. As expected, no such sites were found in the CCR2 gene. Instead, four sites, each of which carries 3-base mismatches with the on-target site, were found (Fig. 4A). The T7E1 assays showed that mutations were not detected at these sites (assay sensitivity, ∼0.5%), demonstrating exquisite specificities of RGENs (Fig. 4B). Furthermore, PCR was used to detect the induction of chromosomal deletions in cells separately transfected with plasmids encoding the ZFN and RGEN specific to CCR5. Whereas the ZFN induced deletions, the RGEN did not (Fig. 4C).

Next, RGENs was reprogrammed by replacing the CCR5-specific guide RNA with a newly-synthesized RNA designed to target the human C4BPB gene, which encodes the beta chain of C4b-binding protein, a transcription factor. This RGEN induced mutations at the chromosomal target site in K562 cells at high frequencies (Fig. 3B). Mutation frequencies measured by the T7E1 assay and by direct sequencing were 14% and 8.3% (= 4 mutant clones/48 clones), respectively. Out of four mutant sequences, two clones contained a single-base or two-base insertion precisely at the cleavage site, a pattern that was also observed at the CCR5 target site. These results indicate that RGENs cleave chromosomal target DNA at expected positions in cells.

### Example 2: Proteinaceous RGEN-mediated genome editing (reference example)

RGENs can be delivered into cells in many different forms. RGENs consist of Cas9 protein, crRNA, and tracrRNA. The two RNAs can be fused to form a single-chain guide RNA (sgRNA). A plasmid that encodes Cas9 under a promoter such as CMV or CAG can be transfected into cells. crRNA, tracrRNA, or sgRNA can also be expressed in cells using plasmids that encode these RNAs. Use of plasmids, however, often results in integration of the whole or part of the plasmids in the host genome. The bacterial sequences incorporated in plasmid DNA can cause unwanted immune response *in vivo.* Cells transfected with plasmid for cell therapy or animals and plants derived from DNA-transfected cells must go through a costly and lengthy regulation procedure before market approval in most developed countries. Furthermore, plasmid DNA can persist in cells for several days post-transfection, aggravating off-target effects of RGENs.

Here, we used recombinant Cas9 protein complexed with *in vitro* transcribed guide RNA to induce targeted disruption of endogenous genes in human cells. Recombinant Cas9 protein fused with the hexa-histidine tag was expressed in and purified from E. coli using standard Ni ion affinity chromatography and gel filtration. Purifed recombinant Cas9 protein was concentrated in storage buffer (20 mM HEPES pH 7.5, 150 mM KC1, 1 mM DTT, and 10% glycerol). Cas9 protein/sgRNA complex was introduced directly into K562 cells by nucleofection: 1x10⁶ K562 cells were transfected with 22.5-225 (1.4-14 µM) of Cas9 protein mixed with 100ug (29 µM) of *in vitro* transcribed sgRNA (or crRNA 40ug and tracrRNA 80ug) in 100µl solution using the 4D-Nucleofector, SF Cell Line 4D-Nucleofector X Kit, Program FF-120 (Lonza) according to the manufacturer's protocol. After nucleofection, cells were placed in growth media in 6-well plates and incubated for 48hr. When 2x10⁵ K562 cells were transfected with 1/5 scale-downed protocol, 4.5-45 µg of Cas9 protein mixed with 6-60ug of *in vitro* transcribed sgRNA (or crRNA 8 µg and tracrRNA 16 µg) were used and nucleofected in 20µl solution. Nucleofected cell were then placed in growth media in 48-well plates. After 48hr, cells were collected and genomic DNA was isolated. The genomic DNA region spanning the target site was PCR-amplified and subjected to the T7E1 assay.

As shown in Fig. 10, Cas9 protien/sgRNA complex induced targeted mutation at the CCR5 locus at frequencies that ranged from 4.8 to 38% in a sgRNA or Cas9 protein dose-dependent manner, on par with the frequency obtained with Cas9 plasmid transfection (45%). Cas9 protein/crRNA/tracrRNA complex was able to induce mutations at a frequency of 9.4%. Cas9 protein alone failed to induce mutations. When 2x10⁵ cells were transfected with 1/5 scale-downed doses of Cas9 protein and sgRNA, mutation frequencies at the CCR5 locus ranged from 2.7 to 57 % in a dose-dependent manner, greater than that obtained with co-transfection of Cas9 plasmid and sgRNA plasmid (32%).

We also tested Cas9 protein/sgRNA complex that targets the ABCC11 gene and found that this complex induced indels at a frequency of 35%, demonstrating general utility of this method.

**Table 2**

| Sequences of guide RNA | | | | |
|---|---|---|---|---|
| Target | RNA type | RNA sequence (5' to 3') | Length | SEQ ID NO |
| CCR5 | sgRNA | GGUGACAUCAAUUAUUAUACAUGUUUUAG AGCUAGAAAUAGCAAGUUAAAAUAAGGCU AGUCCGUUAUCAACUUGAAAAAGUGGCACC GAGUCGGUGCUUUUUUU | 104bp | 28 |
| | crRNA | GGUGACAUCAAUUAUUAUACAUGUUUUAG AGCUAUGCUGUUUUG | 44bp | 29 |
| | tracrRNA | GGAACCAUUCAAAACAGCAUAGCAAGUUAA AAUAAGGCUAGUCCGUUAUCAACUUGAAA | 86bp | 30 |
| | | AAGUGGCACCGAGUCGGUGCUUUUUUU | | |

### Example 3: RNA-guided genome editing in mice (reference example)

To examine the gene-targeting potential of RGENs in pronuclear (PN)-stage mouse embryos, the forkhead box N1 (Foxnl) gene, which is important for thymus development and keratinocyte differentiation (Nehls et al., 1996), and the protein kinase, DNA activated, catalytic polypeptide (Prkdc) gene, which encodes an enzyme critical for DNA DSB repair and recombination (Taccioli et al., 1998) were used.

To evaluate the genome-editing activity of the Foxnl-RGEN, we injected Cas9 mRNA (10-ng/µl solution) with various doses of the sgRNA (Fig. 5a) into the cytoplasm of PN-stage mouse embryos, and conducted T7 endonuclease I (T7E1) assays (Kim et al. 2009) using genomic DNAs obtained from *in vitro* cultivated embryos (Fig. 6a).

Alternatively, we directly injected the RGEN in the form of recombinant Cas9 protein (0.3 to 30 ng/µl) complexed with the two-fold molar excess of Foxnl-specific sgRNA (0.14 to 14 ng/µl) into the cytoplasm or pronucleus of one-cell mouse embryos, and analyzed mutations in the FoxnI gene using *in vitro* cultivated embryos (Fig. 7).

Specifically, Cas9 mRNA and sgRNAs were synthesized *in vitro* from linear DNA templates using the mMESSAGE mMACHINE T7 Ultra kit (Ambion) and MEGAshortscript T7 kit (Ambion), respectively, according to the manufacturers' instructions, and were diluted with appropriate amounts of diethyl pyrocarbonate (DEPC, Sigma)-treated injection buffer (0.25 mM EDTA, 10 mM Tris, pH 7.4). Templates for sgRNA synthesis were generated using oligonucleotides listed in Table 3. Recombinant Cas9 protein was obtained from ToolGen, Inc.

**Table 3**

| RNA Name | Direction | Sequence (5' to 3') | SEQ ID NO |
|---|---|---|---|
| Foxn1 #1 sgRNA | F | GAAATTAATACGACTCACTATAGGCAGTCT GACGTCACACTTCCGTTTTAGAGCTAGAAA TAGCAAGTTAAAATAAGGCTAGTCCG | 31 |
| Foxn1 #2 sgRNA | F | GAAATTAATACGACTCACTATAGGACTTCC AGGCTCCACCCGACGTTTTAGAGCTAGAAA TAGCAAGTTAAAATAAGGCTAGTCCG | 32 |
| Foxn1 #3 sgRNA | F | GAAATTAATACGACTCACTATAGGCCAGG CTCCACCCGACTGGAGTTTTAGAGCTAGAA ATAGCAAGTTAAAATAAGGCTAGTCCG | 33 |
| Foxn1 #4 sgRNA | F | GAAATTAATACGACTCACTATAGGACTGGA GGGCGAACCCCAAGGTTTTAGAGCTAGAAA TAGCAAGTTAAAATAAGGCTAGTCCG | 34 |
| Foxn1 #5 sgRNA | F | GAAATTAATACGACTCACTATAGGACCCCA AGGGGACCTCATGCGTTTTAGAGCTAGAAA TAGCAAGTTAAAATAAGGCTAGTCCG | 35 |
| Prkdc #1 sgRNA | F | GAAATTAATACGACTCACTATAGGTTAGTT TTTCCAGAGACTTGTTTTAGAGCTAGAAAT AGCAAGTTAAAATAAGGCTAGTCCG | 36 |
| Prkdc #2 sgRNA | F | GAAATTAATACGACTCACTATAGGTTGGTT TGCTTGTGTTTATCGTTTTAGAGCTAGAAAT AGCAAGTTAAAATAAGGCTAGTCCG | 37 |
| Prkdc #3 sgRNA | F | GAAATTAATACGACTCACTATAGGCACAAG CAAACCAAAGTCTCGTTTTAGAGCTAGAAAT AGCAAGTTAAAATAAGGCTAGTCCG | 38 |
| Prkdc #4 sgRNA | F | GAAATTAATACGACTCACTATAGGCCTCAA TGCTAAGCGACTTCGTTTTAGAGCTAGAAA TAGCAAGTTAAAATAAGGCTAGTCCG | 39 |

All animal experiments were performed in accordance with the Korean Food and Drug Administration (KFDA) guidelines. Protocols were reviewed and approved by the Institutional Animal Care and Use Committees (IACUC) of the Laboratory Animal Research Center at Yonsei University (Permit Number: 2013-0099). All mice were maintained in the specific pathogen-free facility of the Yonsei Laboratory Animal Research Center. FVB/NTac (Taconic) and ICR mouse strains were used as embryo donors and foster mothers, respectively. Female FVB/NTac mice (7-8 weeks old) were super-ovulated by intra-peritoneal injections of 5 IU pregnant mare serum gonadotropin (PMSG, Sigma) and 5 IU human chorionic gonadotropin (hCG, Sigma) at 48-hour intervals. The super-ovulated female mice were mated to FVB/NTac stud males, and fertilized embryos were collected from oviducts.

Cas9 mRNA and sgRNAs in M2 medium (Sigma) were injected into the cytoplasm of fertilized eggs with well-recognized pronuclei using a Piezo-driven micromanipulator (Prime Tech).

In the case of injection of recombinant Cas9 protein, the recombinant Cas9 protein: Foxnl-sgRNA complex was diluted with DEPC-treated injection buffer (0.25 mM EDTA, 10 mM Tris, pH 7.4) and injected into male pronuclei using a TransferMan NK2 micromanipulator and a FemtoJet microinjector (Eppendorf).

The manipulated embryos were transferred into the oviducts of pseudopregnant foster mothers to produce live animals, or were cultivated *in vitro* for further analyses.

To screen F0 mice and *in vitro* cultivated mouse embryos with RGEN-induced mutations, T7E1 assays were performed as previously described using genomic DNA samples from tail biopsies and lysates of whole embryos (Cho et al., 2013).

Briefly, the genomic region encompassing the RGEN target site was PCR-amplified, melted, and re-annealed to form heteroduplex DNA, which was treated with T7 endonuclease 1 (New England Biolabs), and then analyzed by agarose gel electrophoresis. Potential off-target sites were identified by searching with bowtie 0.12.9 and were also similarly monitored by T7E1 assays. The primer pairs used in these assays were listed in Tables 4 and 5.

**Table 4**

| Primers used in the T7E1 assay | | | |
|---|---|---|---|
| Gene | Direction | Sequence(5' to 3') | SEQ ID NO |
| Foxn1 | F1 | GTCTGTCTATCATCTCTTCCCTTCTCTCC | 40 |
| | F2 | TCCCTAATCCGATGGCTAGCTCCAG | 41 |
| | R1 | ACGAGCAGCTGAAGTTAGCATGC | 42 |
| | R2 | CTACTCAATGCTCTTAGAGCTACCAGGCTTGC | 43 |
| Prkdc | F | GACTGTTGTGGGGAGGGCCG | 44 |
| | F2 | GGGAGGGCCGAAAGTCTTATTTTG | 45 |
| | R1 | CCTGAAGACTGAAGTTGGCAGAAGTGAG | 46 |
| | R2 | CTTTAGGGCTTCTTCTCTACAATCACG | 47 |

**Table 5**

| Primers used for amplification of off-target sites | | | | |
|---|---|---|---|---|
| Gene | Notation | Direction | Sequence(5' to 3') | SEQ ID NO |
| Foxnl | off 1 | F | CTCGGTGTGTAGCCCTGAC | 48 |
| | | R | AGACTGGCCTGGAACTCACAG | 49 |
| | off 2 | F | CACTAAAGCCTGTCAGGAAGCCG | 50 |
| | | R | CTGTGGAGAGCACACAGCAGC | 51 |
| | off 3 | F | GCTGCGACCTGAGACCATG | 52 |
| | | R | CTTCAATGGCTTCCTGCTTAGGCT AC | 53 |
| | off 4 | F | GGTTCAGATGAGGCCATCCTTTC | 54 |
| | | R | CCTGATCTGCAGGCTTAACCCTT G | 55 |
| | | | | |
| Prkdc | off 1 | F | CTCACCTGCACATCACATGTGG | 56 |
| | | R | GGCATCCACCCTATGGGGTC | 57 |
| | off 2 | F | GCCTTGACCTAGAGCTTAAAGAG CC | 58 |
| | | R | GGTCTTGTTAGCAGGAAGGACAC TG | 59 |
| | off 3 | F | AAAACTCTGCTTGATGGGATATG TGGG | 60 |
| | | R | CTCTCACTGGTTATCTGTGCTCCT TC | 61 |
| | off 4 | F | GGATCAATAGGTGGTGGGGGATG | 62 |
| | | R | GTGAATGACACAATGTGACAGCT TCAG | 63 |
| | off 5 | F | CACAAGACAGACCTCTCAACATT CAGTC | 64 |
| | | R | GTGCATGCATATAATCCATTCTG ATTGCTCTC | 65 |
| | off 6 | F1 | GGGAGGCAGAGGCAGGT | 66 |
| | | F2 | GGATCTCTGTGAGTTTGAGGCCA | 67 |
| | | R1 | GCTCCAGAACTCACTCTTAGGCT C | 68 |

Mutant founders identified by the T7E1 assay were further analyzed by fPCR. Appropriate regions of genomic DNA were sequenced as described previously (Sung et al., 2013). For routine PCR genotyping of F1 progenies, the following primer pairs were used for both wild-type and mutant alleles: 5'-CTACTCCCTCCGCAGTCTGA-3' (SEQ ID NO: 69) and 5'-CCAGGCCTAGGTTCCAGGTA-3' (SEQ ID NO: 70) for the Foxnl gene, 5'-CCCCAGCATTGCAGATTTCC-3' (SEQ ID NO: 71) and 5'-AGGGCTTCTTCTCTACAATCACG-3' (SEQ ID NO: 72) for Prkdc gene.

In the case of injection of Cas9 mRNA, mutant fractions (the number of mutant embryos/the number of total embryos) were dose-dependent, ranging from 33% (1 ng/µl sgRNA) to 91% (100 ng/µl) (Fig. 6b). Sequence analysis confirmed mutations in the Foxnl gene; most mutations were small deletions (Fig. 6c), reminiscent of those induced by ZFNs and TALENs (Kim et al., 2013).

In the case of injection of Cas9 protein, these injection doses and methods minimally affected the survival and development of mouse embryos *in vitro:* over 70% of RGEN-injected embryos hatched out normally in both experiments. Again, mutant fractions obtained with Cas9 protein injection were dose-dependent, and reached up to 88% at the highest dose via pronucleus injection and to 71% via intra-cytoplasmic injection (Figs. 7a and 7b). Similar to the mutation patterns induced by Cas9 mRNA plus sgRNA (Fig. 6c), those induced by the Cas9 protein-sgRNA complex were mostly small deletions (Fig. 7c). These results clearly demonstrate that RGENs have high gene-targeting activity in mouse embryos.

Encouraged by the high mutant frequencies and low cytotoxicity induced by RGENs, we produced live animals by transferring the mouse embryos into the oviducts of pseudopregnant foster mothers.

Notably, the birth rates were very high, ranging from 58% to 73%, and were not affected by the increasing doses of Foxnl-sgRNA (Table 6).

**Table 6**

| RGEN-mediated gene-targeting in FVB/NTac mice | | | | | | |
|---|---|---|---|---|---|---|
| Target Gene | Cas9 mRNA + sgRNA (ng/µl) | Inj ected embryos | Transferred embryos (%) | Total newborns (% ) | Live newborns* (%) | Founders† ( %) |
| Foxn1 | 10 + 1 | 76 | 62 (82) | 45 (73) | 31 (50) | 12 (39) |
| | 10 + 10 | 104 | 90 (87) | 52 (58) | 58 (64) | 33 (57) |
| | 10 + 100 | 100 | 90 (90) | 62 (69) | 58 (64) | 54 (93) |
| | Total | 280 | 242 (86) | 159 (66) | 147 (61) | 99 (67) |
| Prkdc | 50 + 50 | 73 | 58 (79) | 35 (60) | 33 (57) | 11 (33) |
| | 50 + 100 | 79 | 59 (75) | 22 (37) | 21 (36) | 7 (33) |
| | 50 + 250 | 94 | 73 (78) | 37 (51) | 37 (51) | 21 (57) |
| | Total | 246 | 190 (77) | 94 (49) | 91 (48) | 39 (43) |

Out of 147 newborns, we obtained 99 mutant founder mice. Consistent with the results observed in cultivated embryos (Fig. 6b), mutant fractions were proportional to the doses of Foxnl-sgRNA, and reached up to 93% (100 ng/µl Foxnl-sgRNA) (Tables 6 and 7, Fig. 5b).

**Table 7**

| DNA sequences of Foxnl mutant alleles identified from a subset of T7E1-positive mutant founders | | | |
|---|---|---|---|
| ACTTCCAGGCTCCACCCGACTGGAGGGCGAACCCCAA GGGGACCTCATGCAGG | del+ins | # | Founder mice |
| ACTTCCAGGC------------------ AACCCCAAGGGGACCTCATGCAGG | Δ19 | 1 | 20 |
| ACTTCCAGGC----------------- GAACCCCAAGGGGACCTCATGCAGG | Δ18 | 1 | 115 |
| ACTTCCAGGCTCC | Δ60 | 1 | 19 |
| ACTTCCAGGCTCC | Δ44 | 1 | 108 |
| ACTTCCAGGCTCC-------------------- CAAGGGGACCTCATGCAGG | Δ21 | 1 | 64 |
| ACTTCCAGGCTCC------------ TTAGGAGGCGAACCCCAAGGGGACCTCA | Δ12+6 | 1 | 126 |
| ACTTCCAGGCTCCACC------------- TCATGCAGG | Δ28 | 1 | 5 |
| ACTTCCAGGCTCCACCC--------------------- CCAAGGGACCTCATG | Δ21+4 | 1 | 61 |
| ACTTCCAGGCTCCACCC------------------ AAGGGGACCTCATGCAGG | Δ18 | 2 | 95, 29 |
| ACTTCCAGGCTCCACCC------------------ CAAGGGGACCTCATGCAGG | Δ17 | 7 | 12, 14, 27, 66, 108, 114, 126 |
| ACTTCCAGGCTCCACCC--------------- ACCCAAGGGGACCTCATGCAG | Δ15+1 | 1 | 32 |
| ACTTCCAGGCTCCACCC---------------- CACCCAAGGGGACCTCATGCA | Δ15+2 | 1 | 124 |
| ACTTCCAGGCTCCACCC------------- ACCCCAAGGGGACCTCATGCAGG | Δ13 | 1 | 32 |
| ACTTCCAGGCTCCACCC-------- GGCGAACCCCAAGGGGACCTCATGCAGG | Δ8 | 1 | 110 |
| ACTTCCAGGCTCCACCCT------------------- GGGGACCTCATGCAGG | Δ20+1 | 1 | 29 |
| ACTTCCAGGCTCCACCCG----------- AACCCCAAGGGGACCTCATGCAGG | Δ11 | 1 | 111 |
| ACTTCCAGGCTCCACCCGA---------------------- ACCTCATGCAGG | Δ22 | 1 | 79 |
| ACTTCCAGGCTCCACCCGA------------------ GGGGACCTCATGCAGG | Δ18 | 2 | 13, 127 |
| ACTTCCAGGCTCCACCCCA------------- AGGGGACCTCATGCAGG | Δ17 | 1 | 24 |
| ACTTCCAGGCTCCACCCGA----------- ACCCCAAGGGGACCTCATGCAGG | Δ11 | 5 | 14, 53, 58, 69, 124 |
| ACTTCCAGGCTCCACCCGA---------- GACCCCAAGGGGACCTCATGCAGG | Δ10 | 1 | 14 |
| ACTTCCAGGCTCCACCCGA----- GGGCGAACCCCAAGGGGACCTCATGCAGG | Δ5 | 3 | 53, 79, 115 |
| ACTTCCAGGCTCCACCCGAC---------------------- CTCATGCAGG | Δ23 | 1 | 108 |
| ACTTCCAGGCTCCACCCGAC----------- CCCCAAGGGGACCTCATGCAGG | Δ11 | 1 | 3 |
| ACTTCCAGGCTCCACCCGAC----------- GAAGGGCCCCAAGGGGACCTCA | Δ11+6 | 1 | 66 |
| ACTTCCAGGCTCCACCCGAC-------- GAACCCCAAGGGGACCTCATGCAGG | Δ8 | 2 | 3, 66 |
| ACTTCCAGGCTCCACCCGAC----- GGCGAACCCCAAGGGGACCTCATGCAGG | Δ5 | 1 | 27 |
| ACTTCCAGGCTCCACCCGAC-- GTGCTTGAGGGCGAACCCCAAGGGGACCTCA | Δ2+6 | 2 | 5 |
| ACTTCCAGGCTCCACCCGACT------ CACTATCTTCTGGGCTCCTCCATGTC | Δ6+25 | 2 | 21, 114 |
| ACTTCCAGGCTCCACCCGACT---- TGGCGAACCCCAAGGGGACCTCATGCAG | Δ4+1 | 1 | 53 |
| ACTTCCAGGCTCCACCCGACT-- TGCAGGGCGAACCCCAAGGGGACCTCATGC | Δ2+3 | 1 | 126 |
| ACTTCCAGGCTCCACCCGACTTGGAGGGCGAACCCCA AGGGGACCTCATGCAG | +1 | 15 | 3, 5, 12, 19, 29, 55, 56, 61, 66, 68, 81, 108, 111, 124, 127 |
| ACTTCCAGGCTCCACCCGACTTTGGAGGGCGAACCCC AAGGGGACCTCATGCA | +2 | 2 | 79, 120 |
| ACTTCCAGGCTCCACCCGACTGTTGGAGGGCGAACCC CAAGGGGACCTCATGC | +3 | 1 | 55 |
| ACTTCCAGGCTCCACCCGACTGGAG(+455)GGCGAACC CCAAGGGGACCTCC | +455 | 1 | 13 |

To generate Prkdc-targeted mice, we applied a 5-fold higher concentration of Cas9 mRNA (50 ng/µl) with increasing doses of Prkdc-sgRNA (50, 100, and 250 ng/µl). Again, the birth rates were very high, ranging from 51% to 60%, enough to produce a sufficient number of newborns for the analysis (Table 6). The mutant fraction was 57% (21 mutant founders among 37 newborns) at the maximum dose of Prkdc-sgRNA. These birth rates obtained with RGENs were approximately 2- to 10-fold higher than those with TALENs reported in our previous study (Sung et al., 2013). These results demonstrate that RGENs are potent gene-targeting reagents with minimal toxicity.

To test the germ-line transmission of the mutant alleles, we crossed the Foxnl mutant founder #108, a mosaic with four different alleles (Fig. 5c, and Table 8) with wild-type mice, and monitored the genotypes of F1 offspring.

**Table 8**

| Genotypes of Foxnl mutant mice | | | |
|---|---|---|---|
| **Founder NO.** | **sgRNA (ng/ml)** | **Genotyping Summary** | **Detected alleles** |
| 58* | 1 | not determined | Δ11 |
| 19 | 100 | bi-allelic | Δ60/+1 |
| 20 | 100 | bi-allelic | Δ67/Δ19 |
| 13 | 100 | bi-allelic | Δ18/+455 |
| 32 | 10 | bi-allelic (heterozygote) | Δ13/Δ15+1 |
| 115 | 10 | bi-allelic (heterozygote) | Δ18/Δ5 |
| 111 | 10 | bi-allelic (heterozygote) | Δ11/+1 |
| 110 | 10 | bi-allelic (homozygote) | Δ8/Δ8 |
| 120 | 10 | bi-allelic (homozygote) | +2/+2 |
| 81 | 100 | heterozygote | +1/WT |
| 69 | 100 | homozygote | Δ11/Δ11 |
| 55 | 1 | mosaic | Δ18/Δ1/+1/+3 |
| 56 | 1 | mosaic | Δ127/Δ41/Δ2/+1 |
| 127 | 1 | mosaic | Δ18/+1/WT |
| 53 | 1 | mosaic | Δ11/Δ5/Δ4+1/WT |
| 27 | 10 | mosaic | Δ17/Δ5/WT |
| 29 | 10 | mosaic | Δ18/Δ20+1/+1 |
| 95 | 10 | mosaic | Δ18/Δ14/Δ8/Δ4 |
| 108 | 10 | mosaic | +1/Δ17/Δ23/Δ44 |
| 114 | 10 | mosaic | Δ17/Δ8/Δ6+25 |
| 124 | 10 | mosaic | Δ11/Δ15+2/+1 |
| 126 | 10 | mosaic | Δ17/Δ2+3/Δ12+6 |
| 12 | 100 | mosaic | Δ30/Δ28/Δ17/+1 |
| 5 | 100 | mosaic | Δ28/Δ11/Δ2+6/+1 |
| 14 | 100 | mosaic | Δ17/Δ11/Δ10 |
| 21 | 100 | mosaic | Δ127/Δ41/Δ2/Δ6+25 |
| 24 | 100 | mosaic | Δ17/+1/WT |
| 64 | 100 | mosaic | Δ31/Δ21/+1/WT |
| 68 | 100 | mosaic | Δ17/Δ11/+1/WT |
| 79 | 100 | mosaic | Δ22/Δ5/+2/WT |
| 61 | 100 | mosaic | Δ21+4/Δ6/+1/+9 |
| 66** | 100 | mosaic | Δ17/Δ8/Δ11+6/+1/WT |
| 3 | 100 | mosaic | Δ11/Δ8/+1 |
| Underlined alleles were sequenced. | | | |
| Alleles in red, detected by sequencing, but not by fPCR. | | | |
| *only one clone sequenced. | | | |
| **Not determined by fPCR. | | | |

As expected, all the progenies were heterozygous mutants possessing the wild-type allele and one of the mutant alleles (Fig. 5d). We also confirmed the germ-line transmission in independent founder mice of Foxnl (Fig. 8) and Prkdc (Fig. 9). To the best of our knowledge, these results provide the first evidence that RGEN-induced mutant alleles are stably transmitted to F1 progenies in animals.

### Example 4: RNA-guided genome editing in plants

### 4-1. Production of Cas9 protein

The Cas9 coding sequence (4104 bps), derived from Streptococcus pyogenes strain M1 GAS (NC_002737.1), was cloned to pET28-b(+) plasmid. A nuclear targeting sequence (NLS) was included at the protein N terminus to ensure the localization of the protein to the nucleus. pET28-b(+) plasmid containing Cas9 ORF was transformed into BL21(DE3). Cas9 was then induced using 0.2mM IPTG for 16 hrs at 18°C and purified using Ni-NTA agarose beads (Qiagen) following the manufacturer's instructions. Purified Cas9 protein was concentrated using Ultracel - 100K (Millipore).

### 4-2. Production of guide RNA

The genomic sequence of the Arabidopsis gene encoding the BRI1 was screened for the presence of a NGG motif, the so called protospacer adjacent motif (PAM), in an exon which is required for Cas9 targeting To disrupt the BRI1 gene in Arabidopsis, we identified two RGEN target sites in an exon that contain the NGG motif. sgRNAs were produced in vitor using template DNA. Each template DNA was generated by extension with two partially overlapped oligonucleotides (Macrogen, Table X1) and Phusion polymerase (Thermo Scientific) using the following conditions - 98°C 30 sec {98°C 10 sec, 54°C 20 sec, 72°C 2 min}x20, 72°C 5 min.

**Table 9**

| Oligonucleotides for the production of the template DNA for *in vitro* transcription | | |
|---|---|---|
| Oligonucleotides | Sequence (5'-3') | SEQ ID NO |
| BRI1 target 1 (Forward) | GAAATTAATACGACTCACTATAGGTTTGAA AGATGGAAGCGCGGGTTTTAGAGCTAGAA ATAGCAAGTTAAAATAAGGCTAGTCCG | 73 |
| BRI1 target 2 (Forward) | GAAATTAATACGACTCACTATAGGTGAAAC TAAACTGGTCCACAGTTTTAGAGCTAGAAA TAGCAAGTTAAAATAAGGCTAGTCCG | 74 |
| Universal (Reverse) | AAAAAAGCACCGACTCGGTGCCACTTTTTC AAGTTGATAACGGACTAGCCTTATTTTAAC | 75 |
| | TTGC | |

The extended DNA was purified and used as a template for the *in vitro* production of the guide RNA's using the MEGAshortscript T7 kit (Life Technologies). Guide RNA were then purified by Phenol/Chloroform extraction and ethanol precipitation. To prepare Cas9/sgRNA complexes, 10 ul of purified Cas9 protein (12 µg/µl) and 4 ul each of two sgRNAs (11 µg/µl) were mixed in 20 µl NEB3 buffer (New England Biolabs) and incubated for 10 min at 37°C.

### 4-3. Transfection of Cas9/sgRNA complex to protoplast

The leaves of 4-week-old Arabidopsis seedlings grown aseptically in petri dishes were digested in enzyme solution (1% cellulose R10, 0.5% macerozyme R10, 450 mM mannitol, 20mM MES pH 5.7 and CPW salt) for 8∼16 hrs at 25°C with 40 rpm shaking in the dark. Enzyme/protoplast solutions were filtered and centrifuged at 100 X g for 3∼5 min. Protoplasts were re-suspended in CPW solution after counting cells under the microscope (X100) using a hemacytometer. Finally, protoplasts were re-suspended at 1X10⁶/ml in MMG solution (4mM HEPES pH 5.7, 400 mM mannitol and 15 mM MgCl2). To transfect the protoplasts with Cas9/sgRNA complex, 200 µL (200,000 protoplasts) of the protoplast suspension were gently mixed with 3.3 or 10 uL of Cas9/sgRNA complex [Cas9 protein (6 µg/µL) and two sgRNAs (2.2 µg/µL each)] and 200 ul of 40% polyethylene glycol transfection buffer (40% PEG4000, 200 mM mannitol and 100 mM CaCl2) in 2 ml tubes. After 5∼20 min incubation at room temperature, transfection was stopped by adding wash buffer with W5 solution (2 mM MES pH 5.7, 154 mM NaCl, 125 mM CaCl2 and 5 mM KCl). Protoplasts were then collected by centrifugation for 5 min at 100 X g, washed with 1 ml of W5 solution, centrifuged for another 5 min at 100 X g. The density of protoplasts was adjusted to 1 X 10⁵ /ml and they were cultured in modified KM 8p liquid medium with 400 mM glucose.

### 4-4. Detection of mutations in Arabidopsis protoplasts and plants

After 24 hr or 72 hr post-transfection, protoplasts were collected and genomic DNA was isolated. The genomic DNA region spanning the two target sites was PCR-amplified and subjected to the T7E1 assay. As shown in Figure 11, indels were induced by RGENs at high frequencies that ranged from 50% to 70%. Surprisingly, mutations were induced at 24 hr post-transfection. Apparently, Cas9 protein functions immediately after transfection. PCR products were purified and cloned into T-Blunt PCR Cloning Kit (Solgent). Plasmids were purified and subjected to Sanger sequencing with M13F primer. One mutant sequence had a 7-bp deletion at one site(Figure 12). The other three mutant sequences had deletions of ∼220-bp DNA segments between the two RGEN site.

### Example 5: Cas9 protein transduction using a cell-penetrating peptide or protein transduction domain (reference example)

### 5-1. Construction of His-Cas9-encoding plasmid

Cas9 with a cysteine at the C-terminal was prepared by PCR amplification using the previously described Cas9 plasmid {Cho, 2013 #166} as the template and cloned into pET28-(a) vector (Novagen, Merk Millipore, Germany) containing His-tag at the N-terminus.

### 5-2. Cell culture

293T (Human embryonic kidney cell line), and HeLa (human ovarian cancer cell line) were grown in DMEM (GIBCO-BRL Rockville) supplemented with 10% FBS and 1% penicillin and streptomycin.

### 5-3. Expression and purification of Cas9 protein

To express the Cas9 protein, E. coli BL21 cells were transformed with thepET28-(a) vector encoding Cas9and plated onto Luria-Bertani (LB) agar medium containing 50 µg/mL kanamycin (Amresco, Solon, OH). Next day, a single colony was picked and cultured in LB broth containing 50 µg/mL kanamycin at 37°C overnight. Following day, this starter culture at 0.1 OD600 was inoculated into Luria broth containing 50 µg/mL kanamycin and incubated for 2 hrs at 37°C until OD600 reached to 0.6-0.8. To induce Cas9 protein expression, the cells were cultured at 30°C overnight after addition of isopropyl-β-D-thiogalactopyranoside (IPTG) (Promega, Madison, WI) to the final concentration of 0.5mM.

The cells were collected by centrifugation at 4000 rpm for 15-20 mins, resuspendedin a lysis buffer (20mM Tris-Cl pH8.0, 300mM NaCl, 20mM imidazole, IX protease inhibitor cocktail, 1 mg/ml lysozyme), and lysed by sonication (40% duty, 10 sec pulse, 30 sec rest, for 10 mins on ice). The soluble fraction was separated as the supernatant after centrifugation at 15,000 rpm for 20 mins at 4°C. Cas9 protein was purified at 4°C using a column containing Ni-NTA agarose resin (QIAGEN) and AKTA prime instrument (AKTA prime, GE Healthcare, UK). During this chromatography step, soluble protein fractions were loaded onto Ni-NTA agarose resin column (GE Healthcare, UK) at the flow rate of 1 mL/min. The column was washed with a washing buffer (20mM Tris-Cl pH8.0, 300mM NaCl, 20mM imidazole, IX protease inhibitor cocktail) and the bound protein was eluted at the flow rate of 0.5 ml/min with an elution buffer (20mM Tris-Cl pH8.0, 300mM NaCl, 250mM imidazole, IX protease inhibitor cocktail). The pooled eluted fraction was concentrated and dialyzed against storage buffer (50 mM Tris-HCl, pH8.0, 200 mM KCl, 0.1 mM EDTA, 1 mM DTT, 0.5 mM PMSF, 20% Glycerol). Protein concentration was quantitated by Bradford assay (Biorad, Hercules, CA) and purity was analyzed by SDS-PAGE using bovine serum albumin as the control.

### 5-4. Conjugation of Cas9 to 9R4L

1mg Cas9 protein diluted in PBS at the concentration of 1mg/mL and 50 µg of maleimide-9R4L peptide in 25µL DW (Peptron, Korea) weregently mixed using a rotor at room temperature for 2 hrsand at 4°C overnight. To remove unconjugated maleimide-9R4L, the samples were dialyzed using 50kDamolecular weight cutoff membrane against of DPBS (pH 7.4) at 4°C for 24 hrs. Cas9-9R4L protein was collected from the dialysis membrane and the protein amount was determined using Bradford assay.

### 5-5. Preparation of sgRNA-9R4L

sgRNA (1 µg)was gently added to various amounts of C9R4LC peptide (ranging from 1 to 40 weight ratio) in 100 µl of DPBS (pH 7.4). This mixture was incubated at room temperature for 30 mins and diluted to 10 folds using RNAse-free deionized water. The hydrodynamic diameter and z-potential of the formed nanoparticles were measured using dynamic light scattering (Zetasizer-nano analyzer ZS; Malvern instruments, Worcestershire, UK).

### 5-6. Cas9 protein and sgRNA treatments

Cas9-9R4L and sgRNA-C9R4LC were treated to the cells as follows: 1µg of sgRNA and 15 µg of C9R4LC peptide were added to 250 mL of OPTIMEM medium and incubated at room temperature for 30 mins. At 24 hrs after seeding, cells were washed with OPTIMEM medium and treated with sgRNA-C9R4LC complex for 4 hrs at 37°C. Cells were washed again with OPTIMEM medium and treated with Cas9-9R4L for 2 hrs at 37°C. After treatment, culture media was replaced with serum-containing complete medium and incubated at 37°C for 24 hrs before the next treatment. Same procedure was followed for multiple treatments of Cas9 and sgRNA for three consecutive days.

### 5-7. Cas9-9R4L and sgRNA-9R4L can edit endogenous genes in cultured mammalian cells without the use of additional delivery tools

To determine whether Cas9-9R4L and sgRNA-9R4L can edit endogenous genes in cultured mammalian cells without the use of additional delivery tools, we treated 293 cells with Cas9-9R4L and sgRNA-9R4L targeting the CCR5 gene and analyzed the genomic DNA. T7E1 assay showed that 9% of CCR5 gene was disrupted in cells treated with both Cas9-9R4L and sgRNA-9R4L and that the CCR5 gene disruption was not observed in control cells including those untreated, treated with either Cas9-9R or sgRNA-9R4L, or treated with both unmodified Cas-9 and sgRNA (Fig 13), suggesting that the treatment with Cas9-9R4L protein and sgRNA conjugated with 9R4L, but not unmodified Cas9 and sgRNA, can lead to efficient genome editing in mammalian cells.

### Example 6: Control of off-target mutation according to guide RNA structure (reference example)

Recently, three groups reported that RGENs had off-target effects in human cells. To our surprise, RGENs induced mutations efficiently at off-target sites that differ by 3 to 5 nucleotides from on-target sites. We noticed, however, that there were several differences between our RGENs and those used by others. First, we used dualRNA, which is crRNA plus tracrRNA, rather than single-guide RNA (sgRNA) that is composed of essential portions of crRNA and tracrRNA. Second, we transfected K562 cells (but not HeLa cells) with synthetic crRNA rather than plasmids encoding crRNA. HeLa cells were transfected with crRNA-encoding plasmids. Other groups used sgRNA-encoding plasmids. Third, our guide RNA had two additional guanine nucleotides at the 5' end, which are required for efficient transcription by T7 polymerase *in vitro.* No such additional nucleotides were included in the sgRNA used by others. Thus, the RNA sequence of our guide RNA can be shown as 5'-GGX₂₀, whereas 5'-GX₁₉, in which X₂₀ or GX₁₉ corresponds to the 20-bp target sequence, represents the sequence used by others. The first guanine nucleotide is required for transcription by RNA polymerase in cells. To test whether off-target RGEN effects can be attributed to these differences, we chose four RGENs that induced off-target mutations in human cells at high frequencies(13). First, we compared our method of using *in vitro* transcribed dualRNA with the method of transfecting sgRNA-encoding plasmids in K562 cells and measured mutation frequencies at the on-target and off-target sites via the T7E1 assay. Three RGENs showed comparable mutation frequencies at on-target and off-target sites regardless of the composition of guide RNA. Interestingly, one RGEN (VEFGA site 1) did not induce indels at one validated off-target site, which differs by three nucleotides from the on-target site (termed OT1-11, Fig. 14), when synthetic dualRNA was used. But the synthetic dualRNA did not discriminate the other validated off-target site (OT1-3), which differs by two nucleotides from the on-target site.

Next, we tested whether the addition of two guanine nucleotides at the 5' end of sgRNA could make RGENs more specific by comparing 5'-GGX₂₀ (or 5'-GGGX₁₉) sgRNA with 5'-GX₁₉ sgRNA. Four GX₁₉ sgRNAs complexed with Cas9 induced indels equally efficiently at on-target and off-target sites, tolerating up to four nucleotide mismatches. In sharp contrast, GGX₂₀ sgRNAs discriminated off-target sites effectively. In fact, the T7E1 assay barely detected RGEN-induced indels at six out of the seven validated off-target sites when we used the four GGX₂₀ sgRNAs (Fig. 15). We noticed, however, that two GGX₂₀ sgRNAs (VEGFA sites 1 and 3) were less active at on-target sites than were the corresponding GX₁₉ sgRNAs. These results show that the extra nucleotides at the 5' end can affect mutation frequencies at on-target and off-target sites, perhaps by altering guide RNA stability, concentration, or secondary structure.

These results suggest that three factors-the use of synthetic guide RNA rather than guide RNA-encoding plasmids, dualRNA rather than sgRNA, and GGX₂₀ sgRNA rather than GX₁₉ sgRNA-have cumulative effects on the discrimination of off-target sites.

### Example 7: Paired Cas9 nickases (reference example)

In principle, single-strand breaks (SSBs) cannot be repaired by error-prone NHEJ but still trigger high fidelity homology-directed repair (HDR) or base excision repair. But nickase-induced targeted mutagenesis via HDR is much less efficient than is nuclease-induced mutagenesis. We reasoned that paired Cas9 nickases would produce composite DSBs, which trigger DNA repair via NHEJ or HDR, leading to efficient mutagenesis (Fig. 16A). Furthermore, paired nickases would double the specificity of Cas9-based genome editing.

We first tested several Cas9 nucleases and nickases designed to target sites in the AAVS1 locus (Fig. 16B) *in vitro* via fluorescent capillary electrophoresis. Unlike Cas9 nucleases that cleaved both strands of DNA substrates, Cas9 nickases composed of guide RNA and a mutant form of Cas9 in which a catalytic aspartate residue is changed to an alanine (D10A Cas9) cleaved only one strand, producing site-specific nicks (Fig. 16C,D). Interestingly, however, some nickases (AS1, AS2, AS3, and S6 in Fig. 17A) induced indels at target sites in human cells, suggesting that nicks can be converted to DSBs, albeit inefficiently, *in vivo.* Paired Cas9 nickases producing two adjacent nicks on opposite DNA strands yielded indels at frequencies that ranged from 14% to 91%, comparable to the effects of paired nucleases (Fig. 17A). The repair of two nicks that would produce 5' overhangs led to the formation of indels much more frequently than those producing 3' overhangs at three genomic loci (Fig. 17A and Fig. 18). In addition, paired nickases enabled targeted genome editing via homology-directed repair more efficiently than did single nickases (Fig. 19).

We next measured mutation frequencies of paired nickases and nucleases at off-target sites using deep sequencing. Cas9 nucleases complexed with three sgRNAs induced off-target mutations at six sites that differ by one or two nucleotides from their corresponding on-target sites with frequencies that ranged from 0.5% to 10% (Fig. 17B). In contrast, paired Cas9 nickases did not produce indels above the detection limit of 0.1% at any of the six off-target sites. The S2 Off-1 site that differs by a single nucleotide at the first position in the PAM (*i.e.,* N in NGG) from its on-target site can be considered as another on-target site. As expected, the Cas9 nuclease complexed with the S2 sgRNA was equally efficient at this site and the on-target site. In sharp contrast, D10A Cas9 complexed with the S2 and AS2 sgRNAs discriminated this site from the on-target site by a factor of 270 fold. This paired nickase also discriminated the AS2 off-target sites (Off-1 and Off-9 in Fig. 17B) from the on-target site by factors of 160 fold and 990 fold, respectively.

### Example 8: Chromosomal DNA splicing induced by paired Cas9 nickases (reference example)

Two concurrent DSBs produced by engineered nucleases such as ZFNs and TALENs can promote large deletions of the intervening chromosomal segments has reproted. We tested whether two SSBs induced by paired Cas9 nickases can also produce deletions in human cells. We used PCR to detect deletion events and found that seven paired nickases induced deletions of up to 1.1-kbp chromosomal segments as efficiently as paired Cas9 nucleases did (Fig.20 A, B). DNA sequences of the PCR products confirmed the deletion events (Fig. 20C). Interestingly, the sgRNA-matching sequence remained intact in two out of seven deletion-specific PCR amplicons (underlined in Fig. 20C). In contrast, Cas9 nuclease pairs did not produce sequences that contained intact target sites. This finding suggests that two distant nicks were not converted to two separate DSBs to promote deletions of the intervening chromosomal segment. In addition, it is unlikely that two nicks separated by more than a 100 bp can produce a composite DSB with large overhangs under physiological conditions because the melting temperature is very high.

We propose that two distant nicks are repaired by strand displacement in a head-to-head direction, resulting in the formation of a DSB in the middle, whose repair via NHEJ causes small deletions (Fig. 20D). Because the two target sites remain intact during this process, nickases can induce SSBs again, triggering the cycle repeatedly until the target sites are deleted. This mechanism explains why two offset nicks producing 5' overhangs but not those producing 3' overhangs induced indels efficiently at three loci.

We then investigated whether Cas9 nucleases and nickases can induce unwanted chromosomal translocations that result from NHEJ repair of on-target and off-target DNA cleavages (Fig. 21A). We were able to detect translocations induced by Cas9 nucleases using PCR (Fig. 21B,C). No such PCR products were amplified using genomic DNA isolated from cells transfected with the plasmids encoding the AS2+S3 Cas9 nickase pair. This result is in line with the fact that both AS2 and S3 nickases, unlike their corresponding nucleases, did not produce indels at off-target sites (Fig. 17B).

These results suggest that paired Cas9 nickases allow targeted mutagenesis and large deletions of up to 1-kbp chromosomal segments in human cells. Importantly, paired nickases did not induce indels at off-target sites at which their corresponding nucleases induce mutations. Furthermore, unlike nucleases, paired nickases did not promote unwanted translocations associated with off-target DNA cleavages. In principle, paired nickases double the specificity of Cas9-mediated mutagenesis and will broaden the utility of RNA-guided enzymes in applications that require precise genome editing such as gene and cell therapy. One caveat to this approach is that two highly active sgRNAs are needed to make an efficient nickase pair, limiting targetable sites. As shown in this and other studies, not all sgRNAs are equally active. When single clones rather than populations of cells are used for further studies or applications, the choice of guide RNAs that represent unique sequences in the genome and the use of optimized guide RNAs would suffice to avoid off-target mutations associated with Cas9 nucleases. We propose that both Cas9 nucleases and paired nickases are powerful options that will facilitate precision genome editing in cells and organisms.

### Example 9: Genotyping with CRISPR/Cas-derived RNA-guided endonucleases (reference example)

Next, We reasoned that RGENs can be used in Restriction fragment length polymorphism (RFLP) analysis, replacing conventional restriction enzymes. Engineered nucleases including RGENs induce indels at target sites, when the DSBs caused by the nucleases are repaired by the error-prone non-homologous end-joining (NHEJ) system. RGENs that are designed to recognize the target sequences cannot cleave mutant sequences with indels but will cleave wildtype target sequences efficiently.

### 9-1. RGEN components

crRNA and tracrRNA were prepared by *in vitro* transcription using MEGAshortcript T7 kit (Ambion) according to the manufacturer's instruction. Transcribed RNAs were resolved on a 8% denaturing urea-PAGE gel. The gel slice containing RNA was cut out and transferred to elution buffer. RNA was recovered in nuclease-free water followed by phenol:chloroform extraction, chloroform extraction, and ethanol precipitation. Purified RNA was quantified by spectrometry. Templates for crRNA were prepared by annealing an oligonucleotide whose sequence is shown as
5'-GAAATTAATACGACTCACTATAGGX₂₀GTTTTAGAGCTATGCTGTTTTG-3'(SEQ ID NO: 76), in which X₂₀ is the target sequence, and its complementary oligonucleotide. The template for tracrRNA was synthesized by extension of forward and reverse oligonucleotides 
(5'-GAAATTAATACGACTCACTATAGGAACCATTCAAAACAGCATAGCAAGTT
AAAATAAGGCTAGTCCG-3' (SEQ ID NO: 77)
 and 5'-AAAAAAAGCACCGACTCGGTGCCACTTTTTCAAGTTGATAACGGACTAGCC TTATTTTAACTTGCTATG-3'(SEQ ID NO: 78) using Phusion polymerase (New England Biolabs).

### 9-2. Recombinant Cas9 protein purification

The Cas9 DNA construct used in our previous Example, which encodes Cas9 fused to the His6-tag at the C terminus, was inserted in the pET-28a expression vector. The recombinant Cas9 protein was expressed in *E. coli* strain BL21(DE3) cultured in LB medium at 25°C for 4 hour after induction with 1 mM IPTG. Cells were harvested and resuspended in buffer containing 20 mM Tris PH 8.0, 500 mM NaCl, 5 mM immidazole, and 1 mM PMSF. Cells were frozen in liquid nitrogen, thawed at 4°C, and sonicated. After centrifugation, the Cas9 protein in the lysate was bound to Ni-NTA agarose resin (Qiagen), washed with buffer containing 20 mM Tris pH 8.0, 500 mM NaCl, and 20 mM immidazole, and eluted with buffer containing 20 mM Tris pH 8.0, 500 mM NaCl, and 250 mM immidazole. Purified Cas9 protein was dialyzed against 20 mM HEPES (pH 7.5), 150 mM KC1, 1 mM DTT, and 10% glycerol and analyzed by SDS-PAGE.

### 9-3. T7 endonuclease I assay

The T7E1 assay was performed as following. In brief, PCR products amplified using genomic DNA were denatured at 95°C, reannealed at 16°C, and incubated with 5 units of T7 Endonuclease I (New England BioLabs) for 20 min at 37°C. The reaction products were resolved using 2 to 2.5% agarose gel electrophoresis.

### 9-4. RGEN-RFLP assay

PCR products (100-150 ng) were incubated for 60 min at 37°C with optimized concentrations (Table 10) of Cas9 protein, tracrRNA, crRNA in 10 µl NEB buffer 3 (IX). After the cleavage reaction, RNase A (4 µg) was added, and the reaction mixture was incubated for 30 min at 37°C to remove RNA. Reactions were stopped with 6X stop solution buffer containing 30% glycerol, 1.2% SDS, and 100 mM EDTA. Products were resolved with 1-2.5% agarose gel electrophoresis and visualized with EtBr staining.

**Table 10**

| Concentration of RGEN components in RFLP assays | | | |
|---|---|---|---|
| Target Name | Cas9 (ng/µl) | crRNA (ng/µl) | tracrRNA (ng/µl) |
| *C4BPB* | *100* | *25* | *60* |
| *PIBF*-NGG-RGEN | *100* | *25* | *60* |
| *HLA-B* | *1.2* | *0.3* | *0.7* |
| *CCR5-ZFN* | *100* | *25* | *60* |
| *CTNNB1* Wild type specific | *30* | *10* | *20* |
| *CTNNB1* mutant specific | *30* | *10* | *20* |
| *CCR5* WT-specific | *100* | *25* | *60* |
| *CCR5* Δ32-specific | *10* | *2.5* | *6* |
| *KRAS* WT specific(wt) | *30* | *10* | *20* |
| *KRAS* mutant specific(m8) | *30* | *10* | *20* |
| *KRAS* WT specific (m6) | *30* | *10* | *20* |
| *KRAS* mutant specific (m6,8) | *30* | *10* | *20* |
| *PIK3CA WT specific (wt)* | *100* | *25* | *60* |
| *PIK3CA* mutant specific(m4) | *30* | *10* | *20* |
| *PIK3CA* WT specific (m7) | *100* | *25* | *60* |
| *PIK3CA* mutant specific(m4,7) | *30* | *10* | *20* |
| *BRAF* WT-specific | *30* | *10* | *20* |
| *BRAF* mutant-specific | *100* | *25* | *60* |
| *NRAS* WT-specific | *100* | *25* | *60* |
| *NRAS* mutant-specific | *30* | *10* | *20* |
| *IDH* WT-specific | *30* | *10* | *20* |
| *IDH* mutant-specific | *30* | *10* | *20* |
| *PIBF*-NAG-RGEN | *30* | *10* | *60* |

**Table 11**

| Primers | | | |
|---|---|---|---|
| **Gene(site)** | **Direction** | **Sequence(5' to 3')** | **SEQ ID NO** |
| CCR5(RGEN) | F1 | CTCCATGGTGCTATAGAGCA | 79 |
| | F2 | GAGCCAAGCTCTCCATCTAGT | 80 |
| | R | GCCCTGTCAAGAGTTGACAC | 81 |
| CCR5(ZFN) | F | GCACAGGGTGGAACAAGATGGA | 82 |
| | R | GCCAGGTACCTATCGATTGTCAGG | 83 |
| CCR5(del32) | F | GAGCCAAGCTCTCCATCTAGT | 84 |
| | R | ACTCTGACTG GGTCACCAGC | 85 |
| *C4BPB* | *F1* | *TATTTGGCTGGTTGAAAGGG* | *86* |
| | *R1* | *AAAGTCATGAAATAAACACACCCA* | *87* |
| | *F2* | *CTGCATTGATATGGTAGTACCATG* | *88* |
| | *R2* | *GCTGTTCATTGCAATGGAATG* | *89* |
| CTNNB1 | F | ATGGAGTTGGACATGGCCATGG | 90 |
| | R | ACTCACTATCCACAGTTCAGCATTTACC | 91 |
| *KRAS* | *F* | *TGGAGATAGCTGTCAGCAACTTT* | *92* |
| | *R* | *CAACAAAGCAAAGGTAAAGTTGGTAATAG* | *93* |
| *PIK3CA* | *F* | *GGTTTCAGGAGATGTGTTACAAGGC* | *94* |
| | *R* | *GATTGTGCAATTCCTATGCAATCGGTC* | *95* |
| *NRAS* | *F* | *CACTGGGTACTTAATCTGTAGCCTC* | *96* |
| | *R* | *GGTTCCAAGTCATTCCCAGTAGC* | *97* |
| *IDH1* | *F* | *CATCACTGCAGTTGTAGGTTATAACTATCC* | *98* |
| | *R* | *TTGAAAACCACAGATCTGGTTGAACC* | *99* |
| *BRAF* | *F* | *GGAGTGCCAAGAGAATATCTGG* | *100* |
| | *R* | *CTGAAACTGGTTTCAAAATATTCGTTTTAAGG* | *101* |
| *PIBF* | *F* | *GCTCTGTATGCCCTGTAGTAGG* | *102* |
| | *R* | *TTTGCATCTGACCTTACCTTTG* | *103* |

### 9-5. Plasmid cleavage assay

Restriction enzyme-treated linearized plasmid (100 ng) was incubated for 60 min at 37 °C with Cas9 protein (0.1 µg), tracrRNA (60 ng), and crRNA (25 ng) in 10 µl NEB 3 buffer (IX). Reactions were stopped with 6X stop solution containing 30% glycerol, 1.2% SDS, and100 mM EDTA. Products were resolved with 1% agarose gel electrophoresis and visualized with EtBr staining.

### 9-6. Strategy of RFLP

New RGENs with desired DNA specificities can be readily created by replacing crRNA; no de novo purification of custom proteins is required once recombinant Cas9 protein is available. Engineered nucleases, including RGENs, induce small insertions or deletions (indels) at target sites when the DSBs caused by the nucleases are repaired by error-prone non-homologous end-joining (NHEJ). RGENs that are designed to recognize the target sequences cleave wild-type sequences efficiently but cannot cleave mutant sequences with indels (Fig. 22).

We first tested whether RGENs can differentially cleave plasmids that contain wild-type or modified C4BPB target sequences that harbor 1- to 3-base indels at the cleavage site. None of the six plasmids with these indels were cleaved by a C4BPB-specific RGEN5 composed of target-specific crRNA, tracrRNA, and recombinant Cas9 protein (Fig. 23). In contrast, the plasmid with the intact target sequence was cleaved efficiently by this RGEN.

### 9-7. Detection of mutations induced by the same RGENs using RGEN-mediated RFLP

Next, to test the feasibility of RGEN-mediated RFLP for detection of mutations induced by the same RGENs, we utilized gene-modified K562 human cancer cell clones established using an RGEN targeting C4BPB gene (Table 12).

**Table 12**

| Target sequence of RGENs used in this study | | |
|---|---|---|
| Gene | Target sequence | SEQ ID NO |
| human C4BPB | AATGACCACTACATCCTCAAGGG | 104 |
| mouse Pibfl | AGATGATGTCTCATCATCAGAGG | 105 |

C4BPB mutant clones used in this study have various mutations ranging from 94 bp deletion to 67 bp insertion (Fig. 24A). Importantly, all mutations occurred in mutant clones resulted in the loss of RGEN target site. Among 6 C4BPB clones analyzed, 4 clones have both wildtype and mutant alleles (+/-) and 2 clones have only mutant alleles (-/-).

The PCR products spanning the RGEN target site amplified from wildtype K562 genomic DNA were digested completely by the RGEN composed of target-specific crRNA, tracrRNA, and recombinant Cas9 protein expressed in and purified from E. coli (Fig. 24B/Lane 1). When the C4BPB mutant clones were subjected to RFLP analysis using the RGEN, PCR amplicons of +/- clones that contained both wildtype and mutant alleles were partially digested, and those of -/- cloned that did not contain the wildtype allele were not digested at all, yielding no cleavage products corresponding to the wildtype sequence (Fig. 24B). Even a single-base insertion at the target site blocked the digestion (#12 and #28 clones) of amplified mutant alleles by the C4BPB RGEN, showing the high specificity of RGEN-mediated RFLP. We subjected the PCR amplicons to the mismatch-sensitive T7E1 assay in parallel (Fig. 24B). Notably, the T7E1 assay was not able to distinguish -/- clones from +/- clones. To make it matters worse, the T7E1 assay cannot distinguish homozygous mutant clones that contain the same mutant sequence from wildtype clones, because annealing of the same mutant sequence will form a homoduplex. Thus, RGEN-mediated RFLP has a critical advantage over the conventional mismatch-sensitive nuclease assay in the analysis of mutant clones induced by engineered nucleases including ZFNs, TALENs and RGENs.

### 9-8. Quantitative assay for RGEN-RFLP analysis

We also investigated whether RGEN-RFLP analysis is a quantitative method. Genomic DNA samples isolated from the C4BPB null clone and the wild-type cells were mixed at various ratios and used for PCR amplifications. The PCR products were subjected to RGEN genotyping and the T7E1 assay in parallel (Fig. 25b). As expected, DNA cleavage by the RGEN was proportional to the wild type to mutant ratio. In contrast, results of the T7E1 assay correlated poorly with mutation frequencies inferred from the ratios and were inaccurate, especially at high mutant %, a situation in which complementary mutant sequences can hybridize with each other to form homoduplexes.

### 9-9. Analysis of mutant mouse founders using a RGEN-mediated RFLP genotyping

We also applied RGEN-mediated RFLP genotyping (RGEN genotyping in short) to the analysis of mutant mouse founders that had been established by injection of TALENs into mouse one-cell embryos (Fig. 26A). We designed and used an RGEN that recognized the TALEN target site in the Pibfl gene (Table 10). Genomic DNA was isolated from a wildtype mouse and mutant mice and subjected to RGEN genotyping after PCR amplification. RGEN genotyping successfully detected various mutations, which ranged from one to 27-bp deletions (Fig. 26B). Unlike the T7E1 assay, RGEN genotyping enabled differential detection of +/- and -/- founder.

### 9-10. Detection of mutations induced in human cells by a CCR5-specific ZFN using RGENs

In addition, we used RGENs to detect mutations induced in human cells by a CCR5-specific ZFN, representing yet another class of engineered nucleases (Fig. 27). These results show that RGENs can detect mutations induced by nucleases other than RGENs themselves. In fact, we expect that RGENs can be designed to detect mutations induced by most, if not all, engineered nucleases. The only limitation in the design of an RGEN genotyping assay is the requirement for the GG or AG (CC or CT on the complementary strand) dinucleotide in the PAM sequence recognized by the Cas9 protein, which occurs once per 4 bp on average. Indels induced anywhere within the seed region of several bases in crRNA and the PAM nucleotides are expected to disrupt RGEN-catalyzed DNA cleavage. Indeed, we identified at least one RGEN site in most (98%) of the ZFN and TALEN sites.

### 9-11. Detection of polymorphisms or variations using RGEN

Next, we designed and tested a new RGEN that targets a highly polymorphic locus, HLA-B, that encodes Human Leukocyte Antigen B (a.k.a. MHC class I protein) (Fig. 28). HeLa cells were transfected with RGEN plasmids, and the genomic DNA was subjected to T7E1 and RGEN-RFLP analyses in parallel. T7E1 produced false positive bands that resulted from sequence polymorphisms near the target site (Fig. 25c). As expected, however, the same RGEN used for gene disruption cleaved PCR products from wild-type cells completely but those from RGEN-transfected cells partially, indicating the presence of RGEN-induced indels at the target site. This result shows that RGEN-RFLP analysis has a clear advantage over the T7E1 assay, especially when it is not known whether target genes have polymorphisms or variations in cells of interest.

### 9-12. Detection of recurrent mutations found in cancer and naturally-occurring polymorphisms through RGEN-RFLP analysis

RGEN-RFLP analysis has applications beyond genotyping of engineered nuclease-induced mutations. We sought to use RGEN genotyping to detect recurrent mutations found in cancer and naturally-occurring polymorphisms. We chose the human colorectal cancer cell line, HCT116, which carries a gain-of-function 3-bp deletion in the oncogenic CTNNB1 gene encoding beta-catenin. PCR products amplified from HCT116 genomic DNA were cleaved partially by both wild-type-specific and mutant-specific RGENs, in line with the heterozygous genotype in HCT116 cells (Fig. 29a). In sharp contrast, PCR products amplified from DNA from HeLa cells harboring only wild-type alleles were digested completely by the wild-type-specific RGEN and were not cleaved at all by the mutation-specific RGEN.

We also noted that HEK293 cells harbor the 32-bp deletion (del32) in the CCR5 gene, which encodes an essential co-receptor of HIV infection: Homozygous del32 CCR5 carriers are immune to HIV infection. We designed one RGEN specific to the del32 allele and the other to the wild-type allele. As expected, the wild-type-specific RGEN cleaved the PCR products obtained from K562, SKBR3, or HeLa cells (used as wild-type controls) completely but those from HEK293 cells partially (Fig. 30a), confirming the presence of the uncleavable del32 allele in HEK293 cells. Unexpectedly, however, the del32-specific RGEN cleaved the PCR products from wild-type cells as efficiently as those from HEK293 cells. Interestingly, this RGEN had an off-target site with a single-base mismatch immediately downstream of the on-target site (Fig. 30). These results suggest that RGENs can be used to detect naturally-occurring indels but cannot distinguish sequences with single nucleotide polymorphisms or point mutations due to their off-target effects.

To genotype oncogenic single-nucleotide variations using RGENs, we attenuated RGEN activity by employing a single-base mismatched guide RNA instead of a perfectly-matched RNA. RGENs that contained the perfectly-matched guide RNA specific to the wild-type sequence or mutant sequence cleaved both sequences (Figs. 31a and 32a). In contrast, RGENs that contained a single-base mismatched guide RNA distinguished the two sequences, enabling genotyping of three recurrent oncogenic point mutations in the KRAS, PIK3CA, and IDH1 genes in human cancer cell lines (Fig. 29b and Figs. 33a,b). In addition, we were able to detect point mutations in the BRAF and NRAS genes using RGENs that recognize the NAG PAM sequence (Figs. 33c,d). We believe that we can use RGEN-RFLP to genotype almost any, if not all, mutations or polymorphisms in the human and other genomes.

The above data proposes RGENs as providing a platform to use simple and robust RFLP analysis for various sequence variations. With high flexibility in reprogramming target sequence, RGENs can be used to detect various genetic variations (single nucleotide variations, small insertion/deletions, structural variations) such as disease-related recurring mutations, genotypes related to drug-response by a patient and also mutations induced by engineered nucleases in cells. Here, we used RGEN genotyping to detect mutations induced by engineered nucleases in cells and animals. In principle, one could also use RGENs that will specifically detect and cleave naturally-occurring variations and mutations.

### References

1. M. Jinek et al., Science 337, 816 (Aug 17, 2012).
2. H. Kim, E. Um, S. R. Cho, C. Jung, J. S. Kim, Nat Methods 8, 941 (Nov, 2011).
3. H. J. Kim, H. J. Lee, H. Kim, S. W. Cho, J. S. Kim, Genome Res 19, 1279 (Jul, 2009).
4. E. E. Perez et al., Nat Biotechnol 26, 808 (Jul, 2008).
5. J. C. Miller et al., Nat Biotechnol 29, 143 (Feb, 2011).
6. C. Mussolino et al., Nucleic Acids Res 39, 9283 (Nov, 2011).
7. J. Cohen, Science 332, 784 (May 13, 2011).
8. V. Pattanayak, C. L. Ramirez, J. K. Joung, D. R. Liu, Nat Methods 8, 765 (Sep, 2011).
9. R. Gabriel et al., Nat Biotechnol 29, 816 (Sep, 2011).
10. E. Kim et al., Genome Res, (Apr 20, 2012).
11. H. J. Lee, J. Kweon, E. Kim, S. Kim, J. S. Kim, Genome Res 22, 539 (Mar, 2012).
12. H. J. Lee, E. Kim, J. S. Kim, Genome Res 20, 81 (Jan, 2010).
13. Fu Y, Foden JA, Khayter C, Maeder ML, Reyon D, Joung JK, Sander JD. Highfrequency off-target mutagenesis induced by CRISPR-Cas nucleases in human cells. Nat Biotech advance online publication (2013)
## Claims

1. A Type II Clustered Regularly interspaced Short Palindromic Repeats (CRISPR)/CRISPR-associated protein 9 (Cas9) composition for cleaving a target DNA in a plant cell, wherein the target DNA is endogenous DNA, and wherein the composition comprises a complex of:
(a) a CRISPR-associated protein 9 (Cas9) polypeptide; and
(b) a guide RNA specific to the target DNA,
wherein the guide RNA is:
(i) a dual guide RNA comprising a CRISPR RNA (crRNA) and a trans activating crRNA (tracrRNA); or
(ii) a single-chain guide RNA comprising a CRISPR RNA (crRNA) fused to a trans activating crRNA (tracrRNA),
wherein the composition further comprises polyethylene glycol (PEG), and wherein the plant cell is a protoplast.

2. The Type II CRISPR/Cas9 composition of claim 1, wherein the composition comprises two guide RNAs, wherein each guide RNA is specific for one of the strands of the target DNA, and wherein the Cas9 polypeptide is a Cas9 nickase.

3. A method of cleaving a target DNA in a plant protoplast, wherein the target DNA is endogenous DNA, the method comprising introducing into the plant protoplast a complex of:
(a) a CRISPR-associated protein 9 (Cas9) polypeptide; and
(b) a guide RNA specific to the target DNA,
wherein the guide RNA is:
(i) a dual guide RNA comprising a CRISPR RNA (crRNA) and a trans activating crRNA (tracrRNA); or
(ii) a single-chain guide RNA comprising a CRISPR RNA (crRNA) fused to a trans activating crRNA (tracrRNA),
wherein the introduction is by polyethylene glycol (PEG)-mediated transfection.

4. A method of introducing two nicks in different strands of a target DNA in a plant protoplast, wherein the target DNA is endogenous DNA, the method comprising introducing into the plant protoplast a complex of:
(a) a CRISPR-associated protein 9 (Cas9) polypeptide, wherein the Cas9 polypeptide is a Cas9 nickase; and
(b) two guide RNAs, wherein each of the two guide RNAs is specific for one of the strands of the target DNA,
wherein the guide RNA is:
(i) a dual guide RNA comprising a CRISPR RNA (crRNA) and a trans activating crRNA (tracrRNA); or
(ii) a single-chain guide RNA comprising a CRISPR RNA (crRNA) fused to a trans activating crRNA (tracrRNA),
wherein the introduction is by polyethylene glycol (PEG)-mediated transfection.

5. The method of claim 4, wherein the two nicks are separated by at least 100 base pairs.

6. The method of claims 3 to 5, wherein the PEG-mediated transfection comprises transfection in a 40% PEG transfection buffer, and wherein the 40% PEG transfection buffer comprises 40% PEG4000, 200 mM mannitol and 100 mM CaCl₂.

7. A kit for cleaving a target DNA in a plant cell comprising the composition of claim 1 or 2, wherein the plant cell is a protoplast.

8. The Type II CRISPR/Cas9 composition of claim 1 or 2, the method of claims 3 to 6, or the kit of claim 7, wherein the Cas9 protein is from the genus *Streptococcus.*

9. The Type II CRISPR/Cas9 composition of claim 1 or 2, the method of claims 3 to 6, or the kit of claim 7, wherein the Cas9 protein is from *Streptococcus pyogenes.*

10. The Type II CRISPR/Cas9 composition of claim 2, the method of claims 4 to 6, or the kit of claim 7, wherein the Cas9 nickase is a mutant form of Cas9, in which the catalytic aspartate residue is changed to any other amino acid, preferably alanine.

11. The Type II CRISPR/Cas9 composition of claim 1 or 2, the method of claims 3 to 6, or the kit of claim 7, wherein the Cas9 polypeptide comprises a Cas9 protein and a nuclear localization signal (NLS), wherein the NLS is at the N-terminus of the Cas9 protein.

12. The Type II CRISPR/Cas9 composition of claim 1 or 2, the method of claims 3 to 6, or the kit of claim 7, wherein the crRNA comprises two additional guanine nucleotides at its 5' end.

13. The Type II CRISPR/Cas9 composition of claim 1 or 2, the method of claims 3 to 6, or the kit of claim 7, wherein the target DNA is genomic DNA.

14. The Type II CRISPR/Cas9 composition of claim 1 or 2, the method of claims 3 to 6, or the kit of claim 7, wherein the Cas9 protein is from *Streptococcus pyogenes* and wherein the target DNA comprises a trinucleotide protospacer adjacent motif (PAM) recognized by Cas9, wherein the PAM consists of the trinucleotide 5'-NGG-3'.

## Patentansprüche

1. Typ II-Clustered Regularly Interspaced Short Palindromic Repeats (CRISPR)/CRISPR-assoziiertes Protein 9 (Cas9)-Zusammensetzung zum Schneiden einer Ziel-DNA in einer Pflanzenzelle, wobei die Ziel-DNA endogene DNA ist, und wobei die Zusammensetzung einen Komplex umfasst aus:
(a) einem CRISPR-assoziiertes Protein 9 (Cas9) Polypeptid; und
(b) einer guide-RNA spezifisch für die Ziel-DNA,
wobei die guide-RNA ist:
(i) eine duale guide-RNA, die eine CRISPR-RNA (crRNA) und eine trans-aktivierende crRNA (tracrRNA) umfasst; oder
(ii) eine einzelsträngige guide-RNA, umfassend eine CRISPR RNA (crRNA), fusioniert an eine trans-aktivierende crRNA (tracrRNA),
wobei die Zusammensetzung ferner Polyethylenglykol (PEG) umfasst und wobei die Pflanzenzelle ein Protoplast ist.

2. Typ-II-CRISPR/Cas9-Zusammensetzung nach Anspruch 1, wobei die Zusammensetzung zwei guide-RNAs umfasst, wobei jede guide-RNA für einen der Stränge der Ziel-DNA spezifisch ist, und wobei das Cas9-Polypeptid eine Cas9-Nickase ist.

3. Verfahren zum Spalten einer Ziel-DNA in einem Pflanzenprotoplasten, wobei die Ziel-DNA endogene DNA ist, wobei das Verfahren das Einführen eines Komplexes aus folgenden Bestandteilen in den Pflanzenprotoplasten umfasst:
(a) ein CRISPR-assoziiertes Protein 9 (Cas9) Polypeptid; und
(b) eine guide-RNA spezifisch für die Ziel-DNA,
wobei die guide-RNA ist:
(i) eine duale guide-RNA, die eine CRISPR-RNA (crRNA) und eine trans-aktivierende crRNA (tracrRNA) umfasst; oder
(ii) eine einzelsträngige guide-RNA, umfassend eine CRISPR RNA (crRNA), fusioniert an eine trans-aktivierende crRNA (tracrRNA),
wobei die Einführung durch Polyethylenglykol (PEG)-vermittelte Transfektion erfolgt.

4. Verfahren zum Einführen von zwei Brüchen in verschiedenen Strängen einer Ziel-DNA in einem Pflanzenprotoplasten, wobei die Ziel-DNA endogene DNA ist, wobei das Verfahren das Einführen eines Komplexes aus folgenden Bestandteilen in den Pflanzenprotoplasten umfasst:
(a) ein CRISPR-assoziiertes Protein 9 (Cas9) Polypeptid, wobei das Cas9-Polypeptid eine Cas9-Nickase ist; und
(b) zwei guide-RNAs, wobei jede der beiden guide-RNAs spezifisch für einen der Stränge der Ziel-DNA ist,
wobei die guide-RNA ist:
(i) eine duale guide-RNA, die eine CRISPR-RNA (crRNA) und eine trans-aktivierende crRNA (tracrRNA) umfasst; oder
(ii) eine einzelsträngige guide-RNA, umfassend eine CRISPR RNA (crRNA), fusioniert an eine trans-aktivierende crRNA (tracrRNA),
wobei die Einführung durch Polyethylenglykol (PEG)-vermittelte Transfektion erfolgt.

5. Verfahren nach Anspruch 4, wobei die beiden Brüche durch mindestens 100 Basenpaare getrennt sind.

6. Verfahren nach den Ansprüchen 3 bis 5, wobei die PEG-vermittelte Transfektion eine Transfektion in einem 40%-igen PEG-Transfektionspuffer umfasst und wobei der 40%-ige PEG-Transfektionspuffer 40 % PEG4000, 200 mM Mannitol und 100 mM CaCl₂ umfasst.

7. Kit zum Schneiden einer Ziel-DNA in einer Pflanzenzelle, umfassend die Zusammensetzung nach Anspruch 1 oder 2, wobei die Pflanzenzelle ein Protoplast ist.

8. Typ-II-CRISPR/Cas9-Zusammensetzung nach Anspruch 1 oder 2, das Verfahren nach den Ansprüchen 3 bis 6 oder das Kit nach Anspruch 7, wobei das Cas9-Protein aus der Gattung *Streptococcus* stammt.

9. Typ-II-CRISPR/Cas9-Zusammensetzung nach Anspruch 1 oder 2, das Verfahren nach den Ansprüchen 3 bis 6 oder das Kit nach Anspruch 7, wobei das Cas9-Protein aus *Streptococcus pyogenes* stammt.

10. Typ-II-CRISPR/Cas9-Zusammensetzung nach Anspruch 2, das Verfahren nach den Ansprüchen 4 bis 6 oder das Kit nach Anspruch 7, wobei die Cas9-Nickase eine mutierte Form von Cas9 ist, bei der der katalytische Aspartat-Rest in eine beliebige andere Aminosäure, vorzugsweise Alanin, geändert ist.

11. Typ-II-CRISPR/Cas9-Zusammensetzung nach Anspruch 1 oder 2, das Verfahren nach den Ansprüchen 3 bis 6 oder das Kit nach Anspruch 7, wobei das Cas9-Polypeptid ein Cas9-Protein und ein Kernlokalisierungssignal (nuclear localization signal; NLS) umfasst, wobei das NLS am N-Terminus des Cas9-Proteins ist.

12. Typ-II-CRISPR/Cas9-Zusammensetzung nach Anspruch 1 oder 2, das Verfahren nach den Ansprüchen 3 bis 6 oder das Kit nach Anspruch 7, wobei die crRNA zwei zusätzliche Guanin-Nukleotide an ihrem 5'-Ende umfasst

13. Typ-II-CRISPR/Cas9-Zusammensetzung nach Anspruch 1 oder 2, das Verfahren nach den Ansprüchen 3 bis 6 oder das Kit nach Anspruch 7, wobei die Ziel-DNA genomische DNA ist.

14. Typ-II-CRISPR/Cas9-Zusammensetzung nach Anspruch 1 oder 2, Verfahren nach Anspruch 3 bis 6 oder Kit nach Anspruch 7, wobei das Cas9-Protein aus *Streptococcus pyogenes* stammt und wobei die Ziel-DNA ein von Cas9 erkanntes Trinukleotid-Vorabstandshalter-angrenzendes-Motiv (Protospacer Adjacent Motif, PAM) umfasst, wobei das PAM aus dem Trinukleotid 5'-NGG-3' besteht.

## Revendications

1. Composition de Courtes Répétitions Palindromiques Groupées et Régulièrement espacées (CRISPR)/d'une protéine associée à CRISPR 9 (Cas9) pour cliver un ADN cible dans une cellule végétale, dans laquelle l'ADN cible est un ADN endogène, et dans laquelle la composition comprend un complexe de :
(a) un polypeptide de la protéine associée à CRISPR 9 (Cas9); et
(b) un ARN guide spécifique à l'ADN cible,
dans laquelle l'ARN guide est :
(i) un double ARN guide comprenant un ARN CRISPR (crARN) et un crARN d'activation trans (tracrARN); ou
(ii) un ARN guide à chaîne unique comprenant un ARN CRISPR (crARN) fusionné à un crARN trans-activateur (tracrARN),
dans laquelle la composition comprend en outre du polyéthylèneglycol (PEG), et dans laquelle la cellule végétale est un protoplaste.

2. Composition CRISPR/Cas9 de type II selon la revendication 1, dans laquelle la composition comprend deux ARN guides, dans laquelle chaque ARN guide est spécifique d'un des brins de l'ADN cible, et dans laquelle le polypeptide Cas9 est une nickase Cas9.

3. Procédé de clivage d'un ADN cible dans un protoplaste végétal, dans lequel l'ADN cible est un ADN endogène, le procédé comprenant l'introduction dans le protoplaste végétal d'un complexe de :
(a) un polypeptide de la protéine associée à CRISPR 9 (Cas9); et
(b) un ARN guide spécifique à l'ADN cible,
dans lequel l'ARN guide est :
(i) un double ARN guide comprenant un ARN CRISPR (crARN) et un crARN d'activation trans (tracrARN); ou
(ii) un ARN guide à chaîne unique comprenant un ARN CRISPR (crARN) fusionné à un crARN trans-activateur (tracrARN),
où l'introduction se fait par transfection médiée par le polyéthylène glycol (PEG).

4. Procédé d'introduction de deux entailles dans des brins différents d'un ADN cible dans un protoplaste végétal, dans lequel l'ADN cible est un ADN endogène, le procédé comprenant l'introduction dans le protoplaste végétal d'un complexe de :
(a) un polypeptide de la protéine associée à CRISPR 9 (Cas9), dans lequel le polypeptide Cas9 est une nickase Cas9; et
(b) deux ARN guides, dans lesquels chacun des deux ARN guides est spécifique d'un des brins de l'ADN cible,
dans lequel l'ARN guide est :
(i) un double ARN guide comprenant un ARN CRISPR (crARN) et un crARN d'activation trans (tracrARN); ou
(ii) un ARN guide à chaîne unique comprenant un ARN CRISPR (crARN) fusionné à un crARN trans-activateur (tracrARN),
où l'introduction se fait par transfection médiée par le polyéthylène glycol (PEG).

5. Procédé selon la revendication 4, dans lequel les deux entailles sont séparées par au moins 100 paires de bases.

6. Procédé selon les revendications 3 à 5, dans lequel la transfection médiée par le PEG comprend la transfection dans un tampon de transfection à 40 % de PEG, et dans lequel le tampon de transfection PEG 40 % comprend 40 % de PEG4000, 200 mM de mannitol et 100 mM de CaCl₂.

7. Kit de clivage d'un ADN cible dans une cellule végétale comprenant la composition de la revendication 1 ou 2, dans lequel la cellule végétale est un protoplaste.

8. Composition CRISPR/Cas9 de type II selon la revendication 1 ou 2, le procédé selon les revendications 3 à 6, ou le kit selon la revendication 7, dans laquelle/lequel la protéine Cas9 est du genre Streptococcus.

9. Composition CRISPR/Cas9 de type II selon la revendication 1 ou 2, le procédé selon les revendications 3 à 6, ou le kit selon la revendication 7, dans laquelle/lequel la protéine Cas9 provient de Streptococcus pyogenes.

10. Composition CRISPR/Cas9 de type II selon la revendication 2, le procédé selon les revendications 4 à 6, ou le kit selon la revendication 7, dans laquelle/lequel la nickase Cas9 est une forme mutante de Cas9, dans laquelle le résidu aspartate catalytique est changé en tout autre acide aminé, de préférence en alanine.

11. Composition CRISPR/Cas9 de type II selon la revendication 1 ou 2, le procédé selon les revendications 3 à 6, ou le kit selon la revendication 7, dans laquelle/lequel le polypeptide Cas9 comprend une protéine Cas9 et un signal de localisation nucléaire (NLS), dans laquelle le NLS est en N-terminal de la protéine Cas9.

12. Composition CRISPR/Cas9 de type II selon la revendication 1 ou 2, le procédé selon les revendications 3 à 6, ou le kit selon la revendication 7, dans laquelle/lequel le crARN comprend deux nucléotides guanine supplémentaires à son extrémité 5'.

13. Composition CRISPR/Cas9 de type II selon la revendication 1 ou 2, le procédé selon les revendications 3 à 6, ou le kit selon la revendication 7, dans laquelle/lequel l'ADN cible est un ADN génomique.

14. Composition CRISPR/Cas9 de type II selon la revendication 1 ou 2, le procédé selon les revendications 3 à 6, ou le kit selon la revendication 7, dans laquelle/lequel la protéine Cas9 provient de Streptococcus pyogenes et dans laquelle/lequel l'ADN cible comprend un motif adjacent proto-espaceur (PAM) trinucléotidique reconnu par Cas9, dans lequel le PAM est constitué du trinucléotide 5'-NGG-3'.
